(19)

Europäisches
Patentamt
European
Patent Office
Office européen
des brevets

(11) **EP 3 332 799 A1**

(12) **EUROPEAN PATENT APPLICATION**

(43) Date of publication:
**13.06.2018 Bulletin 2018/24**

(21) Application number: **17190659.7**

(22) Date of filing: **20.04.2012**

(51) Int Cl.:
*A61K 38/29* (2006.01)     *A61P 19/10* (2006.01)
*A61K 9/20* (2006.01)     *A61M 37/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.04.2011  US 201161478466 P
20.12.2011  US 201161578120 P**

(62) Document number(s) of the earlier application(s) in
accordance with Art. 76 EPC:
**12773751.8 / 2 699 252**

(71) Applicants:
• **Radius Health, Inc.
Waltham, MA 02451 (US)**
• **3M Innovative Properties Company,
a wholly owned subsidiary of 3M company
Saint Paul, MN 55144-1000 (US)**

(72) Inventors:
• **HATTERSLEY, Gary
Stow, MA 01775 (US)**

• **HANSEN, Kris J.
Afton, MN 55001 (US)**
• **DETERMAN, Amy S.
Mahtomdedi, MN 55115 (US)**
• **ZHANG, Ying
Woodbury, MN 55125 (US)**

(74) Representative: **J A Kemp
14 South Square
Gray's Inn
London WC1R 5JJ (GB)**

Remarks:
•This application was filed on 12.09.2017 as a
divisional application to the application mentioned
under INID code 62.
•Claims filed after the date of filing of the
application/after the date of receipt of the divisional
application (Rule 68(4) EPC).

(54) **METHOD OF DRUG DELIVERY FOR PTH, PTHRP AND RELATED PEPTIDES**

(57)     The present invention provides compositions, devices, methods and processes related to the intradermal delivery of PTHrP and PTHrP analogues, particularly [Glu$^{22,25}$, Leu$^{23,28,31}$, Aib$_{29}$, Lys2$^{6,30}$]hPTHrP(1-34)NH$_2$.

**EP 3 332 799 A1**

**Description**

RELATED APPLICATIONS

This application claims the benefit of U.S. Provisional Application No. 61/478,466, filed April 22, 2011 and U.S. Provisional Application No. 61/578,120, filed December 20, 2011. The entire contents of the above applications are incorporated herein by reference.

BACKGROUND OF THE INVENTION

Parathyroid hormone-related protein ("PTHrP") is a 139 to 173 amino acid-protein. PTHrP, especially the C-terminal 1-36 secretory product and certain analogs, are known to be useful for the treatment of osteoporosis and related disorders by stimulating bone formation to improve bone mineral density (BMD). PTHrP analogues having excellent pharmacological properties and parenteral storage stable compositions thereof are described in Int. Publ. No. WO 2008/063279, the entire contents of which are hereby incorporated by reference. The effective delivery of PTHrP analogues by routes other than subcutaneous could provide potential advantages such as improved patient satisfaction and compliance.

One alternative to subcutaneous delivery is delivery by a microneedle or microprojection patch ("MNP") route. According to a standard definition, transdermal delivery refers to delivery of a drug substance across the skin. While certain types of drugs can be formulated and delivered using, for example, transdermal patches that allow for the passive diffusion of the drug across the skin, not all drugs perform well in the transdermal venue. One of the common reasons why a particular drug or class of drugs does not effectively penetrate through the skin to reach systemic circulation is the particular nature of the outermost skin layer.

The outermost skin layer in humans is called the stratum corneum and it is composed primarily of several layers of dead skin cells. The stratum corneum poses a formidable barrier to the transdermal delivery of a drug because unless the drug is capable of diffusing through the stratum corneum layer, it will not efficiently enter the circulation - the stratum corneum is not vascularized. As such, many large molecules or drugs of high water solubility cannot effectively diffuse through the stratum corneum, especially charged macromolecules such as peptides.

It is believed that treatments employing PTHrP analogues are most therapeutic if the pharmacokinetics are controlled, thereby achieving bone anabolic effects without losing efficacy of causing bone loss. As such, use of microprojection patches can result in complicated therapies if effective and reproducible coating of the microprojections is not achieved. Improved methods of delivering PTHrP analogues are needed.

SUMMARY OF THE INVENTION

One way for a drug, such as a peptide drug, to bypass the stratum corneum is to use small piercing elements to deliver a drug through the stratum corneum and place the drug into the intradermal space, sometimes referred to as intradermal delivery. For purposes of conveying meaning in the context of this invention description, the terms "transdermal" and "intradermal" are interchangeable when referring to the microprojection or microneedle assisted delivery of the PTHrP, PTHrP analogues including [Glu$^{22,25}$, Leu$^{23,28,31}$, Aib$^{29}$, Lys$^{26,30}$]PTHrP(1-34)NH$_2$. These small piercing elements can take the form of microprojections comprising various materials, shapes and dimensions. In some instances they can take the form of microneedles.

The present invention relates to drug formulations (e.g., aqueous formulations) comprising PTHrP and PTHrP analogues useful for coating microprojections for use in microprojection patch arrays, methods of coating microprojections and microprojection patch arrays, drug-coated microprojections and drug-coated microprojection patch arrays. The present invention also relates to the intradermal delivery of PTHrP and PTHrP analogues and methods of treating osteoporosis, osteopenia, fractured bones and osteoarthritis using transdermal delivery, for example, using drug-coated microprojections and microprojection arrays. In particular, the PTHrP analogue for use in embodiments of the invention is [Glu$^{22,25}$, Leu$^{23,28,31}$, Aib$^{29}$, Lys$^{26,30}$]PTHrP(1-34)NH$_2$.

The sequence of native hPTHrP (1-34) is as follows:

Ala Val Ser Glu His Gln Leu Leu His Asp Lys Gly Lys Ser Ile Gln Asp Leu Arg Arg Arg Phe Phe Leu His His Leu Ile Ala Glu Ile His Thr Ala (SEQ ID NO:1).

In a particular embodiment, the PTHrP analogue is [Glu$^{22,25}$, Leu$^{23,28,31}$, Aib$^{29}$, Lys$^{26,30}$]hPTHrP(1-34)NH$_2$ (SEQ ID NO.: 2).

In one aspect, the present invention relates to formulations containing PTHrP or PTHrP 1-34 analogues including [Glu$^{22,25}$, Leu$^{23,28,31}$, Aib$^{29}$, Lys$^{26,30}$]PTHrP(1-34)NH$_2$. In particular, these formulations are useful for coating one or more microprojections or a microprojection array including a microneedle patch array ("MNP") with said PTHrP or PTHrP 1-34 analogues including $[Glu^{22,25}, Leu^{23,28,31}, Aib^{29}, Lys^{26,30}]PTHrP(1-34)NH_2$. These formulations can be described by their contents including the percent of PTHrP or PTHrP analogue including $[Glu^{22,25}, Leu^{23,28,31}, Aib^{29}, Lys^{26,30}]PTHrP(1-34)NH_2$. The coating formulation refers to the formulation composition that is used to coat the microprojections. By way of a nonlimiting example in order to help understand the process and use of the described embodiments, a microprojection array comprises at least one but usually a plurality of microprojections that are typically affixed to a backing material and are coated by a formulation (e.g., an aqueous formulation) that contains a PTHrP analogue including $[Glu^{22,25}, Leu^{23,28,31}, Aib^{29}, Lys^{26,30}]PTHrP(1-34)NH_2$ at a defined by weight concentration. The percent by weight in the coating formulation is not typically the percent by weight in the drug delivery device as used since the coating formulation is designed to be useful for coating the drug onto the microprojections and then the coated microprojections are often subject to further processing (e.g. drying) and storage conditions that will likely affect the proportions of ingredients in the final composition. Where the array of microprojections or microneedles is affixed to a flexible backing material, that array is sometimes referred to as a microprojection patch array or microneedle patch array or simply microneedle patch. The microneedle patch may contain an adhesive material in order to facilitate its staying in place while the drug is released from the projections or needles of the patch.

In one embodiment of this invention, the formulation useful for coating one or more microprojections or a microprojection array is an aqueous formulation comprising at least 5% by weight of $[Glu^{22,25}, Leu^{23,28,31}, Aib^{29}, Lys^{26,30}]PTHrP(1-34)NH_2$. In a related embodiment, an aqueous formulation useful for coating one or more microprojections or a microprojection array comprising at least 10% by weight of $[Glu^{22,25}, Leu^{23,28,31}, Aib^{29}, Lys^{26,30}]PTHrP(1-34)NH_2$ is described. In yet other embodiments, an aqueous formulation useful for coating one or more microprojections or a microprojection array comprising at least 20%, or at least 30%, or at least 40%, or at least 45% by weight of $[Glu^{22,25}, Leu^{23,28,31}, Aib^{29}, Lys^{26,30}]PTHrP(1-34)NH_2$ is described. In certain embodiments of this invention, an aqueous formulation useful for coating one or more microprojections or a microprojection array comprising between 40% and 63% by weight $[Glu^{22,25}, Leu^{23,28,31}, Aib^{29}, Lys^{26,30}]PTHrP(1-34)NH_2$ is described. In certain embodiments of this invention, an aqueous formulation useful for coating one or more microprojections or a microprojection array comprising between 43% and 63% by weight $[Glu^{22,25}, Leu^{23,28,31}, Aib^{29}, Lys^{26,30}]PTHrP(1-34)NH_2$ is described.

In some embodiments of this invention, an aqueous formulation useful for coating one or more microprojections or a microprojection array comprising 5% to 15% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 12.5% to 20% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 15% to 60% by weight $[Glu^{22,25}, Leu^{23,28,31}, Aib^{29}, Lys^{26,30}]PTHrP(1-34)NH_2$ is described.

In some embodiments of this invention, an aqueous formulation useful for coating one or more microprojections or a microprojection array comprising 43%-48% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 46%-52% by weight $[Glu^{22,25}, Leu^{23,28,31}, Aib^{29}, Lys^{26,30}]PTHrP(1-34)NH_2$ is described.

In some embodiments of this invention, an aqueous formulation useful for coating one or more microprojections or a microprojection array comprising 40%-48% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 40%-46% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 40%-52% by weight $[Glu^{22,25}, Leu^{23,28,31}, Aib^{29}, Lys^{26,30}]PTHrP(1-34)NH_2$ is described.

In some embodiments of this invention, an aqueous formulation useful for coating one or more microprojections or a microprojection array comprising 50%-62% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 52%-60% by weight $[Glu^{22,25}, Leu^{23,28,31}, Aib^{29}, Lys^{26,30}]PTHrP(1-34)NH_2$ is described. In other embodiments of this invention, an aqueous formulation useful for coating one or more microprojections or a microprojection array comprising 54%-58% by weight $[Glu^{22,25}, Leu^{23,28,31}, Aib^{29}, Lys^{26,30}]PTHrP(1-34)NH_2$ is described.

In other embodiments of this invention, an aqueous formulation useful for coating one or more microprojections or a microprojection array comprises 54% by weight $[Glu^{22,25}, Leu^{23,28,31}, Aib^{29}, Lys^{26,30}]PTHrP(1-34)NH_2$ and 46% by weight PBS. In some embodiments of this invention, an aqueous formulation useful for coating one or more microprojections or a microprojection array comprises 58% by weight $[Glu^{22,25}, Leu^{23,28,31}, Aib^{29}, Lys^{26,30}]PTHrP(1-34)NH_2$ and 42% by weight PBS.

It should be appreciated that for purposes of describing this invention unless otherwise stated, the percent by weight of peptide such as [Glu$^{22,25}$, Leu$^{23,28,31}$, Aib$^{29}$, Lys$^{26,30}$]PTHrP(1-34)NH$_2$ refers to normalized peptide content and excludes the presence of various co-excipients, counterions, etc. Percent by weight refers to percent weight of peptide content over the total weight of the formulation being discussed. So for example, when a peptide is synthesized it may contain water, cosolvents (such as acetic acid), counter ions, water, etc. In order to adjust for batch to batch variance, it is preferred in the present context to refer to the pure peptide content meaning content exclusive of said additional cosolvents, counter ions, water, and other non-peptidic components.

In certain embodiments of this invention, the term "suitable for coating a microprojection array" means that the formulation is useful for coating a microprojection array. The term useful in this context means that the aqueous formulation is useful for coating the array in a manner that is consistent with that arrays eventual use in a mammal, preferably a human. The formulations may be coated on a microneedle or a microprojection array using various techniques known in the art such as dip-coating by dipping the array into a formulation, brushing a formulation onto an array, or applying aliquots of a formuation onto an array. Examples of coating microneedle arrays can be found, for example, in United States Patent Application Publication No. 2008/0051699.

The aqueous formulation comprising [Glu$^{22,25}$, Leu$^{23,28,31}$, Aib$^{29}$, Lys$^{26,30}$]PTHrP(1-34)NH$_2$ in any of the concentration ranges described may further comprise additional excipients. Additional excipients can include, for example, stabilizing agents, buffers and/or amphiphilic surfactants.

In some embodiments, one or more saccharides or polysaccharides are included as excipients in the aqueous formulation. In certain embodiments, the polysaccharide hydroxyethyl cellulose (HEC) is an added excipient. In another embodiment, the aqueous formulation comprises sucrose.

In some embodiments, buffered saline solutions are included in the aqueous formulation. Suitable buffered saline solutions include phosphate buffered saline (PBS), Tris buffered saline (TBS), saline-sodium acetate buffer (SSA), and saline-sodium citrate buffer (SSC). In one embodiment, the aqueous formulation comprising [Glu$^{22,25}$, Leu$^{23,28,31}$, Aib$^{29}$, Lys$^{26,30}$]PTHrP(1-34)NH$_2$ may further comprise phosphate buffered saline (PBS buffer). In one aspect of this embodiment, the PBS buffer used in the aqueous formulation has a pH of from 6.6 to 8.2. In another aspect of this embodiment, the PBS used in the aqueous formulation has a pH of from 6.8 to 8, or from 7.0 to 7.8, or from 7.2 to 7.6, or about 7.4, or 7.4. In yet another aspect of this embodiment, the PBS buffer is from 0.5X to 10X buffer concentration, or from 0.5X to 5X, or 1X. In a particular embodiment, the aqueous formulation useful for coating one or more microprojections or a microprojection array comprises [Glu$^{22,25}$, Leu$^{23,28,31}$, Aib$^{29}$, Lys$^{26,30}$]PTHrP(1-34)NH$_2$ and PBS as the sole excipient. In a specific aspect of this embodiment, the PBS has a 1X buffer concentration. In a more particular embodiment, the aqueous formulation useful for coating one or more microprojections or a microprojection array comprises [Glu$^{22,25}$, Leu$^{23,28,31}$, Aib$^{29}$, Lys$^{26,30}$]PTHrP(1-34)NH$_2$ and 1X PBS as the sole excipient wherein the peptide is present at about 50%-62% by weight, such as 52%-60% by weight such as 54%-58% by weight. In another particular embodiment, the aqueous formulation useful for coating one or more microprojections or a microprojection array comprises [Glu$^{22,25}$, Leu$^{23,28,31}$, Aib$^{29}$, Lys$^{26,30}$]PTHrP(1-34)NH$_2$ and 1X PBS as the sole excipient wherein the peptide is present at about 58% by weight and the PBS is present at about 42% by weight.

In certain embodiments of this invention, the microneedle coating formulations can be characterized by their final pH. One of ordinary skill in the art will appreciate that the pH of the final coating formulation can be different from the pH of the buffer used to co-formulate the peptide, such as [Glu$^{22,25}$, Leu$^{23,28,31}$, Aib$^{29}$, Lys$^{26,30}$]PTHrP(1-34)NH$_2$, especially when the peptide is highly concentrated and/or contains significant amounts of other pH-affecting co-solutes such acetic acid. In particular, the coating formulations of peptides such as [Glu$^{22,25}$, Leu$^{23,28,31}$, Aib$^{29}$, Lys$^{26,30}$]PTHrP(1-34)NH$_2$ may have pH values lower than the pH of the included buffer, such as an included PBS buffer. For example, some embodiments of the coating formulations of this invention may have a pH that falls between 3 and 8, or 3 and 7, or 3.5 and 6.5, or 4 and 6, or 4.5 and 5.5.

In some embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may further contain from 3% to 20% acetate present as the acetate ion and/or acetic acid by weight - in the aqueous coating formulation. In other embodiments, the [Glu$^{22,25}$, Leu$^{23,28,31}$, Aib$^{29}$, Lys$^{26,30}$]PTHrP(1-34)NH$_2$ may contain from 3% and 15% acetate present as the acetate ion and/or acetic acid by weight used in the aqueous formulation. In certain embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may contain from 4% and 10% acetate present as the acetate ion and/or acetic acid by weight in the aqueous formulation.

In certain embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may further contain from 1% to 15% trifluoroacetic acid present as the trifluoroacetate ion and/or trifluoroacetic acid by weight in the aqueous formulation. In other embodiments, the [Glu$^{22,25}$, Leu$^{23,28,31}$, Aib$^{29}$, Lys$^{26,30}$]PTHrP(1-34)NH$_2$ may further contain from 1% to 10% trifluoroacetic acid present as the trifluoroacetate ion and/or trifluoroacetic acid by weight in the aqueous formulation.

In certain embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may further contain from 1% to 15% histidine by weight in the aqueous formulation. In other embodiments, the [Glu$^{22,25}$, Leu$^{23,28,31}$, Aib$^{29}$, Lys$^{26,30}$]PTHrP(1-34)NH$_2$ may further contain from 1% to 10% histidine by weight in the aqueous formulation. In other embodiments, the [Glu$^{22,25}$, Leu$^{23,28,31}$, Aib$^{29}$, Lys$^{26,30}$]PTHrP(1-34)NH$_2$ may further contain from 1% to 7% histidine by weight in the aqueous formulation. In certain embodiments, the aqueous formulations suitable for the preparation of one or more drug-coated microprojections or drug-coated microprojection arrays may further contain 3% histidine or about 3% histidine. In some embodiments, the aqueous formulations suitable for the preparation of one or more drug-coated microprojections or drug-coated microprojection arrays may further contain 5% histidine or about 5% histidine. In some embodiments, the aqueous formulations suitable for the preparation of one or more drug-coated microprojections or drug-coated microprojection arrays may further contain 10% histidine or about 10% histidine.

In certain embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may further contain from 1% to 15% potassium chloride by weight in the aqueous formulation. In other embodiments, the [Glu$^{22,25}$, Leu$^{23,28,31}$, Aib$^{29}$, Lys$^{26,30}$]PTHrP(1-34)NH$_2$ may further contain from 2% to 10% potassium chloride by weight in the aqueous formulation. In some embodiments, the [Glu$^{22,25}$, Leu$^{23,28,31}$, Aib$^{29}$, Lys$^{26,30}$]PTHrP(1-34)NH$_2$ may further contain 9% potassium chloride by weight in the aqueous formulation. In certain embodiments, the [Glu$^{22,25}$, Leu$^{23,28,31}$, Aib$^{29}$, Lys$^{26,30}$]PTHrP(1-34)NH$_2$ may further contain about 9% potassium chloride by weight in the aqueous formulation.In certain embodiments, the [Glu$^{22,25}$, Leu$^{23,28,31}$, Aib$^{29}$, Lys$^{26,30}$]PTHrP(1-34)NH$_2$ used in the preparation of aqueous formulation solutions suitable for the preparation of one or more drug-coated microprojections or drug-coated microprojection arrays may further contain from 1% to 15% arginine by weight in the aqueous formulation. In other embodiments, the [Glu$^{22,25}$, Leu$^{23,28,31}$, Aib$^{29}$, Lys$^{26,30}$]PTHrP(1-34)NH$_2$ may further contain from 1% to 7% arginine by weight in the aqueous formulation. In certain embodiments, the aqueous formulations suitable for the preparation of one or more drug-coated microprojections or drug-coated microprojection arrays may further contain 3% arginine or about 3% arginine. In some embodiments, the aqueous formulations suitable for the preparation of one or more drug-coated microprojections or drug-coated microprojection arrays may further contain 5% arginine or about 5% arginine.

In some embodiments of this invention, the viscosity of the aqueous formulation comprising [Glu$^{22,25}$, Leu$^{23,28,31}$, Aib$^{29}$, Lys$^{26,30}$]PTHrP(1-34)NH$_2$ suitable for coating microprojections is between 500 centipoises and 10,000 centipoises at room temperature and a high shear rate. In additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 750 centipoises at room temperature and a high shear rate. In yet additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 1000 centipoises at room temperature and a high shear rate. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 2000 centipoises at room temperature and a high shear rate. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 10,000 centipoises at room temperature and a high shear rate.

As used herein, "room temperature" means a temperature in the the range from 20 °C to 25 °C, inclusive. In some aspects, the temperature is 23 °C or 25 °C. As used herein, "a high shear rate" means a shear rate equal to or greater than 100 s$^{-1}$. In some embodiments, the shear rate is 100 s$^{-1}$ or 128 s$^{-1}$.

In some embodiments of this invention, the viscosity of the aqueous formulation comprising [Glu$^{22,25}$, Leu$^{23,28,31}$, Aib$^{29}$, Lys$^{26,30}$]PTHrP(1-34)NH$_2$ suitable for coating microprojections is greater than 500 centipoises when measured at 23°C and a shear rate of 128 s$^{-1}$. In some embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 600 centipoises at 23°C and a shear rate of 128s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 700 centipoises at 23°C and a shear rate of 128s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 800 centipoises at 23°C and a shear rate of 128 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1000 centipoises at 23°C and a shear rate of 128 s$^{-1}$. In still yet additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1250 centipoises at 23°C and a shear rate of 128 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1500 at 23°C and a shear rate of 128 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 2500 at 23°C and a shear rate of 128s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 3500 at 23°C and a shear rate of 128s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 4500 at 23°C and a shear rate of 128s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 5500 at 23°C and a shear rate of 128s$^{-1}$. In yet still additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 750 centipoises at 23°C and a shear rate of 128s$^{-1}$. In yet additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 1000 centipoises at 23°C and a shear rate of 128s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 2000 centipoises at 23°C and a shear rate of 128s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 10,000 centipoises at 23°C and a shear rate of 128s$^{-1}$.

In some embodiments of this invention, the viscosity of the aqueous formulation comprising [Glu$^{22,25}$, Leu$^{23,28,31}$, Aib$^{29}$, Lys$^{26,30}$]PTHrP(1-34)NH$_2$ suitable for coating microprojections is greater than 500 centipoises when measured at 25°C and a shear rate of 100 s$^{-1}$. In some embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 600 centipoises at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 700 centipoises at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 800 centipoises at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1000 centipoises at 25°C and a shear rate of 100 s$^{-1}$. In still yet additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1250 centipoises at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 1500 at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 2500 at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 3500 at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 4500 at 25 °C and a shear rate of 100 s$^{-1}$. In certain embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is greater than 5500 at 25 °C and a shear rate of 100 s$^{-1}$. In yet still additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 750 centipoises at 25 °C and a shear rate of 100 s$^{-1}$. In yet additional embodiments of this invention, the viscosity of the formulation containing [Glu$^{22,25}$, Leu$^{23,28,31}$, Aib$^{29}$, Lys$^{26,30}$]PTHrP(1-34)NH$_2$ suitable for coating microprojections is between 500 centipoises and 1000 centipoises at 25 °C and a shear rate of 100 s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 2000 centipoises at 25 °C and a shear rate of 100 s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 2000 centipoises and 3000 centipoises at 25 °C and a shear rate of 100 s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 1000 centipoises and 3000 centipoises at 25 °C and a shear rate of 100 s$^{-1}$. In some embodiments of this invention, the viscosity of [Glu$^{22,25}$, Leu$^{23,28,31}$, Aib$^{29}$, Lys$^{26,30}$]PTHrP(1-34)NH$_2$ of the aqueous formulation for the coating of the microprojections is between 2000 centipoises and 2500 centipoises at 25 °C and a shear rate of 100 s$^{-1}$. In some embodiments of this invention, the viscosity of [Glu$^{22,25}$, Leu$^{23,28,31}$, Aib$^{29}$, Lys$^{21,30}$]PTHrP(1-34)NH$_2$ of the aqueous formulation for the coating of the microprojections is between 1000 centipoises and 10,000 centipoises at 25 °C and a shear rate of 100 s$^{-1}$.

The shear viscosity is a measurement of the resistance of a fluid to being deformed by shear stress. Various instruments can be used for viscosity testing, including rheometers, for example rheometers from TA Instruments (New Castle, DE).

In some aspects, the invention described herein relates to a drug delivery device comprising a microprojection array comprising a plurality of microprojections wherein one or more of said microprojections is coated with [Glu$^{22,25}$, Leu$^{23,28,31}$, Aib$^{29}$, Lys$^{26,30}$]PTHrP(1-34)NH$_2$, In certain embodiments of this invention, the microprojections are more than 100 microns but less than 1,000 microns in length. In certain embodiments of this invention, the microprojections are more than 250 microns but less than 750 microns in length. In some embodiments of this invention, the microprojections are between 400 and 600 microns in length. In certain embodiments, the microprojections are about 500 microns in length. In some embodiments, the microprojections are 500 microns in length.

In some embodiments of this invention, the [Glu$^{22,25}$, Leu$^{23,28,31}$, Aib$^{29}$, Lys$^{21,31}$]PTHrP(1-34)NH$_2$ coated microprojections are microneedles. For the purpose of this invention, the term microneedle means a microprojection that has a base and a tip wherein said tip has a lesser diameter, width, perimeter or circumference than said base. In one embodiment of this invention, the microneedles have a tapered design meaning that the microneedle from base to tip reflects a relatively constant narrowing over the length. In certain aspects of this invention, the microneedles have the greatest diameter, width, perimeter or circumference at the base compared to anywhere else on said microneedle. In certain embodiments of this invention, the ratio of the width at the base of the microneedle to the width at tip of the microneedle is greater than 2. In related embodiments, of this invention, the diameter, width, perimeter or circumference at the base of the microneedle to the diameter, width, perimeter or circumference at tip of the microneedle ratio is greater than 4. In related embodiments, of this invention, the diameter, width, perimeter or circumference at the base of the microneedle to the diameter, width, perimeter or circumference at tip of the microneedle ratio is greater than 6. In some embodiments, the needles have a generally circular perimeter about the axis that is broader at the base than the tip. In certain embodiments, the microneedles are pyramidal in shape, with an approximately rectangular base that tapers to an apex wherein said apex is approximately rectangular. In certain embodiments, the microneedles are pyramidal in shape, with a square base that tapers to an apex wherein said apex is approximately square. In certain embodiments, the microneedles are pyramidal in shape with a rectangular or square base and a shape that is not readily characterized as rectangular or square at the top.

In some embodiments of this invention, the microprojection array comprises a backing sheet or member wherein the plurality of microprojections are affixed to said backing sheet or member. In certain embodiments of this invention, the vertical axis of said microprojections extend at an angle of at least 45 degrees from the backing sheet or member. In certain embodiments, said microprojections extend at an angle of at least 60 degrees from the backing sheet or member. In some embodiments, the microprojections are perpendicular to said sheet or member. In certain embodiments, the microprojection arrays of this invention comprises a plurality of microprojections that are made from the same material as the backing sheet or member. In certain embodiments, the microneedle arrays of this invention comprises a plurality of microneedles that are made from the same material as the backing sheet or member. In some embodiments, the microprojection arrays of this invention comprises a plurality of microprojections that are integral with the backing sheet or member. In some aspects, the microprojection arrays of this invention comprises a plurality of microprojections that are made by an injection molding process. In certain embodiments, the microprojection arrays of this invention comprises a plurality of microprojections that are made from the same material as the backing sheet or member wherein said microprojection array is made by a molding process. In certain embodiments, the microneedle arrays of this invention comprises a plurality of microneedles that are made from the same material as the backing sheet or member wherein said microprojection array is made by an injection molding process.

In certain embodiments of this invention, the microprojections and/or microneedles are made from carbon containing polymers wherein said microprojections and/or needles can be defined according to their flexural modulus. In some embodiments, this invention comprises arrays comprising microprojections and/or microneedles coated with [Glu$^{22,25}$, Leu$^{23,28,31}$, Aib$^{29}$, Lys$^{26,30}$]PTHrP(1-34)NH$_2$ wherein said microprojections and/or microneedles are made from carbon containing polymers having a flexural modulus of greater than 1,000 MPa (ISO 178). In certain embodiments, this invention comprises arrays comprising microprojections and/or microneedles made from carbon containing polymers having a flexural modulus of greater than 2,000 MPa (ISO 178). In yet other embodiments, this invention comprises arrays comprising microprojections and/or microneedles made from carbon containing polymers having a flexural modulus of greater than 3,000 MPa (ISO 178). In yet other embodiments, this invention comprises arrays comprising microprojections and/or microneedles made from carbon containing polymers having a flexural modulus of between 3,000 MPa (ISO 178) and 15,000 MPa (ISO 178). In some embodiments, this invention comprises arrays comprising microprojections and/or microneedles made from carbon containing polymers having a flexural modulus of between 5,000 MPa (ISO 178) and 12,000 MPa (ISO 178). In some embodiments, this invention comprises arrays comprising microprojections and/or microneedles made from carbon containing polymers having a flexural modulus of between 8,000 MPa (ISO 178) and 12,000 MPa (ISO 178). In some embodiments, this invention comprises arrays comprising microprojections and/or microneedles made from carbon containing polymers having a flexural modulus of between 9,000 MPa (ISO 178) and 10,000 MPa (ISO 178).

As used herein, "ISO 178" refers to ISO test standards for determination of flexural properties of plastics.

One embodiment of this invention includes a microprojection array comprising a plurality of microneedles wherein one or more of said microneedles is coated with $[Glu^{22,25}, Leu^{23,28,31}, Aib^{29}, Lys^{26,30}]PTHrP(1-34)NH_2$ wherein said microprojection array has a density of needles of between 20 and 1,000 needles per $cm^2$. In certain embodiments of this invention, a microprojection array comprising a plurality of microneedles wherein one or more of said microneedles is coated with $[Glu^{22,25}, Leu^{23,28,31}, Aib^{29}, Lys^{26,30}]PTHrP(1-34)NH_2$ has a density of needles of between 100 and 500 needles per $cm^2$.

In some embodiments of this invention, a $[Glu^{22,25}, Leu^{23,28,31}, Aib^{29}, Lys^{21,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ contains between 50 and 600 microprojections. In certain embodiments of this invention, a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ contains between 100 and 500 microprojections is described. In certain embodiments, this invention includes a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ containing between 250 and 400 microprojections. In some embodiments, this invention comprises a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ and containing between 300 and 375 microprojections. In certain embodiments, this invention comprises a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ and containing about 366 microprojections. In some embodiments of this invention, a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ and containing 366 microprojections is described. In certain embodiments, this invention comprises a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ and containing about 316 microprojections. In certain embodiments, this invention comprises a $[Glu^{22,25}, Leu^{23,28,31}, Aib^{29}, Lys^{26,30}]PTHrP(1-34)NH_2$ coated microprojection array suitable for the intradermal delivery of an effective amount of $[Glu^{22,25}, Leu^{23,28,31}, Aib^{29}, Lys^{26,30}]PTHrP(1-34)NH_2$ and containing about 320 microprojections. In some embodiments, the microprojections are microneedles.

In some embodiments of this invention, the term "coated" means that one or more of the microprojections or microneedles of a microprojection array comprise $[Glu^{22,25}, Leu^{23,28,31}, Aib^{29}, Lys^{26,30}]PTHrP(1-34)NH_2$ on at least part of the surface of said microprojection or microneedle. In some embodiments, more than 1% and less than 50% of the total microprojections or microneedle surface area is coated by the aqueous formulation comprising $[Glu^{22,25}, Leu^{23,28,31}, Aib^{29}, Lys^{26,30}]PTHrP(1-34)NH_2$. In certain embodiments, more than 2% and less than 40% of the total microprojections or microneedle surface area is coated by the aqueous formulation comprising $[Glu^{22,25}, Leu^{23,28,31}, Aib^{29}, Lys^{26,31}]PTHrP(1-34)NH_2$. In certain embodiments, more than 5% and less than 35% of the total microprojections or microneedle surface area is coated by the aqueous formulation comprising $[Glu^{22,25}, Leu^{23,28,31}, Aib^{29}, Lys^{26,30}]PTHrP(1-34)NH_2$. In certain embodiments, more than 30% and less than 50% of the total microprojections or microneedle surface area is coated by the aqueous formulation comprising $[Glu^{22,25}, Leu^{23,28,31}, Aib^{29}, Lys^{26,30}]PTHrP(1-34)NH_2$. In certain embodiments, the aqueous formulation comprising $[Glu^{22,25}, Leu^{23,28,31}, Aib^{29}, Lys^{26,30}]PTHrP(1-34)NH_2$ coats from about 30% to about 50% of the top of the microprojections or microneedle (as used herein, "top" means the end of the microprojection or microneedle which would contact the skin).In the context of this description, the term total microprojections or microneedle surface area means the microprojections or microneedle surface area of all of the microprojections or microneedles present on a microprojections or microneedle array where said array comprises a plurality of microprojections or microneedles. In certain embodiments of this invention, said coated microprojections or microneedles are prepared by dipping an array comprising said microprojections or microneedles into an aqueous formulation comprising $[Glu^{22,25}, Leu^{23,28,31}, Aib^{29}, Lys^{26,30}]PTHrP(1-34)NH_2$ and then removing said array and allowing the array to dry. In some embodiments, accelerated drying conditions are applied to said array. In certain embodiments, said accelerated drying conditions include one or more of providing a circulating air flow, desiccants, vacuum and/or heat.

In some embodiments, this invention comprises a microprojection array comprising a plurality of $[Glu^{22,25}, Leu^{23,28,31}, Aib^{29}, Lys^{26,30}]PTHrP(1-34)NH_2$ coated microprojections wherein said microprojection array comprises at least 63.75 $\mu g$ of $[Glu^{22,25}, Leu^{23,28,31}, Aib^{29}, Lys^{26,30}]PTHrP(1-34)NH_2$. In some embodiments, this invention describes a microprojection array comprising a plurality of $[Glu^{22,25}, Leu^{23,28,31}, Aib^{29}, Lys^{26,30}]PTHrP(1-34)NH_2$ coated microprojections wherein said microprojection array comprises between 63.75 and 86.25 $\mu g$ of $[Glu^{22,25}, Leu^{23,28,31}, Aib^{29}, Lys^{26,30}]PTHrP(1-34)NH_2$. In some embodiments, this invention describes a microprojection array comprising a plurality of $[Glu^{22,25}, Leu^{23,28,31}, Aib^{29}, Lys^{26,30}]PTHrP(1-34)NH_2$ coated microprojections wherein said microprojection array comprises about 75 $\mu g$ of $[Glu^{22,25}, Leu^{23,28,31}, Aib^{29}, Lys^{26,30}]PTHrP(1-34)NH_2$. In certain embodiments, this invention describes a microprojection array comprising a plurality of $[Glu^{22,25}, Leu^{23,28,31}, Aib^{29}, Lys^{26,30}]PTHrP(1-34)NH_2$ coated

microprojections wherein said microprojection array comprises 75 $\mu$g of [Glu$^{22,25}$, Leu$^{23,28,31}$, Aib$^{29}$, Lys$^{26,30}$]PTHrP(1-34)NH$_2$. In some embodiments said microprojection array is a microneedle array.

In certain aspects, this invention comprises a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises at least 85 $\mu$g of [Glu$^{22,25}$, Leu$^{23,28,31}$, Aib$^{29}$, Lys$^{26,30}$]PTHrP(1-34)NH$_2$. In some embodiments, this invention describes an array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises between 85 $\mu$g and 115 $\mu$g of [Glu$^{22,25}$, Leu$^{23,28,31}$, Aib$^{29}$, Lys$^{26,30}$]PTHrP(1-34)NH$_2$, In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said array comprises about 100 $\mu$g of [Glu$^{22,25}$, Leu$^{23,28,31}$, Aib$^{29}$, Lys$^{26,30}$]PTHrP(1-34)NH$_2$. In certain embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises 100 $\mu$g of [Glu$^{22,25}$, Leu$^{23,28,31}$, Aib$^{29}$, Lys$^{26,30}$]PTHrP(1-34)NH$_2$. In some embodiments said microprojection array is a microneedle array. In certain aspects, this invention comprises a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said array comprises at least 106.25 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises between 106.25 $\mu$g and 143.75 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises about 125 $\mu$g of [Glu$^{22,25}$, Leu$^{23,28,31}$, Aib$^{29}$, Lys$^{26,30}$]PTHrP(1-34)NH$_2$. In certain embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said array comprises 125 $\mu$g of [Glu$^{22,25}$, Leu$^{23,28,31}$, Aib$^{29}$, Lys$^{26,30}$]PTHrP(1-34)NH$_2$. In some embodiments said microprojection array is a microneedle array.

In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises at least 127.5 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises between 127.5 $\mu$g and 172.5 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises about 150 $\mu$g of [Glu$^{22,25}$, Leu$^{23,28,31}$, Aib$^{29}$, Lys$^{26,31}$]PTHrP(1-34)NH$_2$. In certain embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises 150 $\mu$g of [Glu$^{22,25}$, Leu$^{23,28,31}$, Aib$^{29}$, Lys$^{26,30}$]PTHrP(1-34)NH$_2$. In some embodiments said microprojection array is a microneedle array.

In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises at least 170 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises between 170 $\mu$g and 230 $\mu$g of [Glu$^{22,25}$, Leu$^{23,28,31}$, Aib$^{29}$, Lys$^{26,30}$]PTHrP(1-34)NH$_2$. In some embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises about 200 $\mu$g of [Glu$^{22,25}$, Leu$^{23,28,31}$, Aib$^{29}$, Lys$^{26,30}$]PTHrP(1-34)NH$_2$. In certain embodiments, this invention describes a microprojection array comprising a plurality of [Glu$^{22,25}$, Leu$^{23,28,31}$, Aib$^{29}$, Lys$^{26,30}$]PTHrP(1-34)NH$_2$ coated microprojections wherein said microprojection array comprises 200 $\mu$g of [Glu$^{22,25}$, Leu$^{23,28,31}$, Aib$^{29}$, Lys$^{26,30}$]PTHrP(1-34)NH$_2$. In some embodiments said microprojection array is a microneedle array.

In some aspects of these embodiments, aqueous formulations comprising 5-15% [Glu$^{22,25}$, Leu$^{23,28,31}$, Aib$^{29}$, Lys$^{26,30}$]PTHrP(1-34)NH$_2$ are used to prepare a microprojection array comprising 20 $\mu$g of [Glu$^{22,25}$, Leu$^{22,28,31}$, Aib$^{29}$, Lys$^{26,30}$]PTHrP(1-34)NH$_2$. In other aspects of these embodiments, aqueous formulations comprising 12.5-20% [Glu$^{22,25}$, Leu$^{23,28,31}$, Aib$^{29}$, Lys$^{26,30}$]PTHrP(1-34)NH$_2$ are used to prepare a microprojection array comprising 40 $\mu$g of [Glu$^{22,25}$, Leu$^{23,28,31}$, Aib$^{29}$, Lys$^{26,30}$]PTHrP(1-34)NH$_2$. In some aspects of these embodiments, aqueous formulations comprising 15-60% [Glu$^{22,25}$, Leu$^{23,28,31}$, Aib$^{29}$, Lys$^{26,30}$]PTHrP(1-34)NH$_2$ are used to prepare a microprojection array comprising from 80 to 450 $\mu$g of [Glu$^{22,25}$, Leu$^{23,28,31}$, Aib$^{29}$, Lys$^{26,31}$]PTHrP(1-34)NH$_2$.

In some embodiments, this invention comprises a method of treating osteoporosis in a subject in need thereof comprising the less than daily administration of a microprojection array comprising one or more [Glu$^{22,25}$, Leu$^{23,28,31}$, Aib$^{29}$, Lys$^{26,30}$]PTHrP(1-34)NH$_2$ coated microprojections wherein said administration comprises contacting one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of one or more [Glu$^{22,25}$, Leu$^{23,28,31}$, Aib$^{29}$, Lys$^{26,30}$]PTHrP(1-34)NH$_2$ coated microprojections into the skin. For example, it is believed that an anabolic effect on bone could be achieved by a once per every two days application, once per every three days application, or even a once per week application.

In some embodiments, this invention comprises a method of treating osteoporosis in a subject in need thereof comprising the daily administration of a microprojection array comprising one or more [Glu,$^{22,25}$, Leu$^{23,28,31}$, Aib$^{29}$, Lys$^{26,30}$]PTHrP(1-34)NH$_2$ coated microprojections wherein said administration comprises contacting one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of one or more [Glu$^{22,25}$, Leu$^{23,28,31}$, Aib$^{29}$, Lys$^{21,30}$]PTHrP(1-34)NH$_2$ coated microprojections into the skin. In certain embodiments, the array is left in place with one or more microprojections embedded in the subject's skin for a period of more than 10 minutes and less than 1 hour. In some embodiments, the array is left in place with one or more microprojections embedded in the subject's skin for a period of from 10 minutes to 30 minutes. In certain embodiments, the array is left in place with one or more microprojections embedded in the subject's skin for a period of about 15 minutes. In certain embodiments, the array is left in place with one or more microprojections embedded in the subject's skin for a period of 15 minutes. In some embodiments said microprojection array is a microneedle array.

In certain embodiments, this invention comprises a method of treating osteoporosis in a subject in need thereof comprising the daily administration of a microprojection array comprising one or more [Glu$^{22,25}$, Leu$^{23,28,31}$, Aib$^{29}$, Lys$^{21,30}$]PTHrP(1-34)NH$_2$ coated microprojections wherein said administration comprises contacting the one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of said one or more [Glu$^{22,25}$, Leu$^{23,28,31}$, Aib$^{29}$, Lys$^{26,30}$]PTHrP(1-34)NH$_2$ coated microprojections into the skin. In certain embodiments, the array is left in place with one or more microprojections embedded in the subject's skin for a period of from 3 seconds to 10 minutes. In certain embodiments, the microprojection array is left in place with one or more microprojections embedded in the subject's skin for a period of from 3 seconds to 5 minutes. In certain embodiments, the microprojection array is left in place with one or more microprojections embedded in the subject's skin for a period of from 5 seconds to 3 minutes. In certain embodiments, the microprojection array is left in place with one or more microprojections embedded in the subject's skin for a period of from 5 seconds to 1 minute. In some embodiments, the microprojection array is left in place with one or more microprojections embedded in the subject's skin for a period of from 5 seconds to 30 seconds. In certain preferred embodiments, the microprojection array is left in place with one or more microprojections embedded in the subject's skin for a period of about 15 minutes. In some preferred embodiments, the microprojection array is left in place with one or more microprojections embedded in the subject's skin for a period of about 5 minutes. In other preferred embodiments, the microprojection array is left in place with one or more microprojections embedded in the subject's skin for a period of about 1 minute. In some embodiments, the microprojection array is left in place with one or more microprojections embedded in the subject's skin for a period of about 30 seconds. In certain embodiments, the microprojection array is left in place with one or more microprojections embedded in the subject's skin for a period of about 15 seconds. In certain embodiments, the microprojection array is left in place with one or more microprojections embedded in the subject's skin for a period of about 10 seconds. In certain embodiments, the microprojection array is left in place with one or more microprojections embedded in the subject's skin for a period of about 5 seconds. In certain embodiments, the microprojection array is left in place with said microprojections embedded in the subject's skin for a period of 5, 10 or 15 seconds, 30 seconds, 1 minute, 5 minutes, 10 minutes, 15 minutes or 30 minutes. In some embodiments, the microprojection array is fixed in place for the duration of their residency time on the subject's skin. In certain embodiments, the microprojection array is fixed in place by the presence of an adhesive material on the microprojection array such that the adhesive material adheres to the subject's skin and the microprojection array thereby reducing the possibility that the microprojection array will move substantially during its residency time on said subject's skin. In some embodiments said microprojection array is a microneedle array.

In some embodiments, the administration of [Glu$^{22,25}$, Leu$^{23,28,31}$, Aib$^{29}$, Lys$^{26,30}$]PTHrP(1-34)NH$_2$ by microprojection array is applied with sufficient force to cause one or more of said microprojections to penetrate the subject's skin to a depth of at least 50 micrometers. In some embodiments, the administration of [Glu$^{22,25}$, Leu$^{23,28,31}$, Aib$^{29}$, Lys$^{26,30}$]PTHrP(1-34)NH$_2$ by microprojection array is applied with sufficient force to cause one or more of said microprojections to penetrate the subject's skin to a depth of at least 100 micrometers. In some embodiments, the administration of [Glu$^{22,25}$, Leu$^{23,28,31}$, Aib$^{29}$, Lys$^{26,30}$]PTHrP(1-34)NH$_2$ by microprojection array is applied with sufficient force to cause one or more of said microprojections to penetrate the subject's skin to a depth of at least 200 micrometers.

In certain embodiments, the force applied to the array is applied manually wherein said array is held in the administering person's hand, who may or may not be the person receiving the drug, and applied to the site of administration. In some embodiments, the force applied to the array is applied manually to an applicator wherein said applicator is affixed to the array. In certain embodiments, said applicator is capable of storing a fixed force and said force can be released to the array with sufficient energy to administer the drug in accordance with the principles of this invention. In some embodiments, the microprojection array is applied using force by discharging a spring-loaded applicator. Applicators suitable for the administration of microprojection arrays in accordance with the methods of this invention are known to those of ordinary skill in the art. For example, suitable applicators are described in U.S. Patent Application Publications No. 2009/0198189 and 2005/0096586, the entire contents of each of which are herein incorporated by reference.

In certain embodiments, the drug-coated microprojection arrays described herein are useful for the treatment of osteoporosis. In some embodiments, the drug coated microprojection arrays described herein are useful for the treatment of postmenopausal osteoporosis. In certain embodiments, the drug coated arrays described herein are useful for the treatment of glucocorticoid induced osteoporosis in men or women. In certain embodiments, the methods of treating osteoporosis described herein can be applied to a patient or patient population characterized as being at an elevated risk for bone fracture. In some embodiments, said patient or patient population can be characterized as having bone mineral density at one or more skeletal sites of >1 standard deviation below the norm. In some embodiments, the methods of treating osteoporosis described herein can be applied to a patient or patient population characterized by bone mineral density at one or more skeletal sites of >2 standard deviations below the norm. In some embodiments, the methods of treating osteoporosis described herein can be applied to a patient or patient population characterized by bone mineral density at one or more skeletal sites of >2.5 standard deviations below the norm. In some embodiments, the methods of treating osteoporosis described herein can be applied to a patient or patient population characterized by bone mineral density at one or more skeletal sites of >3 standard deviations below the norm. In certain embodiments, the methods of treating osteoporosis described herein can be applied to patients who have had one or more previous bone fractures. Where said patient has had one or more prior fractures they may also present with a bone mineral density at or below the mean, for example, said patient may have bone mineral density at one or more sites that is at least 1 standard deviations below the mean, or at least 2 standard deviations below the mean, or at least 2.5 standard deviations below the mean or at least 3 standard deviations below the mean. In addition, the methods of treating osteoporosis described herein may be applied to any patient at potentially increased risk of fracture wherein said patient may have one or more characteristics that identify them as being at increased risk such as smoking, consumption of alcohol, use of glucocorticoids, use of tricyclic antidepressants, are at increased risk of falling, have asthma, chronic liver disease, rheumatoid arthritis, type 2 diabetes, endocrine problems, familial history of fractures, poor nutrition or nutritional disorders.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with 75 μg or about 75 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with from 85 μg to 115 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with 100 μg or about 100 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with from 106.25 μg to 143.75 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with 125 μg or about 125 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with from 127.5 μg to 172.5 μg of [$Glu^{22,25}$, $Leu^{23,28,31}$, $Aib^{29}$, $Lys^{26,30}$]PTHrP(1-34)NH$_2$,

In some embodiments, this invention includes a method of treating osteoporosis comprising daily administration of a microprojection array comprising a plurality of [$Glu^{22,25}$, $Leu^{23,28,31}$, $Aib^{29}$, $Lys^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with 150 μg or about 150 μg of [$Glu^{22,25}$, $Leu^{23,28,31}$, $Aib^{29}$, $Lys^{26,30}$]PTHrP(1-34)NH$_2$.

In some embodiments, this invention includes a method of treating osteoporosis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with from 170 μg to 230 μg of [Glu$^{22,25}$, Leu$^{23,28,31}$, Aib$^{29}$, Lys$^{26,30}$]PTHrP(1-34)NH$_2$.

In some embodiments, this invention includes a method of treating osteoporosis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with 200 μg or about 200 μg of [Glu$^{22,25}$, Leu$^{23,28,31}$, Aib$^{29}$, Lys$^{26,30}$]PTHrP(1-34)NH$_2$.

In certain therapeutic arenas, the drug coated arrays of this invention are useful for improving the healing process in people who have suffered from one or more fractures or breaks of one or more bones in their bodies, including either vertebral fractures or non-vertebral fractures (for example, hip or femur fractures). Such improvement is evidenced by an increase in fracture healing rate and/or quality of bone associated with the fractured site and/or patient-reported symptomatic outcomes including such indices of fracture healing such as reduced discomfort, increased flexibility and/or mobility and/or strength. People who have suffered a bone fracture may or may not suffer from concomitant low bone mineral density, but they can benefit from the increased rate of bone formation that the use of the drug coated arrays of this invention can provide. In certain embodiments of this invention, the dosages and administration schedules as described herein for preventing or treating osteoporosis are useful for improving the fracture healing process in people who have experienced bone fractures. In some embodiments, the methods for improving the healing process in people who have suffered from one or more fractures or breaks of one or more bones in their bodies described herein can be applied to a patient with one or more vertebral fractures. In some embodiments, the methods for improving the healing process in people who have suffered from one or more fractures or breaks of one or more bones in their bodies described herein can be applied to a patient with one or more femoral fractures. In some embodiments, the methods for improving the healing process in people who have suffered from one or more fractures or breaks of one or more bones in their bodies described herein can be applied to a patient with one or more radial fractures.

In some embodiments of this invention, a drug coated microprojection array is applied twice daily, or once daily, or once every two days, once every three days or once per week. Therefore, in some embodiments of this invention, a drug coated microprojection array is applied once per day wherein said array is coated with an amount of drug deemed useful for the indication with the amount recommended being those amounts that are useful for preventing or treating osteoporosis as has been otherwise described in this specification. Said daily applications can begin any time after a fracture is detected. In some embodiments, the application of the drug coated microprojection arrays of this invention is started no later than 6 months after a fracture has occurred or is detected. In certain embodiments, said application is started no later than 3 months after a fracture has occurred or is detected. In some embodiments, said application is started no later than 1 month after a fracture has occurred or is detected. In some embodiments, said application is started no later than 2 weeks after a fracture has occurred or is detected. In certain embodiments, said application is started no later than 1 week after a fracture has occurred or is detected. It is recommended that to most effectively utilize the method of treating people with one or more fractured bones is for that treatment to begin soon after a fracture is detected. It should be appreciated that the duration of treatment is contingent upon a number of variables including the extent of the injury, the location of the injury, the rate and degree of recovery, the patient's overall bone health including bone mineral density at other anatomical sites, the discretion of the treating physician and more. Therefore, the treatment of fracture can vary from as little as one or a few once-daily applications up to one or even more than one year of once-daily applications. In some embodiments, the treatment period will be at least 1 application of a drug coated microprojection array as described in this invention. In certain embodiments, the treatment period will be at least one week of once-daily applications. In some embodiments, the treatment period will be at least two weeks of once-daily applications. In some embodiments, the treatment period will be at least four weeks of once-daily applications. In certain embodiments, the treatment period will be at least eight weeks of once-daily applications. In some embodiments, the treatment period will be at least twelve weeks of once-daily applications. In certain embodiments, the treatment period will be at least twenty four weeks of once-daily applications. In some embodiments, the treatment period will be at least one year of once-daily applications.

In certain embodiments, this invention comprises a method of treating fractures or accelerating fracture healing in a subject in need thereof comprising the daily administration of a microprojection array comprising one or more [Glu$^{22,25}$, Leu$^{23,28,31}$, Aib$^{29}$, Lys$^{26,30}$]PTHrP(1-34)NH$_2$ coated microprojections wherein said administration comprises contacting the one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of said one or more [Glu$^{22,25}$, Leu$^{23,28,31}$, Aib$^{29}$, Lys$^{26,30}$]PTHrP(1-34)NH$_2$ coated microprojections into the skin. In certain embodiments, the array is left in place with one or more microprojections embedded in the subject's skin for a period of from 3 seconds to 10 minutes. In certain embodiments, the microprojection array is left in place with one or more microprojections embedded in the subject's skin for a period of from 3 seconds to 5 minutes. In certain embodiments, the microprojection array is left in place with one or more microprojections embedded in the subject's skin for a period of from 5 seconds to 3 minutes. In certain embodiments, the microprojection array is left in place with one or more microprojections embedded in the subject's skin for a period of from 5 seconds to 1 minute. In some embodiments, the microprojection array is left in place with one or more microprojections embedded in the subject's skin for a period of from 5 seconds to 30 seconds. In certain embodiments, the microprojection array is left in place with one or more microprojections embedded in the subject's skin for a period of about 15 minutes. In certain embodiments, the microprojection array is left in place with one or more microprojections embedded in the subject's skin for a period of about 5 minutes. In some embodiments, the microprojection array is left in place with one or more microprojections embedded in the subject's skin for a period of about 1 minute. In some embodiments, the microprojection array is left in place with one or more microprojections embedded in the subject's skin for a period of about 30 seconds. In certain embodiments, the microprojection array is left in place with one or more microprojections embedded in the subject's skin for a period of about 15 seconds. In certain embodiments, the microprojection array is left in place with one or more microprojections embedded in the subject's skin for a period of about 10 seconds. In certain embodiments, the microprojection array is left in place with one or more microprojections embedded in the subject's skin for a period of about 5 seconds. In certain embodiments, the microprojection array is left in place with said microprojections embedded in the subject's skin for a period of 5, 10 or 15 seconds, 30 seconds, 1 minute, 5 minutes, 10 minutes, 15 minutes or 30 minutes. In some embodiments, the microprojection array is fixed in place for the duration of their residency time on the subject's skin. In certain embodiments, the microprojection array is fixed in place by the presence of an adhesive material on the microprojection array such that the adhesive material adheres to the subject's skin and the microprojection array thereby reducing the possibility that the microprojection array will move substantially during its residency time on said subject's skin.

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{26,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with about 75 $\mu$g of [$Glu^{22,25}$, $Leu^{23,28,31}$, $Aib^{29}$, $Lys^{26,30}$]PTHrP(1-34)$NH_2$,

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{26,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with between 85 $\mu$g and 115 $\mu$g of [$Glu^{22,25}$, $Leu^{23,28,31}$, $Aib^{29}$, $Lys^{26,30}$]PTHrP(1-34)$NH_2$.

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{26,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with about 100 $\mu$g of [$Glu^{22,25}$, $Leu^{23,28,31}$, $Aib^{29}$, $Lys^{26,30}$]PTHrP(1-34)$NH_2$.

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{26,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with between 106.25 $\mu$g and 143.75 $\mu$g of [$Glu^{22,25}$, $Leu^{23,28,31}$, $Aib^{29}$, $Lys^{26,30}$]PTHrP(1-34)$NH_2$,

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{26,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with about 125 $\mu$g of [$Glu^{22,25}$, $Leu^{23,28,31}$, $Aib^{29}$, $Lys^{26,31}$]PTHrP(1-34)$NH_2$.

In some embodiments, this invention includes a method of treating fractures or accelerating fracture healing in a subject comprising daily administration of a microprojection array comprising a plurality of [$Glu^{22,25}$, $Leu^{23,28,31}$, $Aib^{29}$, $Lys^{36,30}$]PTHrP(1-34)$NH_2$ coated microprojections to a subject in need thereof wherein said administration comprises contacting one or more of said microprojections of the microprojection array with sufficient force to penetrate the subject's skin and wherein said microprojections are coated with between 127.5 $\mu$g and 172.5 $\mu$g of [$Glu^{22,25}$, $Leu^{23,28,31}$, $Aib^{29}$, $Lys^{26,30}$]PTHrP(1-34)$NH_2$,

In some embodiments, this invention includes a method of treating fractures or accelerating fracture healing in a subject comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 150 μg of [Glu$^{22,25}$, Leu$^{23,28,31}$, Aib$^{29}$, Lys$^{26,30}$]PTHrP(1-34)NH$_2$,

In some embodiments, this invention includes a method of treating fractures or accelerating fracture healing in a subject comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 170 μg and 230 μg of [Glu$^{22,25}$, Leu$^{23,28,31}$, Aib$^{29}$, Lys$^{26,30}$]PTHrP(1-34)NH$_2$.

In some embodiments, this invention includes a method of treating fractures or accelerating fracture healing in a subject comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with about 200 μg of [Glu$^{22,25}$, Leu$^{23,28,31}$, Aib$^{29}$, Lys$^{26,30}$]PTHrP(1-34)NH$_2$.

In some embodiments, the microprojection arrays useful for the method of treating fractures or accelerating fracture healing in a subject comprise microneedles.

The drug-coated microprojection or microneedle arrays of this invention may also be used for the prevention and/or treatment of osteoarthritis. It is recognized that osteoarthritis is accompanied by the loss of cartilage, particularly at the joints. In some cases, the lost cartilage is replaced by bone or bony deposits. The drug coated microprojection arrays of this invention provide methods of treating people with agents that promote the bone remodeling process possibly including the increased production of cartilage and/or the diminution of bony deposits through acceleration of a normal bone remodeling process. Increasing the amount of cartilage in worn joints can have a laudatory effect on the individual measurable by numerous quality of life improvements including decreased pain and increased freedom of motion around the affected joint. The method of treating an individual suffering from osteoarthritis will typically comprise the administration of a drug coated microprojection or microneedle array of this invention typically in a once per day setting. The dosages applied will be typically the same as those dosages that are useful for the prevention and/or treatment of osteoporosis as described herein. Since the signs and symptoms of osteoarthritis are often different than osteoporosis, the treatment of osteoarthritis by the arrays of this invention will take that into account. In particular, while it is envisioned that a once daily administration of the arrays of this invention will remain an important choice, the duration of treatment including the adjudication of a successful outcome will be different. In particular, whereas the effect of an osteoporosis treatment can be readily ascertained by acute temporal effects on bone mineral density and reduction in fracture risk, the effect of treatment for osteoarthritis can be most readily detected via a patient reported reduction of symptoms. In this regard, the treatment of osteoarthritis can be started upon the observation of one or more symptoms of osteoarthritis and may be continued for a time sufficient for the diminution or elimination of one or more of the observed symptoms. Alternatively, the patient can have their treatment monitored by X-ray analysis of the affected joint(s) and the X-ray images interpreted by a qualified examiner in order to help determine if the treatment is having the desired effect. Due to the complexity of osteoarthritis and the ambiguity of correlating X-ray images with patient perception of pain or affected movement, the patient together with their medical practitioner will often decide together whether the treatment regimen is working or whether it should be adjusted.

In certain embodiments of this invention, the drug coated microprojection or microneedle arrays are applied once daily for a time sufficient to achieve a satisfactory reduction in symptoms such as pain, inflammation, swelling and edema. In some embodiments, the drug coated microprojection arrays are applied once daily for a period of at least one week. In certain embodiments, the drug coated microprojection arrays are applied once daily for a period of at least two weeks. In some embodiments, the treatment period will be at least four weeks of once-daily applications. In certain embodiments, the treatment period will be at least eight weeks of once-daily applications. In some embodiments, the treatment period will be at least twelve weeks of once-daily applications. In certain embodiments, the treatment period will be at least twenty four weeks of once-daily applications. In some embodiments, the treatment period will be at least one year of once-daily applications. Regardless of the length of any course of treatments, it should be appreciated that retreatment can be commenced if the symptoms return or worsen or if other indices of the disease indicate that an additional round of treatment could be beneficial.

In certain embodiments, this invention comprises a method of treating osteoarthritis in a subject in need thereof comprising the daily administration of a microprojection array comprising one or more [Glu$^{22,25}$, Leu$^{23,28,31}$, Aib$^{29}$, Lys$^{26,30}$]PTHrP(1-34)NH$_2$ coated microprojections wherein said administration comprises contacting the one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of said one or more [Glu$^{22,25}$,Leu$^{23,28,31}$, Aib$^{29}$, Lys$^{26,30}$]PTHrP(1-34)NH$_2$ coated

microprojections into the skin. In certain embodiments, the array is left in place with one or more microprojections embedded in the subject's skin for a period of from 3 seconds to 10 minutes. In certain embodiments, the microprojection array is left in place with one or more microprojections embedded in the subject's skin for a period of from 3 seconds to 5 minutes. In certain embodiments, the microprojection array is left in place with one or more microprojections embedded in the subject's skin for a period of from 5 seconds to 3 minutes. In certain embodiments, the microprojection array is left in place with one or more microprojections embedded in the subject's skin for a period of from 5 seconds to 1 minute. In some embodiments, the microprojection array is left in place with one or more microprojections embedded in the subject's skin for a period of from 5 seconds to 30 seconds. In certain embodiments, the microprojection array is left in place with one or more microprojections embedded in the subject's skin for a period of about 15 minutes. In certain embodiments, the microprojection array is left in place with one or more microprojections embedded in the subject's skin for a period of about 5 minutes. In some embodiments, the microprojection array is left in place with one or more microprojections embedded in the subject's skin for a period of about 1 minute. In some embodiments, the microprojection array is left in place with one or more microprojections embedded in the subject's skin for a period of about 30 seconds. In certain embodiments, the microprojection array is left in place with one or more microprojections embedded in the subject's skin for a period of about 15 seconds. In certain embodiments, the microprojection array is left in place with one or more microprojections embedded in the subject's skin for a period of about 10 seconds. In certain embodiments, the microprojection array is left in place with one or more microprojections embedded in the subject's skin for a period of about 5 seconds. In certain embodiments, the microprojection array is left in place with said microprojections embedded in the subject's skin for a period of 5, 10 or 15 seconds, 30 seconds, 1 minute, 5 minutes, 10 minutes, 15 minutes or 30 minutes. In some embodiments, the microprojection array is fixed in place for the duration of their residency time on the subject's skin. In certain embodiments, the microprojection array is fixed in place by the presence of an adhesive material on the microprojection array such that the adhesive material adheres to the subject's skin and the microprojection array thereby reducing the possibility that the microprojection array will move substantially during its residency time on said subject's skin.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with about 75 $\mu$g of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 85 $\mu$g and 115 $\mu$g of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with about 100 $\mu$g of [Glu$^{22,25}$, Leu$^{23,28,31}$, Aib$^{29}$, Lys$^{26,30}$]PTHrP(1-34)NH$_2$,

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 106.25 $\mu$g and 143.75 $\mu$g of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with about 125 $\mu$g of [Glu$^{22,25}$, Leu$^{23,28,31}$, Aib$^{29}$, Lys$^{26,30}$]PTHrP(1-34)NH$_2$,

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 127.5 $\mu$g and 172.5 $\mu$g of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 150 μg of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with between 170 μg and 230 μg of [Glu$^{22,25}$, Leu$^{23,28,31}$, Aib$^{29}$, Lys$^{26,30}$]PTHrP(1-34)NH$_2$.

In some embodiments, this invention includes a method of treating osteoarthritis comprising daily administration of a microprojection array comprising a plurality of [Glu$^{22,25}$, Leu$^{23,28,31}$, Aib$^{29}$, Lys$^{26,30}$]PTHrP(1-34)NH$_2$ coated microprojections to a subject in need thereof wherein said administration comprises contacting one or more of said microprojections of the microprojection array with sufficient force to penetrate the subject's skin and wherein said microprojections are coated with about 200 μg of [Glu$^{22,25}$, Leu$^{23,28,31}$, Aib$^{29}$, Lys$^{26,30}$]PTHrP(1-34)NH$_2$.

In some embodiments, the microprojection arrays useful for the treating osteoarthritis comprise microneedles.

In some embodiments, this invention comprises a method of increasing bone mineral density in a subject in need thereof comprising the administration of a microprojection array comprising one or more [Glu$^{22,25}$, Leu$^{23,28,31}$, Aib$^{29}$, Lys$^{26,30}$]PTHrP(1-34)NH$_2$ coated microprojections wherein said administration comprises contacting one or more of said [Glu$^{22,25}$, Leu$^{23,28,31}$, Aib$^{29}$, Lys$^{26,30}$]PTHrP(1-34)NH$_2$ coated microprojections with the skin of the subject using sufficient force to cause penetration of one or more [Glu$^{22,25}$, Leu$^{23,28,31}$, Aib$^{29}$, Lys$^{26,30}$]PTHrP(1-34)NH$_2$ coated microprojections into the skin. For example, it is believed that an anabolic effect on bone could be achieved by a once per every two days application, once per every three days application, or even a once per week application. In particular embodiments, a method of increasing bone mineral density in a subject in need thereof comprise the administration of a microprojection array comprising one or more [Glu$^{22,25}$, Leu$^{23,28,31}$, Aib$^{29}$, Lys$^{26,30}$]PTHrP(1-34)NH$_2$ coated microprojections in doses and dosing schedules as set forth herein for the treatment of osteoporosis, and/or for treating fractures or accelerating fracture healing, and/or for the treatment of osteoarthritis.

## BRIEF DESCRIPTION OF THE DRAWINGS

FIG. 1 is a graph comparing a representative microneedle array pharmacokinetics (PK) profile (09RAD010 Group 1), adjusted to a 20 μg/kg dose, graphed together with the reference subcutaneous (SC) profile.

FIG. 2 is an image of a liquid crystal polymer (LCP) microarray.

FIG. 3 is a side view with dimensions of the microstructures of the LCP array.

FIG. 4 is a graph showing the mean concentrations of [Glu$^{22,25}$, Leu$^{23,28,31}$, Aib$^{29}$, Lys$^{26,30}$]hPTHrP(1-34)NH$_2$ in serum versus time after a single microneedle array application (155342-041, 124 μg).

FIG. 5 is a graph showing the mean concentrations of [Glu$^{22,25}$, Leu$^{23,28,31}$, Aib$^{29}$, Lys$^{26,30}$]hPTHrP(1-34)NH$_2$ in serum versus time after single microneedle array application (155342-016, 103 μg).

FIG. 6 is a graph showing the mean concentrations of [Glu$^{22,25}$, Leu$^{23,28,31}$, Aib$^{29}$, Lys$^{26,30}$]hPTHrP(1-34)NH$_2$ (ng/mL) in serum after a single microneedle array application (155342-064, 56 μg).

FIG. 7 is a graph showing the mean concentrations of [Glu$^{22,25}$, Leu$^{23,28,31}$, Aib$^{29}$, Lys$^{26,30}$]hPTHrP(1-34)NH$_2$ in serum after a single microneedle array application (155342-033, 211 μg).

FIG. 8 is a graph showing the mean concentrations of [Glu$^{22,25}$, Leu$^{23,28,31}$, Aib$^{29}$, Lys$^{26,30}$]hPTHrP(1-34)NH$_2$ (ng/mL) in serum after single microneedle array application (152986-035, 13.6 μg).

FIG 9 is a figure showing change in femoral metaphysis bone mineral density in the osteopenic rat following repeat application of [Glu$^{22,25}$, Leu$^{23,28,31}$, Aib$^{29}$, Lys$^{26,30}$]hPTHrP(1-34)NH$_2$-microneedle arrays

FIG 10 is a figure showing change in lumbar spine bone mineral density in the osteopenic rat following repeat application of [Glu$^{22,25}$, Leu$^{23,28,31}$, Aib$^{29}$, Lys$^{26,30}$]hPTHrP(1-34)NH$_2$-microneedle arrays

FIG. 11 is a graph comparing plasma exposure levels of [Glu$^{22,25}$, Leu$^{23,28,31}$, Aib$^{29}$, Lys$^{26,30}$]hPTHrP(1-34)NH$_2$ in pG/mL after periumbilical application with 100 μg array (15 minute contact and 10 second contact time) and 80 μg subcutaneous administration of [Glu$^{22,25}$, Leu$^{23,28,31}$, Aib$^{29}$, Lys$^{26,30}$]hPTHrP(1-34)NH$_2$.

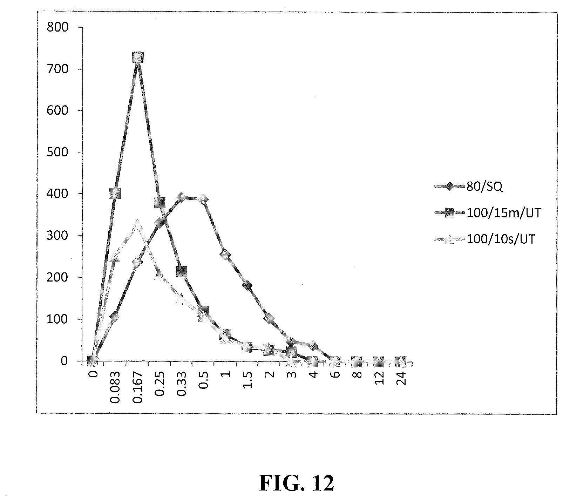

FIG. 12 is a graph comparing plasma exposure levels of [Glu$^{22,25}$, Leu$^{23,28,31}$, Aib$^{29}$, Lys$^{26,30}$]hPTHrP(1-34)NH$_2$ in pg/mL after upper thigh application with 100 μg array (15 minute contact and 10 second contact time) and 80 μg subcutaneous administration of [Glu$^{22,25}$, Leu$^{23,28,31}$, Aib$^{29}$, Lys$^{26,30}$]hPTHrP(1-34)NH$_2$.

FIG. 13 is a graph showing mean change from baseline collagen type 1 cross-linked C-telopeptide (CTX) concentrations following Glu$^{22,25}$, Leu$^{23,28,31}$, Aib$^{29}$, Lys$^{26,30}$]hPTHrP(1-34)NH$_2$ study groups and placebo on days 1, 3, and 7 (Study Period 2) - Linear Scale

FIG. 14 is a graph showing mean change from baseline CTX concentrations following Glu$^{22,25}$, Leu$^{23,28,31}$, Aib$^{29}$, Lys$^{26,30}$]hPTHrP(1-34)NH$_2$ study groups on days 1, 3, and 7 (Study Period 3) - Linear Scale

FIG. 15 is a graph showing mean change from baseline procollagen type 1 amino-terminal propeptide (P1NP) concentrations following Glu$^{22,25}$, Leu$^{23,28,31}$, Aib$^{29}$, Lys$^{26,30}$]hPTHrP(1-34)NH$_2$ study groups and placebo on Days 1, 3, and 7 (Study Period 2) - Linear Scale

FIG. 16 is a graph showing mean change from baseline P1NP concentrations following Glu$^{22,25}$, Leu$^{23,28,31}$, Aib$^{29}$, Lys$^{26,30}$]hPTHrP(1-34)NH$_2$study groups on days 1, 3, and 7(Study Period 3) - Linear Scale

## DETAILED DESCRIPTION OF THE INVENTION

The present invention relates to the use of PTHrP or PTHrP analogues for the prevention or treatment of osteoporosis, osteopenia, osteoporosis, osteoarthritis, or bone fracture or to accelerate bone fracture healing. In particular, the preferred compound for use in the various embodiments of this invention is [Glu$^{22,25}$, Leu$^{23,28,31}$, Aib$^{29}$, Lys$^{26,30}$]hPTHrP(1-34)NH$_2$ or a salt thereof. The bone anabolic agent [Glu$^{22,25}$, Leu$^{23,28,31}$, Aib$^{29}$, Lys$^{26,30}$]hP-THrP(1-34)NH$_2$ has been described in previous publications including Int. Publ. No. WO 2008/063279, US Patent Appln Publn, 2009/0227498 and US Pat No. 5,969,095.

The term "treating" or "treatment" of a mammal, preferably a human is understood to include treating, preventing, or ameliorating the symptoms associated with, or reducing the incidence of, reducing the pathogenesis of, facilitating the recovery from or delaying the onset of the condition being considered including osteopenia, osteoporosis, osteoarthritis, bone fracture, and so forth.

The term "preventing" as used herein is understood to mean preventing or delaying the disease or symptom from occurring in a subject which may be predisposed to the disease or symptom but has not yet been diagnosed as having it.

As used herein, the unit microgram may be represented by either "mcg" or "$\mu$g"; polycarbonate may be represented by the term "PC", and phosphate buffered saline (PBS).

With regard to osteopenia or osteoporosis, it will not matter if the osteoporosis or risk of osteoporosis from which the subject suffers finds its roots in immobilization, age, low gonadal state (e.g. postmenopausal women, testosterone deficient males - including chemically-induced low gonadal -like states induced through use of aromatase inhibitors, anti-androgens, gonadotropin agonist/antagonists and the like), endocrinological disorders (e.g. diabetes, adrenal insufficiency, cushing's syndrome), malnutrition including vitamin D and/or calcium deficiency, rheumatoid arthritis, renal insufficiency, various cancers including myelomas and leukemias, certain inherited forms of osteoporosis and osteoporosis caused by concomitant administration of medicines known or suspected to cause bone loss (e.g. corticosteroids, peroxisome proliferator-activated receptor gamma (PPARgamma) agonists, thyroid medications, lithium therapy, antidepressants, proton pump inhibitors, etc). Whatever the source, osteoporosis risk is most broadly identified by identifying at risk populations but more specifically can be identified by looking at individual risk factors including low bone mineral density and/or prior incidence of fracture in the individual in question. It should be appreciated that the compositions, products, devices and methods of this invention can be applied to at-risk populations or individuals. Because of the highly bone anabolic nature of the compositions and methods of this invention there is particular value in treating populations at especially high risk, including those with bone mineral density at more than 1 standard deviation below the mean, or more than 2 standard deviations below the mean or more than 2.5 standard deviations below the mean. Alternatively or in addition, the compositions and methods of this invention are of particular value for those who have had one or more previous bone fractures, particularly those who have suffered from one or more previous fragility fractures.

With regard to treatment of bone fracture or the acceleration of bone fracture healing, the fractures may be either non-traumatic or traumatic factures, including for example, fragility or osteoporotic fractures, and may occur in either vertebral or nonvertebral bones. In particular, osteoporotic fractures may occur at the hip, spine, wrist, or forearm, though they are not limited to these sites.

The previous published reports relating specifically to [Glu$^{22,25}$, Leu$^{23,28,31}$, Aib$^{29}$, Lys$^{26,30}$]hPTHrP(1-34)NH$_2$ have described the administration to a patient in need thereof by subcutaneous injection (e.g. WO 2008/063279), preferably a daily subcutaneous injection. Due to the particular nature of the anabolic effects of PTH and PTHrP and analogues, it is generally believed that their pharmacokinetics has to be fairly tightly controlled in order to achieve bone anabolic effects without losing efficacy or possibly even leading to bone loss. In particular, it has been noted that a transient, daily exposure to an adequate amount of a PTH, PTHrP or PTHrP analogue can induce anabolic effects on bone with a lag in bone resorption resulting in a net increase in bone density and a corresponding reduction in fractures (see, for example, Neer, et al. New England Journal of Medicine, vol 344; 1434-1441, May 10, 2001). However, the drawbacks of PTH therapy as currently available include side effects such as hypercalcemia even at a low daily dose of 20 $\mu$g per day and the inconvenience of requiring patients to inject themselves subcutaneously every day with the drug. These challenges are compounded by the fact that the patient population most likely to benefit from the therapy are often the elderly and infirm. In this regard, it is worth noting that the PTHrP analogue [Glu$^{22,25}$, Leu$^{23,28,31}$, Aib$^{29}$, Lys$^{26,30}$]hPTHrP(1-34)NH$_2$ is a bone anabolic agent that is particularly efficacious at increasing bone mineral density in osteoporotic patients and of particular interest is its reduced tendency to induce hypercalcemia in patients even at very high doses (e.g. 80 μg sc per day). However, the problem with the inconvenience of a daily injection remains. For this reason, the exciting discovery that a very viable and alternative delivery of the PTHrP analogue [Glu$^{22,25}$, Leu$^{23,28,31}$, Aib$^{29}$, Lys$^{26,30}$]hPTHrP(1-34)NH$_2$ as reported herein is particularly noteworthy.

The alternative delivery described in this patent application relates to the use of microprojection, including microneedle, arrays coated with the PTHrP analogue [Glu$^{22,25}$, Leu$^{23,28,31}$, Aib$^{29}$, Lys$^{26,30}$]hPTHrP(1-34)NH$_2$. In general, the advantages of a microprojection array over a subcutaneous administration of the drug relate to the fact that the microprojections in the array do not need to completely penetrate the dermis in order to effectively deliver the drug substance, thereby providing a relatively painfree delivery route to the patient. Microprojection arrays typically consist of a plurality of microprojections, for example microneedles, fixed to a support material. The microprojections, for example microneedles, are often described as containing a reservoir or channel or mechanism such that the very tiny microprojections, for example microneedles, can transfer enough of the drug substance into the subject undergoing treatment. In some instances, the microprojections, for example microneedles, have been reported to be useful where the microprojections do not contain a separate reservoir but rather are directly coated with the drug substance (see, for example, US Pat Appln Publn No. 2005/0256045). In this latter mode of operation, the technology that has been described to date works best when the drug has a high enough potency so that the very tiny, coated microprojections, for example microneedles, can convey enough of the drug to effectively treat the patient. For the specific example of PTH 1-34 (teriparatide), work has been disclosed using the compound on microneedle arrays where those arrays are coated with enough drug to approximate the exposure of a 20 μg subcutaneous dose (or less) of teriparatide, which is the approved and marketed dose for that compound. While every drug poses its own challenges with regard to any particular form of drug delivery, some challenges can be greater than others. In particular, for the directly coated microprojections, for example microneedles, containing the PTHrP analogue [Glu$^{22,25}$, Leu$^{23,28,31}$, Aib$^{29}$, Lys$^{26,30}$]hPTHrP(1-34)NH$_2$, doses higher than the 20 μg currently marketed dose of teriparatide are preferred. For example, it has been discovered that subcutaneous doses as high as 80 μg of [Glu$^{22,25}$, Leu$^{23,28,31}$, Aib$^{29}$, Lys$^{26,30}$]hPTHrP(1-34)NH$_2$ are highly effective and well-tolerated. Without the aid of some sort of drug retaining reservoir or channel, there are legitimate questions of whether such a large dosing volume can be effectively and reproducibly coated onto the microprojections (e.g., microneedles) and moreover, whether such a large dosing volume can be effectively and reproducibly delivered in a manner that is consonant with the requirement of tightly controlled pharmacokinetics. Beyond the questions associated with the higher dose of this particular drug are the problems inherent to delivering any polypeptide through the skin. While the delivery of teriparatide by an intradermal route has been documented, one should be especially cautious in attempting to extrapolate those results to completely different polypeptides. Differences in solubility, stability, polarity, ionization and many other factors make any comparisons or predictions from one class of compounds to another suspect. In accordance with the features of this invention, the various aspects will be presented both separately and in combination though it should be appreciated that the invention is not limited to the specific combinations described.

In a first aspect of this invention, a formulation for coating the microprojection (e.g., microneedle) delivery device is described. As mentioned previously, the coating formulation ideally provides a suitable concentration, viscosity and stability of the drug and furthermore, the excipients used (if any) in the coating formulation must not be excessively irritating or allergenic to the skin of the animal being treated, especially where the treated animal is a human. In this regard, it has been quite surprisingly discovered that the compounds useful in this invention can be effectively coated onto the microprojections (e.g., microneedles) with or without the addition of traditional stabilizing excipients and still maintain very good drug stability. For purposes of evaluation, several coating formulations containing containing different concentrations of drug and excipient were prepared, and the formulations used to coat polycarbonate or liquid crystalline polymer solid microstructured transdermal system ("sMTS") microprojection arrays with 500 μm square pyramid needle structures spaced 550 μm apex to apex. After coating the drug substance onto the microneedles, the formulation was dried and the stability evaluated.

In Table 1, stability results for aqueous formulations of [Glu$^{22,25}$, Leu$^{23,28,31}$, Aib$^{29}$, Lys$^{26,30}$]hPTHrP(1-34)NH$_2$ with only a PBS buffer 1X (pH 7.4) as an excipient coated on a polycarbonate array after drying are displayed. As can be seen from Table 1, good stability was observed with both coating concentrations as well as good stability independent of final loading doses.

**Table 1: Stability of [Glu$^{22,25}$, Leu$^{23,28,31}$, Aib$^{29}$, Lys$^{26,30}$]hPTHrP(1-34)NH$_2$ on array with only PBS Buffer as excipient**

| Weight % | Storage Conditions | | Initial | 2 week | 4 week |
|---|---|---|---|---|---|
| 20% aqueous solution 32 mcg / array | 4°C | Content (mcg*/array) | 32.3 | 32.9 | 35.0 |
| | | Std. Dev. | 4.1 | 1.9 | 4.7 |
| | | % of Initial Content | 100 | 100 | 100 |
| | 25°C | Content (mcg/array) | 32.3 | 32.2 | 33.0 |
| | | Std. Dev. | 4.1 | 1.7 | 2.0 |
| | | % of Initial Content | 100 | 100 | 100 |
| 60% aqueous solution 142 mcg / array | 4°C | Content (mcg/array) | 141.9 | 166.7 | 166 |
| | | Std. Dev. | 17.0 | 20.0 | 11.7 |
| | | % of Initial Content | 100 | 100 | 100 |
| | 25°C | Content (mcg/array) | 141.9 | 133.3 | 169.6 |
| | | Std. Dev. | 17.0 | 11.6 | 29.4 |
| | | % of Initial Content | 100 | 94 | 100 |
| 60% aqueous solution 387 mcg / array | 4°C | Content (mcg/array) | 386.5 | 368.8 | 361.7 |
| | | Std. Dev. | 57.0 | 58.9 | 7.2 |
| | | % of Initial Content | 100 | 95 | 94% |
| | 25°C | Content (mcg/array) | 386.5 | 390.8 | 291.3 |
| | | Std. Dev. | 57.0 | 35.4 | 18.1 |
| | | % of Initial Content | 100 | 100 | 75% |
| *mcg = μg = microgram | | | | | |

The reported weight percentages in Table 1 refer to crude peptide weight including acetic acid, trifluoroacetic acid and small amounts of water. The actual weight content normalized to peptide is approximately 85% of the listed amount.

Additional experiments were performed for different loading concentrations of [Glu$^{22,25}$, Leu$^{23,28,31}$, Aib$^{29}$, Lys$^{26,30}$]hPTHrP(1-34)NH$_2$ and excipients.

Table 2 summarizes some of those findings. The formulations in Table 2 refer to the formulation concentration and excipients used to coat the microneedle arrays. As was performed previously, the formulation solution was coated onto the microneedle array and the coated microneedle arrays dried prior to the stability evaluation.

**Table 2: Stability of [Glu$^{22,25}$, Leu$^{23,28,31}$, Aib$^{29}$, Lys$^{26,30}$]PTHrP(1-34)NH$_2$ with only PBS and with PBS and other excipients. Storage was at approximately 4 degrees Celsius and ambient RH.**

| Formulation Glu$^{22,25}$, Leu$^{23,28,31}$, Aib$^{29}$, Lys$^{26,30}$]hPTHrP(1-34)NH$_2$ | Desiccant | % of initial (1 week) | % of initial (2 week) |
|---|---|---|---|
| 50% aqueous formulation | No | 91.5 | 93.2 |
| 50% aqueous formulation | Yes | 101.6 | 95.4 |
| 30% aqueous with 30% sucrose | no | 99.1 | 94.3 |
| 30% aqueous with 30% sucrose | yes | 101.6 | 98.6 |
| 30% aqueous with 4.5% HEC* | no | 91.3 | 86.5 |
| 30% with 4.5% HEC | yes | 87.5 | 86.5 |

(continued)

| Formulation Glu$^{22,25}$, Leu$^{23,28,31}$, Aib$^{29}$, Lys$^{26,30}$] hPTHrP(1-34)NH$_2$ | Desiccant | % of initial (1 week) | % of initial (2 week) |
|---|---|---|---|
| 30% aqueous with 17.5% sucrose and 2% HEC | no | 85.7 | 86.5 |
| 30% aqueous with 17.5% sucrose and 2% HEC | yes | 98.9 | 102.3 |
| *Hydroxyethylcellulose | | | |

The reported weight percentages in Table 2 refer to crude peptide weight including acetic acid and water. The actual weight content normalized to peptide is approximately 80% to 90% of the listed amount (that is, acetic acid and water account for 10% to 20% of the crude peptide weight).

In Table 3, stability results for [Glu$^{22,25}$, Leu$^{23,28,31}$, Aib$^{29}$, Lys$^{26,30}$]hPTHrP(1-34)NH$_2$ on an LCP (liquid crystal polymer) microneedle array, with 3% histidine, 5% histidine or 9% potassium chloride as an excipient are displayed. The formulations in Table 3 refer to the excipients used to coat the microneedle arrays. As was performed previously, the formulation solution was coated onto the microneedle array and the microneedle array packaged in the presence or absence of a desiccant prior to the stability evaluation. Desiccants suitable for pharmaceutical applications include silica gel and molecular sieves.

As can be seen from Table 3, good microneedle array coating and [Glu$^{22,25}$, Leu$^{23,28,31}$, Aib$^{29}$, Lys$^{26,30}$]hPTHrP(1-34)NH$_2$ stability was observed in formulations with on PBS as an excipient, or in formulations containing PBS and the additional excipients histidine, or potassium chloride, and stability was enhanced by the presence of a desiccant in the packaging.

**Table 3: Stability of Glu$^{22,25}$, Leu$^{23,28,31}$, Aib$^{29}$, Lys$^{26,30}$]hPTHrP(1-34)NH$_2$ coated microneedle arrays with and without desiccant**

| Formulation | Test | Initial | Time (Months) | | | | | | |
|---|---|---|---|---|---|---|---|---|---|
| | | | 40C/75% RH | | | 25C/60% RH | | 4C/Ambient | |
| | | | 0.5M | 1M | 2M | 1M | 2M | 1M | 2M |
| Control* | Content (mcg/ array) | 124 | 118 | 102 | 91 | 124 | 89 | 126 | 101 |
| | Purity (%) | 98.9 | 95.4 | 92.1 | 91.1 | 95.2 | 92.5 | 98.3 | 97.7 |
| Control with Desiccant | Content (meg/ array) | 124 | 119 | 125 | 97 | 122 | 112 | 128 | 98 |
| | Purity (%) | 98.9 | 97.6 | 96 | 94.3 | 97.9 | 97.4 | 98.6 | 983 |
| 3% Histidine | Content (mcg/ array) | 136 | 135 | 122 | 89 | 138 | 94 | 125 | 123 |
| | Purity (%) | 99.5 | 98.3 | 96.8 | 95.8 | 98.7 | 97,6 | 99.2 | 99.2 |
| 3% Histidine with Desiccant | Content (mcg/ array) | 136 | 137 | 135 | 101 | 139 | 119 | 138 | 104 |
| | Purity (%) | 99.5 | 98.5 | 97.4 | 96.1 | 98.9 | 98.3 | 99.3 | 99.2 |
| 5% Histidine | Content (mcg/ array) | 134 | 125 | 108 | 39 | 115 | 69 | 120 | 66 |
| | Purity (%) | 99.5 | 98.4 | 97.3 | 96.3 | 98.7 | 98 | 99.1 | 99.1 |

(continued)

| | | | Time (Months) | | | | | | |
|---|---|---|---|---|---|---|---|---|---|
| | | | 40C/75% RH | | | 25C/60% RH | | 4C/Ambient | |
| 5% Histidine with Desiccant | Content (mcg/ array) | 134 | 130 | 117 | 44 | 113 | 70 | 120 | 86 |
| | Purity (%) | 99.5 | 98.5 | 98 | 97.5 | 98.9 | 98.7 | 99.2 | 99.2 |
| 9% Potassium Chloride | Content (mcg/ array) | 111 | 94 | 90 | 58 | 96 | 57 | 109 | 74 |
| | Purity (%) | 99.4 | 94.3 | 92.7 | 90.5 | 93.4 | 89,9 | 98.8 | 97.5 |
| 9% Potassium Chloride with Desiccant | Content (mcg/ array) | 111 | 108 | 105 | 87 | 105 | 88 | 105 | 75 |
| | Purity (%) | 99.4 | 98.2 | 97.5 | 96.4 | 98.8 | 98.3 | 98.9 | 99.2 |
| * control formulation includes PBS as sole excipient | | | | | | | | | |

Certain drug coated microarrays were tested in vivo in a preclinical model using Sprague Dawley rats. These studies assessed transdermal delivery of [Glu$^{22,25}$, Leu$^{23,28,31}$, Aib$^{29}$, Lys$^{26,30}$]hPTHrP(1-34)NH$_2$ using microneedle arrays in Sprague Dawley rats. Application of [Glu$^{22,25}$, Leu$^{23,28,31}$, Aib$^{29}$, Lys$^{26,30}$]hPTHrP(1-34)NH$_2$-microneedle arrays to the skin with only short contact times (1-5 minutes) achieved systemic exposure of [Glu$^{22,25}$, Leu$^{23,28,31}$, Aib$^{29}$, Lys$^{26,30}$]hPTHrP(1-34)NH$_2$ and a rapid absorption from the array and rapid elimination.

**Table 4: Studies with microneedle arrays comprising polycarbonate microneedles coated with Glu$^{22,25}$, Leu$^{23,28,31}$, Aib$^{29}$, Lys$^{26,30}$]hPTHrP(1-34)NH$_2$**

| Study Number | Group | Number of Animals | Peptide Content§ ($\mu$g) | Formulation % Peptide | % HEC | Dose Level ($\mu$g/kg) |
|---|---|---|---|---|---|---|
| 09RAD005 | 1 | 9 | 20.0 | 5.0 | 4.5 | 72 |
| 09RAD006 | 1 | 10 | 31.0 | 15.0 | 4.0 | 104 |
| 09RAD006 | 2 | 10 | 22.0 | 10.0 | 4.5 | 76 |
| 09RAD010 | 1 | 8 | 26.2 | 12.5 | 4.5 | 83 |
| 09RAD010 | 2 | 8 | 57.7 | 12.5 | 4.5 | 178 |
| 09RAD011 | 1 | 8 | 67.7 | 16.7 | 4.0 | 227 |
| 09RAD011 | 2 | 8 | 45.6 | 20.5 | 3.5 | 157 |
| 09RAD017 | 1 | 8 | 27.0 | 16.7 | 4.0 | 78 |
| 09RAD017 | 2 | 8 | 27.0 | 16.7 | 4.0 | 79 |
| 09RAD017 | 3 | 8 | 27.0 | 16.7 | 4.0 | 80 |
| 09RAD017 | 4 | 8 | 32.0 | 20.0 | 3.5 | 94 |
| 09RAD018 | 1 | 8 | 141.9 | 59.3 | 0.0 | 433 |
| 09RAD018 | 2 | 8 | 386.5 | 59.3 | 0.0 | 1189 |
| 09RAD030 | 1 | 2 | 27.0 | 16.7 | 4.0 | 105 |
| 09RAD030 | 2 | 3 | 32.0 | 20.0 | 3.5 | 125 |
| 09RAD030 | 3 | 1 | 26.2 | 12.5 | 4.5 | 99 |
| 09RAD030 | 4 | 2 | 27.0 | 16.7 | 4.0 | 103 |
| 09RAD030 | 5 | 3 | 32.0 | 20.0 | 3.5 | 120 |
| 09RAD030 | 6 | 1 | 26.2 | 12.5 | 4.5 | 98 |
| 09RAD048 | 1 | 12 | 9.8 | 49 | 0.0 | 37 |

(continued)

| Study Number | Group | Number of Animals | Peptide Content§ (μg) | Formulation % Peptide | % HEC | Dose Level (μg/kg) |
|---|---|---|---|---|---|---|
| 09RAD048 | 2 | 12 | 54.0 | 49 | 0.0 | 178 |
| 09RAD053 | 1 | 6 | 141.9 | 59.3 | 0.0 | 529 |
| 09RAD053 | 2 | 1 | 386.5 | 59.3 | 0.0 | 1470 |

§ Peptide ([Glu$^{22,25}$, Leu$^{23,28,31}$, Aib$^{29}$, Lys$^{26,30}$]hPTHrP(1-34)NH$_2$) content calculated based on total peptide content including water and acetic acid. Actual peptide content is approximately 80% - 90% of the stated amount.

**Materials and Methods for studies RAD 005, 006, 010, 011, 017, 018, 030, 048, 053 of Table 4**

*Animals*

Male Sprague Dawley rats with jugular vein catheters were purchased from Charles River Laboratories. Once received, they were acclimated for at least 24 hours prior to dosing. Animals were singly housed in polycarbonate ventilated (45 ACH) cages. All animals were provided certified rodent diet (2918 from Harlan Teklad) and water *ad libitum.* The housing environment was maintained between 18-26° C with 30-70% relative humidity with a 12 hr light: 12 hr dark cycle.

*Test Article*

**Table 5: Microneedle arrays used for studies RAD 005, 006, 010, 011, 017, 018, 030, 048, 053**

| Microneedle arrays | Polycarbonate arrays |
|---|---|
| Material of Construction | Polycarbonate ("PC") |
| Number of Microneedles | 366 |
| Flexural Modulus (by ISO 178) | 2300 |
| Grade | Class VI, medical grade polymer |
| Surface area | 5.5 cm$^2$ or ~27 mm in diameter |
| Depth of Penetration (DOP) | 250+/-10μm |
| Height of Microneedles | 500μm |
| Spacing between Microneedles | 550μm apart (tip to tip) |

The finished ([Glu$^{22,25}$, Leu$^{23,28,31}$, Aib$^{29}$, Lys$^{26,30}$]hPTHrP(1-34)NH$_2$) - microneedle array is sealed in a packaging system that insures moisture and light are controlled to maintain a biostatic environment (an environment in which microorganisms can not proliferate). Further, the ([Glu$^{22,21}$, Leu$^{23,28,31}$, Aib$^{29}$, Lys$^{26,30}$]hPTHrP(1-34)NH$_2$ -microneedle array Finished Drug Product is stored under refrigerated conditions until dosing. Microbial release specifications for the drug product are based on the acceptance criteria described in PhEur 5.1.4 and USP <1111>, USP <61>, and <62>. The drug product also meets the endotoxin specifications in Ph.Eur. 2.6.14 and USP <85> and <161>.

Based on the manufacturing processes designed to insure microbial control and on the release specifications governing the release of the drug product prior to use in humans, the ([Glu$^{22,25}$, Leu$^{23,28,31}$, Aib$^{29}$, Lys$^{26,30}$]hPTHrP(1-34)NH$_2$) - microneedle array Finished Drug Product is defined as an ultra low bioburden product.

Microneedle arrays coated with ([Glu$^{22,25}$, Leu$^{23,28,31}$, Aib$^{29}$, Lys$^{26,30}$]hPTHrP(1-34)NH$_2$) were manufactured by dip-coating the microneedle array into an aqueous, PBS buffered solution having the disclosed concentration of ([Glu$^{22,25}$, Leu$^{23,28,31}$, Aib$^{29}$, Lys$^{26,30}$]hPTHrP(1-34)NH$_2$) by weight and additional excipients as noted in Table 4. Other methods of coating microneedles are known in the art. After a dipping step, the arrays are air dried. Various lots of ([Glu$^{22,25}$, Leu$^{23,28,31}$, Aib$^{29}$, Lys$^{26,30}$]hPTHrP(1-34)NH$_2$-microneedle arrays were tested in which the percent (w/w) of ([Glu$^{22,25}$, Leu$^{23,28,31}$, Aib$^{29}$, Lys$^{26,30}$]hPTHrP(1-34)NH$_2$ and hydroxyethyl cellulose (HEC) in the formulation, and the amount of compound coated on the array was varied. Details of the different microneedle arrays lots are given in Table 4. The microneedle arrays are supplied individually packaged in a light protective foil pouch, some with a desiccant and some without and stored at 4°C. Typically, one hour prior to dosing microneedle arrays were removed from refrigeration (approximately 4°C) and allowed to equilibrate to room temperature. In studies 09RAD005 and 09RAD006, the microneedle arrays were applied immediately after removal from the refrigerator, without sufficient time to reach room temperature.

*Dose Administration*

Transdermal dose delivery was assessed by application of ([Glu$^{22,25}$, Leu$^{23,28,31}$, Aib$^{29}$, Lys$^{26,30}$]hP-THrP(1-34)NH$_2$-microneedle arrays to the skin of rats. The standard procedure for skin preparation and microneedle array application is as follows.

One day prior to dosing, an area just above the hind legs on the dorsal surface of the rat was shaved using electric clippers. Nair® hair removal lotion (Church & Dwight Co.) was then applied to the area for 5 to 8 minutes to remove remaining fur stubble. The Nair® lotion was then thoroughly removed using a damp cloth. The next day a microneedle array was applied using a spring-loaded applicator. The microneedle array was left in contact with skin for five minutes, before being removed. During array application and contact time the rats were manually restrained.

Throughout these studies variations to the procedure for microneedle application were explored.

Table 6 summarizes the dosing and application conditions for each study in this set.

**Table 6: Array contact time, temperature and skin preparation**

| Study Number | Group | Array Temperature* | Array Contact Time | Skin Preparation |
|---|---|---|---|---|
| 09RAD005 | 1 | 4°C | 5 Minutes | Clippers and Nair |
| 09RAD006 | 1 | 4°C | 5 Minutes | Clippers and Nair |
| 09RAD006 | 2 | 4°C | 5 Minutes | Clippers and Nair |
| 09RAD010 | 1 | 22°C | 5 Minutes | Clippers and Nair |
| 09RAD010 | 2 | 22°C | 5 Minutes | Clippers and Nair |
| 09RAD011 | 1 | 22°C | 5 Minutes | Clippers and Nair |
| 09RAD011 | 2 | 22°C | 5 Minutes | Clippers and Nair |
| 09RAD017 | 1 | 22°C | 5 Minutes | Clippers and Nair |
| 09RAD017 | 2 | 22°C | 1 Minute | Clippers and Nair |
| 09RAD017 | 3 | 22°C | 5 Minutes | Clippers |
| 09RAD017 | 4 | 22°C | 5 Minutes | Clippers and Nair |
| 09RAD018 | 1 | 22°C | 5 Minutes | Clippers and Nair |
| 09RAD018 | 2 | 22°C | 5 Minutes | Clippers and Nair |
| 09RAD030 | 1 | 22°C | 1 Minute | Clippers |
| 09RAD030 | 2 | 22°C | 1 Minute | Clippers |
| 09RAD030 | 3 | 22°C | 1 Minute | Clippers |
| 09RAD030 | 4 | 22°C | 5 Minutes | Clippers |
| 09RAD030 | 5 | 22°C | 5 Minutes | Clippers |
| 09RAD030 | 6 | 22°C | 5 Minutes | Clippers |
| 09RAD048 | 1 | 22°C | 5 Minutes | Clippers |
| 09RAD048 | 2 | 22°C | 5 Minutes | Clippers |
| 09RAD053 | 1 | 22°C | 5 Minutes | Clippers |
| 09RAD053 | 2 | 22°C | 5 Minutes | Clippers |

* Microneedle arrays applied to rats immediately after removal from refrigerator are designated as "4°C." Microneedle arrays allow to first equilibrate to room temperature are designated as "22°C."

*Serum Collection*

**09RAD005, 09RAD006, 09RAD010, 09RAD011, 09RAD017, 09RAD018:**

Blood was collected at three time points from each animal out of a possible total of five time destinations (5 minutes, 15 minutes, 30 minutes, 45 minutes, 90 minutes) from each rat on a staggered schedule so that all time points would be represented with extra sampling at 15 minutes without overdrawing from any animal. Approximately 1 mL of blood was collected via the catheter from the jugular vein using a syringe and needle from rats for their first two blood draws. For the terminal blood collection, animals were euthanized via $CO_2$ chamber and approximately 1 mL of blood was collected via cardiac puncture. The blood was immediately transferred to a serum separator tube that contained 25 $\mu$L of a 2.5 mg/ml aprotinin (Sigma) solution.

*Blood draw schedule for 09RAD030:*

Blood was collected from each rat 5, 10, 15, 30 and 45 minutes post dose. Approximately 600 $\mu$L of blood was collected via the catheter from the jugular vein using a syringe and needle from rats for their first four blood draws. For the terminal blood collection, animals were euthanized via $CO_2$ chamber and approximately 600 $\mu$L of blood was collected via cardiac puncture. The blood was immediately transferred to a serum separator tube that contained 12 $\mu$L of a 2.5 mg/ml aprotinin (Sigma) solution.

*Blood draw schedule for 09RAD048:*

Blood was collected three or four times from each rat at the time points depicted in the tables below. Approximately 1 mL of blood was collected via the catheter from the jugular vein using a syringe and needle from rats for any non-terminal time points. For the terminal blood collection, animals were euthanized via $CO_2$ chamber and approximately 1 mL of blood was collected via cardiac puncture. The blood was immediately transferred to a serum separator tube that contained 20 $\mu$L of a 2.5 mg/ml aprotinin (Sigma) solution.

*Blood draw schedule for 09RAD053:*

Blood was collected from each rat 5, 15, 30, 45, 90 and 120 minutes post dose. Approximately 500 $\mu$L of blood was collected via the catheter from the jugular vein using a syringe and needle from rats for their first five blood draws. For the terminal blood collection, animals were euthanized via $CO_2$ chamber and approximately 500 $\mu$L of blood was collected via cardiac puncture. The blood was immediately transferred to a serum separator tube that contained 10 $\mu$L of a 2.5 mg/ml aprotinin (Sigma) solution.

*Residual ([Glu$^{22,25}$, Leu$^{23,28,31}$, Aib$^{29}$, Lys$^{26,30}$]hPTHrP(1-34)NH$_2$Analysis*

Residual ([Glu$^{22,25}$, Leu$^{23,28,31}$, Aib$^{29}$, Lys$^{26,30}$]hPTHrP(1-34)NH$_2$ was eluted from all ([Glu$^{22,25}$, Leu$^{23,28,31}$, Aib$^{29}$, Lys$^{26,30}$]hPTHrP(1-34)NH$_2$-microneedle arrays used to dose studies 09RAD010 and 09RAD011. In addition, at least two microneedle arrays from each group that were not used to dose, to confirm initial array drug content, and two uncoated arrays (placebo-microneedle arrays) were eluted. In study 09RAD018, ([Glu$^{22,25}$, Leu$^{23,28,31}$, Aib$^{29}$, Lys$^{26,30}$]hPTHrP(1-34)NH$_2$was eluted from one ([Glu$^{22,25}$, Leu$^{23,28,31}$, Aib$^{29}$, Lys$^{26,30}$]hPTHrP(1-34)NH$_2$-microneedle array per group prior to dosing. ([Glu$^{22,25}$, Leu$^{23,28,31}$, Aib$^{29}$, Lys$^{26,30}$]hPTHrP(1-34)NH$_2$ elution was performed according to the following protocol.

The microneedle array was removed from its adhesive backing using forceps, and was placed, needles down, in a 5 mL snap-cap vial (Nalgene). 1 mL of PBS-Tween® 80 extraction solution (0.2 g Tween/L PBS) was added to the vial so that the array was completely immersed. The vial was placed on an orbital shaker set at 100-150 oscillations per minute for 30 minutes. The array was then removed from the vial and discarded. The vials containing eluted ([Glu$^{22,25}$, Leu$^{23,28,31}$, Aib$^{29}$, Lys$^{26,30}$]hPTHrP(1-34)NH$_2$ in the PBS-Tween 80 solution from studies 09RAD010 and 09RAD011 were stored at -20°C. Samples were then used for HPLC analysis of ([Glu$^{22,25}$, Leu$^{23,28,31}$, Aib$^{29}$, Lys$^{26,30}$]hP-THrP(1-34)NH$_2$ content. The vials containing eluted ([Glu$^{22,25}$, Leu$^{23,28,31}$, Aib$^{29}$, Lys$^{26,30}$]hPTHrP(1-34)NH$_2$ in the PBS-Tween 80 solution from study 09RAD018 were stored at 4°C. Samples were then sent for HPLC analysis of ([Glu$^{22,25}$, Leu$^{23,28,31}$, Aib$^{29}$, Lys$^{26,30}$]hPTHrP(1-34)NH$_2$ content.

The initial drug content of each group of arrays was determined to be the average of the at least two two arrays that were not used to dose. The residual content is the average amount of ([Glu$^{22,25}$, Leu$^{23,28,31}$, Aib$^{29}$, Lys$^{26,30}$]hP-THrP(1-34)NH$_2$ on the remaining arrays. The percent of drug load released was then calculated as:

$$\%Released = Peptide_{INITIAL} - Peptide_{RESIDUAL}) / Peptide_{INITIAL}$$

### Sample Handling and Storage

Blood was kept at room temperature in serum separator tubes containing aprotinin for approximately 45 minutes to allow it to clot. Once clotted, the blood was centrifuged at 2500 rpm for 10 minutes. Serum was transferred to microcentrifuge tubes for storage at -80°C until analysis of ([Glu$^{22,25}$, Leu$^{23,28,31}$, Aib$^{29}$, Lys$^{26,30}$]hPTHrP(1-34)NH$_2$ content by radioimmunoassay, as described below.

### ([Glu$^{22,25}$, Leu$^{23,28,31}$, Aib$^{29}$, Lys$^{26,30}$]hPTHrP(1-34)NH$_2$ Radioimmunoassay

**Assay Buffer Preparation:** 2.00 g of bovine serum albumin (BSA, Sigma) was dissolved in 750 mL of deionized water. 17.4 g of potassium phosphate, dibasic (EMD), 9.0 g of sodium chloride (Sigma), 0.50 g of sodium azide (Sigma), and 1.00 mL of Triton X-100 (Sigma) were added. The pH was adjusted to 7.4 with 1.0 M potassium phosphate (Fisher) and the final volume was adjusted to 1.0 L.

**Standard Curve Preparation:** A 0.1 mg/mL aliquot of ([Glu$^{22,25}$, Leu$^{23,28,31}$, Aib$^{29}$, Lys$^{26,30}$]hPTHrP(1-34)NH$_2$ in 0.1N acetic acid was thawed on ice. A 2000 ng/mL dilution was made in rat serum (Innovative Research) containing aprotinin (0.1 mg/mL, Sigma). This dilution was further diluted to 250 ng/mL in the same serum. The 250 ng/mL solution was used to make an 8 ng/mL solution of ([Glu$^{22,25}$, Leu$^{23,28,31}$, Aib$^{29}$, Lys$^{26,30}$]hPTHrP(1-34)NH$_2$ in the same serum. Finally, this solution was serially diluted 2-fold to obtain the following concentrations: 4, 2, 1, 0.5, 0.25, 0.125, 0.063, 0.031 ng/mL. All dilutions were made and kept on ice until ethanol extraction.

**Sample Preparation:** Study serum samples were thawed on ice and diluted in pooled rat serum containing aprotinin (0.1 mg/mL). Based on historical data dilutions were picked to give a final expected concentration between 0.25-2.0 ng/mL.

**Ethanol Extraction:** 250 μL of standard (in duplicate), diluted sample, or blank serum (for non-specific and matrix binding) was put in a microcentrifuge tube. To each sample, standard, or blank 1 mL of room temperature 95% ethanol was added with a repeat pipette. All of these tubes were vortexed for 2 minutes and stored at 4°C for 30 minutes. The samples were then centrifuged at 3600 rpm at 4°C for 30 minutes. The supernatant was removed from each tube and transferred into a new microcentrifuge tube. All samples were vacuum evaporated for 3 hours at the highest temperature setting (approximately 60°C). Once dry the samples were stored at -80°C overnight.

**Reconstitution:** Samples were removed from the freezer and placed at 4°C for 30 minutes. While working on ice, 100 μL of assay buffer was added to each tube. Samples were vortexed for 3 minutes and then stored at 4°C for 30 minutes.

**Antibody Addition:** A 1:11,000 dilution of ([Glu$^{22,25}$, Leu$^{23,28,31}$, Aib$^{29}$, Lys$^{26,30}$]hPTHrP(1-34)NH$_2$ rabbit antiserum, Ipsen) was made in the assay buffer. 100 μL of this antibody solution was added to all reconstituted samples except for non-specific binding tubes. Samples were vortexed for 30 seconds and stored at 4°C for 20-24 hours.

**Probe Addition:** A stock of [$^{125}$I]-Tyro- ([Glu$^{22,25}$, Leu$^{23,28,31}$, Aib$^{29}$, Lys$^{26,30}$]hPTHrP(1-34)NH$_2$ that was less than 30 days old was removed from the freezer and thawed. The stock was diluted in assay buffer until 100 μL of probe solution read between 9,500-11,000 cpms when counted for 1 minute in 10 mL of scintillation fluid. 100 μL of this solution was added to all sample tubes. The tubes were vortexed for 30 seconds and stored at 4°C for 20-24 hours.

**N-Propanol Extraction:** 1 mL of cold n-propanol was added to each sample. The tubes were vortexed for 30 seconds and then stored at 4°C for 15 minutes. Tubes were centrifuged at 3600 rpm at 4°C for 30 minutes. Finally, the supernatant was poured off into a waste container.

**Liquid Scintillation Counting of Samples:** 200 μL of 0.2N NaOH was added to each sample. Samples were vortexed for approximately 5 minutes until the pellet was completely solubilized. Samples were then transferred into 10 mL of scintillation fluid. 100 μL of glacial acetic acid was added to each scintillation vial to neutralize the solution. All samples were counted for one minute on a Beckman Coulter LS6500.

**Data Analysis:** The $B/B_0$ value was found for each standard and unknown sample in the RIA by using the following equation in Microsoft© Excel 2008:

$$B/B_0 = [(Y\text{-}NSB)/(MB\text{-}NSB)] * 100$$

where:

$B/B_0$ = Percent of radio-labeled ([$^{125}$I]-Tyro- ([Glu$^{22,25}$, Leu$^{23,28,31}$, Aib$^{29}$, Lys$^{26,30}$]hPTHrP(1-34)NH$_2$ bound to the antibody

Y = standard or unknown samples' binding (cpm)
MB = matrix binding, or zero concentration (cpm)
NSB = non-specific binding (cpm)

The $B/B_o$ values of the standards were plotted versus the logarithm of the concentration in GraphPad© Prism 4 and a fitted curve was made using the sigmoidal dose-response (variable slope) analysis. From this curve the unknown sample values were extrapolated. In Excel, the extrapolated values were converted to ng/mL and multiplied by the dilution factor to determine the original concentration of each serum sample. All samples for a given rat that fell in the linear range of the assay before being multiplied by the dilution factor were averaged to determine the reported concentration. In the case that all dilutions for a sample fell outside of the linear range of the assay, the sample was reported as above or below the limit of detection and was excluded from average values.

### Pharmacokinetic (PK) Analysis

The average serum [Glu$^{22,25}$, Leu$^{23,28,31}$, Aib$^{29}$, Lys$^{26,30}$]hPTHrP(1-34)NH$_2$ concentrations at each time point were used to create a pharmacokinetic profile for each dosing group from which pharmacokinetic parameters could be determined.

The exception to this was study 09RAD030. In this study, the group sizes were too small (n=1-3) to reliably determine differences in exposure from varied array contact time. In order to have a higher number of samples per group, the average serum [$^{125}$I]-Tyro- ([Glu$^{22,25}$, Leu$^{23,28,31}$, Aib$^{29}$, Lys$^{26,30}$]hPTHrP(1-34)NH$_2$ concentrations were mathematically adjusted to a standard 20 mcg/kg dose and then all of the one minute array application animals were averaged together. In a likewise manner, the five minute array application animals also became one group. The resulting curves were used to determine the pharmacokinetic parameters.

From the pharmacokinetic profile the maximum concentration ($C_{max}$), the time to maximum concentration ($T_{max}$), the area under the curve ($AUC_{0-t}$), and the half-life ($T_{1/2}$) were calculated using Microsoft© Excel 2008 using the PK functions add-in (Allergan). The relative bioavailability (%F) was calculated using the following equation:

$$\%F = AUC_{0-t}/[AUC_{SC}*(Dose/20)]$$

where:

%F = Bioavailability relative to a 20 $\mu$g/kg subcutaneous dose;
$AUC_{0-t}$ = area under the curve of the microneedle array pharmacokinetic profile (ng*min/mL);
$AUC_{SC}$ = area under the curve of historic 20 $\mu$g/kg subcutaneous pharmacokinetic profile (ng*min/mL);
Dose = [Glu$^{22,25}$, Leu$^{23,28,31}$, Aib$^{29}$, Lys$^{26,30}$]hPTHrP(1-34)NH$_2$ content on microneedle array divided by the average body weight ($\mu$g/kg).

### Results and Discussion

Blood samples were collected at various time points following [Glu$^{22,25}$, Leu$^{23,28,31}$, Aib$^{29}$, Lys$^{26,30}$]PTHrP(1-34)NH$_2$ -microneedle array skin application. Samples were diluted in pooled blank rat serum to enable reliable determination of [Glu$^{22,25}$, Leu$^{23,28,31}$, Aib$^{29}$, Lys$^{26,30}$]hPTHrP(1-34)NH$_2$ content by radioimmunoassay. The concentration of [Glu$^{22,25}$, Leu$^{23,28,31}$, Aib$^{29}$, Lys$^{26,30}$]hPTHrP(1-34)NH$_2$ in each sample dilution was determined by extrapolation from a control standard curve generated on the same day. The linear range of each standard curve varies slightly between assays, but is typically 0.25 to 2.0 ng/mL. Samples that fell outside of the linear range of their assay were excluded from analysis.

The concentration of [Glu$^{22,25}$, Leu$^{23,28,31}$, Aib$^{29}$, Lys$^{26,30}$]hPTHrP(1-34)NH$_2$ in individual serum samples and the average and standard deviation for each time point is listed in Tables 7-16. The average values can be used to create pharmacokinetic curves from which PK parameters are calculated.

Comparison of the PK profiles from several studies demonstrate that the [Glu$^{22,25}$, Leu$^{23,28,31}$, Aib$^{29}$, Lys$^{26,30}$]hPTHrP(1-34)NH$_2$ -microneedle arrays result in a similar and consistent exposure profile with rapid absorption and elimination of [Glu$^{22,25}$, Leu$^{23,28,31}$, Aib$^{29}$, Lys$^{26,30}$]hPTHrP(1-34)NH$_2$. For the [Glu$^{22,25}$, Leu$^{23,28,31}$, Aib$^{29}$, Lys$^{26,30}$]hPTHrP(1-34)NH$_2$ -microneedle array lots across the nine studies, the $T_{max}$ occured between 5 and 15 minutes and the $t_{1/2}$ is 14-27 minutes, with the exception of study 09RAD005 were the half-life was calculated to be 43 minutes.

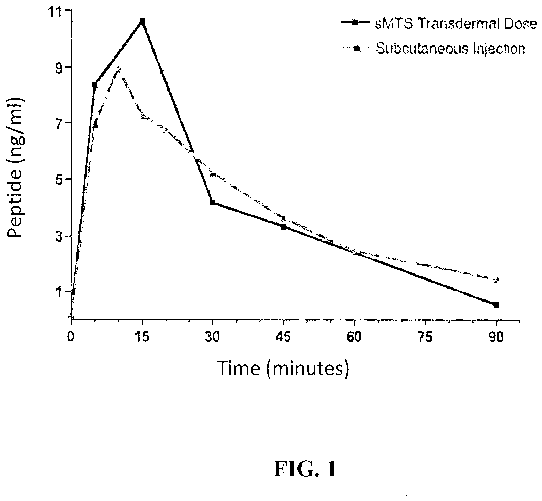

In addition to being consistent between [Glu$^{22,25}$, Leu$^{23,28,31}$, Aib$^{29}$, Lys$^{26,30}$]hPTHrP(1-34)NH$_2$-microneedle lots, these profiles are comparable to historical reference SC injection PK data. Figure 1 is a representative microneedle array PK profile (09RAD010 Group 1), adjusted to a 20 $\mu$g/kg dose, graphed together with the reference SC profile. For this reference SC data, the $t_{1/2}$ is 31 minutes and the $T_{max}$ 10 minutes.

The relative bioavailability of [Glu$^{22,25}$, Leu$^{23,28,31}$, Aib$^{29}$, Lys$^{26,30}$]hPTHrP(1-34)NH$_2$ delivered by sMTS arrays was compared to SC injection (Table 17).

$C_{max}$ values are generally proportional to bioavailability. When the $C_{max}$ is adjusted to a standard 20 μg /kg dose, the [Glu$^{22,25}$, Leu$^{23,28,31}$, Aib$^{29}$, Lys$^{26,30}$]hPTHrP(1-34)NH$_2$-microneedle arrays from 09RAD005 and 09RAD006 had an average $C_{max}$ of 4.2 ± 0.7 ng/mL, which is 47 percent of the $C_{max}$ with a 20 μg /kg SC dose (8.9 ng/mL). However, the average $C_{max}$ from the 09RAD010, 09RAD011, 09RAD017, and 09RAD030 [Glu$^{22,25}$, Leu$^{23,28,31}$, Aib$^{29}$, Lys$^{26,30}$]hP-THrP(1-34)NH$_2$. -microneedle arrays is 8.9 ± 1.8 ng/mL, which is approximately 100 percent of subcutaneous injection.

For Tables 7 through 17, "Peptide" refers to [Glu$^{22,25}$, Leu$^{23,28,31}$, Aib$^{29}$, Lys$^{26,30}$]hPTHrP(1-34)NH$_2$; NA indicates no serum sample was collected, ND means value not determined, LLOQ means lower limit of quantification, and ULOQ means upper limit of quantification.

**Table 7: 09RAD005: Concentrations of Peptide (ng/mL) in Serum after Single Peptide-Microneedle Array Application**

| Time (min) | 20.0μg Peptide, 5.0% Formulation, 4°C Arrays, 5 Minute Application, Clippers and Nair | | | | | | | | | | |
|---|---|---|---|---|---|---|---|---|---|---|---|
| | Rat 1 | Rat 2 | Rat 3 | Rat 4 | Rat 5 | Rat 6 | Rat 7 | Rat 8 | Rat 9 | **Mean** | **SD** |
| 5 | 7.21 | 11.80 | 6.46 | 6.52 | 14.70 | NA | NA | NA | NA | **9.34** | **3.73** |
| 15 | 5.58 | 8.88 | 7.82 | 6.73 | 12.12 | 12.42 | 13.93 | 18.00 | 22.02 | **11.94** | **5.44** |
| 30 | 4.94 | 6.52 | 5.93 | 5.63 | 8.96 | NA | NA | NA | NA | **6.40** | **1.54** |
| 45 | NA | NA | NA | NA | NA | 5.09 | 9.57 | 7.29 | 6.54 | **7.12** | **1.87** |
| 90 | NA | NA | NA | NA | NA | 3.09 | 3.62 | 3.71 | 2.13 | **3.14** | **0.73** |

**Table 8: 09RAD006: Concentrations of Peptide (ng/mL) in Serum after Single Peptide-Microneedle Application**

| Time (min) | 31.0μg Peptide, 15.0% Formulation, 4°C Arrays, 5 Minute Application, Clippers and Nair | | | | | | | | | | | |
|---|---|---|---|---|---|---|---|---|---|---|---|---|
| | Rat 1 | Rat 2 | Rat 3 | Rat 4 | Rat 5 | Rat 6 | Rat 7 | Rat 8 | Rat 9 | Rat 10 | **Mean** | **SD** |
| 5 | 14.59 | 20.39 | 25.64 | 32.56 | 8.29 | NA | NA | NA | NA | NA | **20.29** | **9.43** |
| 15 | 19.33 | 27.92 | 24.52 | 45.96 | 8.37 | 15.65 | 34.29 | 22.98 | 20.43 | 16.35 | **23.58** | **10.59** |
| 30 | 9.54 | 11.45 | 13.26 | 22.40 | 5.99 | NA | NA | NA | NA | NA | **12.53** | **6.14** |
| 45 | NA | NA | NA | NA | NA | 2.64 | 6.69 | 4.66 | 5.20 | 4.48 | **4.73** | **1.46** |
| 90 | NA | NA | NA | NA | NA | 1.04 | 1.27 | 1.08 | 1.14 | 0.67 | **1.04** | **0.22** |
| Time (min) | 22.0μg Peptide, 10.0% Formulation, 4°C Arrays, 5 Minute Application, Clippers and Nair | | | | | | | | | | | |
| | Rat 11 | Rat 12 | Rat 13 | Rat 14 | | Rat 15 | Rat 16 | Rat 17 | Rat 18 | Rat 19 | Rat 20 | **Mean** | **SD** |
| 5 | 24.92 | 6.97 | 21.50 | 14.68 | | 17.58 | NA | NA | NA | NA | NA | **17.13** | **6.88** |
| 15 | 18.22 | 7.02 | 13.25 | 12.80 | | 11.06 | 5.74 | 32.74 | 11.63 | 9.06 | 10.15 | **13.17** | **7.71** |
| 30 | 5.79 | 3.53 | 10.30 | 6.50 | | 6.17 | NA | NA | NA | NA | NA | **6.46** | **2.44** |
| 45 | NA | NA | NA | NA | | NA | 1.34 | 5.76 | 3.15 | 2.80 | 3.69 | **3.35** | **1.61** |
| 90 | NA | NA | NA | NA | | NA | 0.29 | 1.31 | 0.71 | 0.59 | 1.08 | **0.80** | **0.40** |

NA No serum sample was collected.

**Table 9: 09RAD010: Concentrations of Peptide (ng/mL) in Serum after Single Peptide-Microneedle Array Application**

| Time (min) | 26.2μg PEPTIDE, 12.5% Formulation, 22°C Arrays, 5 Minutes Application, Clippers and Nair | | | | | | | | | |
|---|---|---|---|---|---|---|---|---|---|---|
| | Rat 1 | Rat 2 | Rat 3 | Rat 4 | Rat 5 | Rat 6 | Rat 7 | Rat 8 | **Mean** | **SD** |
| 5 | 30.01 | 23.97 | 40.60 | 37.68 | 41.01 | NA | NA | NA | **34.65** | **7.43** |
| 15 | NA | NA | NA | 71.00 | 28.44 | 39.18 | 51.08 | 30.15 | **43.97** | **17.58** |
| 30 | 14.63 | 8.45 | 20.51 | NA | NA | NA | 26.97 | 16.01 | **17.31** | **6.91** |
| 45 | 10.87 | 35.06 | NA | 10.18 | 5.18 | 7.85 | NA | NA | **13.83** | **12.08** |
| 90 | NA | NA | 2.37 | NA | NA | 1.99 | 2.39 | 2.13 | **2.22** | **0.19** |

(continued)

| Time (min) | 57.7μg PEPTIDE, 12.5% Formulation, 22°C Arrays, 5 Minutes Application, Clippers and Nair | | | | | | | | | |
| | Rat 9 | Rat 10 | Rat 11 | Rat 12 | Rat 13 | Rat 14 | Rat 15 | Rat 16 | Mean | SD |
|---|---|---|---|---|---|---|---|---|---|---|
| 5 | 57.28 | 42.27 | 28.20 | 49.32 | 38.50 | NA | NA | NA | 43.11 | 11.00 |
| 15 | NA | NA | NA | 63.85 | 62.67 | 77.29 | 71.93 | 28.19 | 60.79 | 19.18 |
| 30 | 31.16 | 33.13 | 16.86 | NA | NA | NA | 101.44 | 18.13 | 40.14 | 35.05 |
| 45 | 50.12 | 45.01 | NA | 15.49 | 15.49 | 14.53 | NA | NA | 28.13 | 17.84 |
| 90 | NA | NA | 3.52 | NA | NA | >ULOQ* | 4.79 | 2.86 | 3.72 | 0.98 |

NA No serum sample was collected.
* Samples > ULOQ were excluded from the average and standard deviation.

**Table 10: 09RAD011: Concentrations of Peptide (ng/mL) in Serum after Single Peptide-Microneedle Array Application**

| Time (min) | 67.7μg Peptide, 16.7% Formulation, 22°C Arrays, 5 Minutes Application, Clippers and Nair | | | | | | | | | |
| | Rat 1 | Rat 2 | Rat 3 | Rat 4 | Rat 5 | Rat 6 | Rat 7 | Rat 8 | Mean | SD |
|---|---|---|---|---|---|---|---|---|---|---|
| 5 | 49.82 | 96.12 | 39.27 | 90.38 | 48.71 | NA | NA | NA | 64.86 | 26.32 |
| 15 | NA | NA | NA | 114.02 | 73.79 | 61.42 | 112.59 | 51.88 | 82.74 | 28.97 |
| 30 | 47.29 | 63.24 | 55.76 | NA | NA | NA | 63.65 | 26.58 | 51.30 | 15.35 |
| 45 | 21.77 | 25.81 | NA | 26.49 | 11.25 | 13.22 | NA | NA | 19.71 | 7.09 |
| 90 | NA | NA | 4.95 | NA | NA | 3.09 | 16.58 | 5.79 | 7.60 | 6.09 |

| Time (min) | 45.6μg Peptide, 20.5% Formulation, 22°C Arrays, 5 Minutes Application, Clippers and Nair | | | | | | | | | |
| | Rat 9 | Rat 10 | Rat 11 | Rat 12 | Rat 13 | Rat 14 | Rat 15 | Rat 16 | Mean | SD |
|---|---|---|---|---|---|---|---|---|---|---|
| 5 | 65.04 | 120.77 | >ULOQ* | 78.95 | >ULOQ* | NA | NA | NA | 88.25 | 29.01 |
| 15 | NA | NA | NA | 98.02 | 83.13 | 67.94 | 54.31 | 76.94 | 76.07 | 16.37 |
| 30 | 21.54 | 22.25 | 24.13 | NA | NA | NA | 76.66 | >ULOQ* | 36.15 | 27.03 |
| 45 | 16.78 | 13.58 | NA | >ULOQ* | 22.91 | 63.85 | NA | NA | 29.28 | 23.37 |
| 90 | NA | NA | 3.31 | NA | NA | 2.22 | 8.83 | >ULOQ* | 4.79 | 3.54 |

NA No serum sample was collected.
* Samples > ULOQ were excluded from the average and standard deviation.

**Table 11: 09RAD017: Concentrations of Peptide (ng/mL) in Serum after Single Peptide-Microneedle Array Application**

| Time (min) | 27.0μg Peptide, 16.7% Formulation, 22°C Arrays, 5 Minutes Application, Clippers and Nair | | | | | | | | | |
| | Rat 1 | Rat 2 | Rat 3 | Rat 4 | Rat 5 | Rat 6 | Rat 7 | Rat 8 | Mean | SD |
|---|---|---|---|---|---|---|---|---|---|---|
| 5 | 21.97 | 12.91 | 22.89 | 15.04 | 29.03 | NA | NA | NA | 20.37 | 6.48 |
| 15 | NA | NA | NA | 16.66 | 27.09 | 32.52 | 34.7 | 44.15 | 31.02 | 10.12 |
| 30 | 16.70 | 5.43 | 16.3 | NA | NA | NA | 26.29 | 25.81 | 18.11 | 8.55 |
| 45 | 9.18 | 3.13 | NA | 6.07 | ND | 9.65 | NA | NA | 7.01 | 3.03 |
| 90 | NA | NA | 1.51 | NA | NA | 2.86 | 2.85 | 1.81 | 2.26 | 0.70 |

| Time (min) | 27.0μg Peptide, 16.7% Formulation, 22°C Arrays, 1 Minute Application, Clippers and Nair | | | | | | | | | |
| | Rat 9 | Rat 10 | Rat 11 | Rat 12 | Rat 13 | Rat 14 | Rat 15 | Rat 16 | Mean | SD |
|---|---|---|---|---|---|---|---|---|---|---|
| 5 | 28.25 | 36.64 | 29.63 | 33.75 | 34.54 | NA | NA | NA | 32.56 | 3.51 |
| 15 | NA | 34.82 | NA | 35.30 | 38.39 | NA | 63.49 | 37.14 | 41.83 | 12.19 |
| 30 | 8.24 | 15.67 | 14.69 | NA | NA | NA | 11.19 | 16.03 | 13.16 | 3.35 |
| 45 | 5.08 | 8.72 | NA | 19.30 | 13.59 | 9.09 | NA | NA | 11.16 | 5.46 |
| 90 | NA | NA | 2.49 | NA | NA | 2.16 | 2.52 | 3.42 | 2.65 | 0.54 |

(continued)

| Time (min) | 27.0μg peptide, 16.7% Formulation, 22°C Arrays, 5 Minutes Application, Clippers | | | | | | | | | |
| | Rat 17 | Rat 18 | Rat 19 | Rat 20 | Rat 21 | Rat 22 | Rat 23 | Rat 24 | Mean | SD |
| 5 | 30.25 | 23.99 | 31.73 | 36.63 | 30.88 | NA | NA | NA | 30.70 | 4.51 |
| 15 | NA | NA | NA | 50.74 | 37.31 | 37.26 | 43.22 | 33.29 | 40.36 | 6.80 |
| 30 | 16.55 | 8.11 | 24.13 | NA | NA | NA | 34.64 | 20.01 | 20.69 | 9.78 |
| 45 | 10.99 | 9.29 | NA | 21.07 | 11.81 | 17.55 | NA | NA | 14.14 | 4.96 |
| 90 | NA | NA | 5.37 | NA | NA | 5.10 | 5.63 | 4.49 | 5.15 | 0.49 |
| Time (min) | 32.0μg Peptide, 20.0% Formulation, 22°C Arrays, 5 Minutes Application, Clippers and Nair | | | | | | | | | |
| | Rat 25 | Rat 26 | Rat 27 | Rat 28 | Rat 29 | Rat 30 | Rat 31 | Rat 32 | Mean | SD |
| 5 | 37.27 | 65.27 | 36.54 | 38.57 | 48.21 | NA | NA | NA | 45.17 | 12.18 |
| 15 | NA | NA | NA | 40.05 | 63.34 | 35.48 | 65.64 | 41.70 | 49.24 | 14.13 |
| 30 | 11.12 | 25.01 | 10.29 | NA | NA | NA | 26.60 | 21.55 | 18.91 | 7.72 |
| 45 | 10.57 | 17.45 | NA | 13.33 | 14.29 | 12.44 | NA | NA | 13.62 | 2.54 |
| 90 | NA | NA | 4.03 | NA | NA | 4.96 | 6.40 | 4.73 | 5.03 | 1.00 |

NA No serum sample was collected.
ND Value not determined for this sample.

**Table 12: 09RAD018: Concentrations of Peptide (ng/mL) in Serum after Single Peptide-Microneedle Array Application**

| Time (min) | 141.9μg Peptide, 59.3% Formulation, 22°C Arrays, 5 Minutes Application, Clippers and Nair | | | | | | | | | |
| | Rat 1 | Rat 2 | Rat 3 | Rat 4 | Rat 5 | Rat 6 | Rat 7 | Rat 8 | Mean | SD |
| 5 | 106.49 | 195.93 | 225.47 | 150.49 | 250.49 | NA | NA | NA | 185.77 | 57.87 |
| 15 | NA | NA | NA | 126.21 | 316.07 | 182.28 | 265.36 | 259.68 | 229.92 | 75.15 |
| 30 | 103.52 | 222.88 | 240.82 | NA | NA | NA | 261.04 | 264.60 | 218.57 | 66.47 |
| 45 | 85.34 | 129.88 | NA | 50.93 | 102.01 | 90.99 | NA | NA | 91.83 | 28.58 |
| 90 | NA | NA | 19.81 | NA | NA | 12.33 | 15.98 | 46.54 | 23.67 | 15.55 |
| Time (min) | 386.5μg Peptide, 59.3% Formulation, 22°C Arrays, 5 Minutes Application, Clippers and Nair | | | | | | | | | |
| | Rat 9 | Rat 10 | Rat 11 | Rat 12 | Rat 13 | Rat 14 | Rat 15 | Rat 16 | Mean | SD |
| 5 | 138.64 | 70.87 | 37.50* | 164.17 | 76.41 | NA | NA | NA | 97.52 | 52.18 |
| 15 | NA | NA | NA | 196.20 | 108.08 | 77.48 | 116.62 | 168.28 | 133.33 | 47.98 |
| 30 | 79.65 | 107.15 | 31.48 | NA | NA | NA | 79.86 | 78.65 | 75.36 | 27.32 |
| 45 | 45.92 | 57.53 | NA | 55.94 | 29.86 | 21.69 | NA | NA | 42.19 | 15.89 |
| 90 | NA | NA | 7.50* | NA | NA | 7.50* | 7.62 | 7.50* | 7.53 | 0.06 |

NA No serum sample was collected.
* Samples < LLOQ. Reported as equal to the dilution factor times the LLOQ (0.25 ng/ml).

**Table 13: 09RAD030: Concentrations of Peptide (ng/mL) in Serum after Single Peptides Microneedle Array Application**

| Time (min) | 27.0μg Peptide, 16.7% Formulation, 22°C Arrays, 1 Minute Application, Clippers | | | |
| | Rat 1 | Rat 2 | Mean | SD |
| 5 | 17.13 | 25.55 | 21.34 | 5.96 |
| 10 | ND | 58.30 | 58.30 | NA |
| 15 | 28.85 | 30.55 | 29.70 | 1.20 |
| 30 | 14.00 | 10.37 | 12.19 | 2.56 |
| 45 | 12.45 | 5.76 | 9.10 | 4.73 |

(continued)

| Time (min) | 32.0μg Peptide, 20.0% Formulation, 22°C Arrays, 1 Minute Application, Clippers | | | | |
| | Rat 3 | Rat 5 | Rat 6 | Mean | SD |
|---|---|---|---|---|---|
| 5 | 20.19 | 29.96 | ND | 25.07 | 6.91 |
| 10 | 36.24 | ND | 32.49 | 34.36 | 2.65 |
| 15 | 44.12 | 29.62 | 28.20 | 33.98 | 8.81 |
| 30 | 23.48 | 13.76 | 16.11 | 17.78 | 5.07 |
| 45 | 19.78 | 8.47 | 9.73 | 12.66 | 6.20 |

| Time (min) | 26.2μg Peptide, 12.5% Formulation, 22°C Arrays, 1 Minute Application, Clippers | | |
| | Rat 4 | Mean | SD |
|---|---|---|---|
| 5 | 41.15 | 41.15 | NA |
| 10 | ND | NA | NA |
| 15 | 42.82 | 42.82 | NA |
| 30 | 17.40 | 17.40 | NA |
| 45 | 12.48 | 12.48 | NA |

| Time (min) | 27.0μg Peptide, 16.7% Formulation, 22°C Arrays, 5 Minute Application, Clippers | | |
| | Rat 7 | Rat 8 | Mean | SD |
|---|---|---|---|---|
| 5 | 34.89 | 19.92 | 27.41 | 10.59 |
| 10 | 31.54 | 15.19 | 23.37 | 11.56 |
| 15 | 33.48 | 28.67 | 31.08 | 3.40 |
| 30 | 15.02 | 9.85 | 12.43 | 3.65 |
| 45 | 7.89 | 6.08 | 6.99 | 1.28 |

| Time (min) | 32.0μg Peptide, 20.0% Formulation, 22°C Arrays, 5 Minute Application, Clippers | | | | |
| | Rat 9 | Rat 11 | Rat 12 | Mean | SD |
|---|---|---|---|---|---|
| 5 | 32.69 | 35.12 | 45.46 | 37.76 | 6.78 |
| 10 | 25.74 | 34.05 | 30.05 | 29.94 | 4.15 |
| 15 | 42.39 | 47.95 | 22.10 | 37.48 | 13.61 |
| 30 | 15.22 | 17.34 | 15.40 | 15.99 | 1.18 |
| 45 | 9.23 | 8.62 | 7.33 | 8.39 | 0.97 |

| Time (min) | 26.2μg Peptide, 12.5% Formulation, 22°C Arrays, 5 Minute Application, Clippers | | |
| | Rat 10 | Mean | SD |
|---|---|---|---|
| 5 | 45.29 | 45.29 | NA |
| 10 | 26.74 | 26.74 | NA |
| 15 | 59.72 | 59.72 | NA |
| 30 | 20.15 | 20.15 | NA |
| 45 | 11.63 | 11.63 | NA |

ND Value not determined for this sample.
NA Parameter cannot be calculated.

**Table 14: 09RAD030 Concentrations of Peptide (ng/mL) in Serum after Single Peptide-Microneedle Array Application Adjusted to 20 μg/kg**

| Time (min) | 1 Minute Application Animals Adjusted to a 20 μg/kg Dose | | | | | | | |
| | Rat 1 | Rat 2 | Rat 3 | Rat 4 | Rat 5 | Rat 6 | Mean | SD |
|---|---|---|---|---|---|---|---|---|
| 5 | 3.30 | 4.79 | 3.28 | 8.36 | 4.87 | ND | 4.92 | 2.07 |
| 10 | ND | 10.93 | 5.89 | ND | ND | 5.06 | 7.29 | 3.18 |
| 15 | 5.56 | 5.72 | 7.17 | 8.70 | 4.81 | 4.39 | 6.06 | 1.61 |
| 30 | 2.70 | 1.94 | 3.81 | 3.53 | 2.24 | 2.51 | 2.79 | 0.74 |

(continued)

| Time (min) | 1 Minute Application Animals Adjusted to a 20 μg/kg Dose | | | | | | | |
|---|---|---|---|---|---|---|---|---|
| | Rat 1 | Rat 2 | Rat 3 | Rat 4 | Rat 5 | Rat 6 | **Mean** | **SD** |
| 45 | 2.40 | 1.08 | 3.21 | 2.54 | 1.38 | 1.51 | **2.02** | **0.82** |
| Time (min) | 5 Minute Application Animals Adjusted to a 20 μg/kg Dose | | | | | | | |
| | Rat 7 | Rat 8 | Rat 9 | Rat 10 | Rat 11 | Rat 12 | **Mean** | **SD** |
| 5 | 6.64 | 3.90 | 5.48 | 9.27 | 5.77 | 7.64 | **6.45** | **1.86** |
| 10 | 6.00 | 2.97 | 4.31 | 5.47 | 5.60 | 5.05 | **4.90** | **1.11** |
| 15 | 6.37 | 5.61 | 7.10 | 12.22 | 7.88 | 3.72 | **7.15** | **2.86** |
| 30 | 2.86 | 1.93 | 2.55 | 4.12 | 2.85 | 2.59 | **2.82** | **0.72** |
| 45 | 1.50 | 1.19 | 1.55 | 2.38 | 1.42 | 1.23 | **1.54** | **0.43** |
| ND Value not determined for this sample. | | | | | | | | |

**Table 15: 09RAD048 Concentrations of Peptide (ng/mL) in Serum after Single Peptide-Microneedle Array Application**

| Time (min) | 9.8μg Peptide, 50% Formulation, 22°C Arrays, 5 Minute Application, Clippers | | | | | | | | | | | | | |
|---|---|---|---|---|---|---|---|---|---|---|---|---|---|---|
| | Rat 1 | Rat 2 | Rat 3 | Rat 4 | Rat 5 | Rat 6 | Rat 7 | Rat 8 | Rat 9 | Rat 10 | Rat 11 | Rat 12 | **Mean** | **SD** |
| 5 | 14.15 | 5.82 | 5.21 | 20.6 | 11.42 | 14.79 | NA | NA | NA | NA | NA | NA | **12.00** | **5.85** |
| 15 | NA | NA | NA | NA | NA | NA | 17.16 | 11.33 | 8.82 | 5.87 | 7.42 | 7.29 | **9.65** | **4.12** |
| 30 | 4.92 | 2.54 | 2.73 | 7.00 | 5.79 | 6.75 | NA | NA | NA | NA | NA | NA | **4.96** | **1.94** |
| 45 | NA | NA | NA | NA | NA | NA | 1.61 | 2.38 | 1.79 | 1.58 | 2.24 | 2.84 | **2.07** | **0.50** |
| 60 | NA | NA | NA | NA | NA | NA | 1.82 | 1.37 | 1.24 | 1.34 | 1.68 | 1.34 | **1.47** | **0.23** |
| 90 | 0.63 | 0.30 | 0.36 | 0.83 | 0.73 | 0.85 | NA | NA | NA | NA | NA | NA | **0.62** | **0.24** |
| 120 | 0.51 | 0.15 | 0.22 | 0.45 | 0.42 | 0.54 | NA | NA | NA | NA | NA | NA | **0.38** | **0.16** |

| Time (min) | 54μg Peptide, 50% Formulation, 22°C Arrays, 5 Minute Application, Clippers | | | | | | | | | | | | | |
|---|---|---|---|---|---|---|---|---|---|---|---|---|---|---|
| | Rat 13 | Rat 14 | Rat 15 | Rat 16 | Rat 17 | Rat 18 | Rat 19 | Rat 20 | Rat 21 | Rat 22 | Rat 23 | Rat 24 | **Mean** | **SD** |
| 5 | 12.34 | 19.45 | 33.03 | 19.15 | 6.64 | 8.28 | NA | NA | NA | NA | NA | NA | **16.48** | **9.71** |
| 15 | NA | NA | NA | NA | NA | NA | 42.24 | 54.92 | 64.23 | 46.83 | 64.88 | 25.65 | **49.79** | **14.91** |
| 30 | 17.62 | 20.41 | 32.65 | 13.11 | 2.08 | 16.95 | NA | NA | NA | NA | NA | NA | **17.14** | **9.95** |
| 45 | NA | NA | NA | NA | NA | NA | 4.53 | 9.08 | 12.79 | 8.85 | 14.36 | 6.60 | **9.37** | **3.69** |
| 60 | NA | NA | NA | NA | NA | NA | 3.54 | 9.02 | 12.02 | 7.41 | 11.12 | 3.41 | **7.75** | **3.68** |
| 90 | 3.17 | 1.36 | 1.55 | 0.85 | 0.65* | 1.11 | NA | NA | NA | NA | NA | NA | **1.45** | **0.90** |
| 180 | 0.22 | 0.31 | 0.32 | 0.14 | 0.13* | 0.76 | NA | NA | NA | NA | NA | NA | **0.31** | **0.23** |

NA No serum sample was collected.

* Samples < LLOQ. Reported as equal to the dilution factor times the LLOQ (0.13 ng/ml).

**Table 16: 09RAD053: Concentrations of [Glu$^{22,25}$, Leu$^{23,28,31}$, Aib$^{29}$, Lys$^{26,30}$]hPTHrP(1-34)NH$_2$ (ng/mL) in Serum after Single Peptide-Microneedle Array Application**

| Time (min) | 141.9μg Peptide, 59.3% Formulation, 22°C Arrays, 5 Minutes Application, Clippers | | | | | | | |
|---|---|---|---|---|---|---|---|---|
| | Rat 1 | Rat 2 | Rat 3 | Rat 4 | Rat 5 | Rat 6 | **Mean** | **SD** |
| 5 | 32.75 | 66.83 | 47.47 | 71.45 | 39.91 | 51.47 | **51.65** | **15.07** |
| 15 | 233.68 | 311.26 | 132.89 | 122.56 | 46.41 | 75.65 | **153.74** | **100.23** |
| 30 | 138.55 | 144.30 | 108.00 | 84.37 | 20.03 | 48.51 | **90.63** | **49.55** |
| 45 | 64.65 | 63.12 | 65.58 | 39.47 | 8.93 | 23.27 | **44.17** | **24.24** |
| 90 | 10.15 | 11.38 | 5.84 | 4.72 | 1.31 | 3.20 | **6.10** | **3.94** |
| 120 | 4.34 | 4.62 | 2.50 | 2.18 | 0.55 | 1.73 | **2.65** | **1.56** |
| Time (min) | 386.5μg Peptide, 59.3% Formulation, 22°C Arrays, 5 Minutes Application, Clippers | | | | | | | |
| | Rat 7 | | | | | | **Mean** | **SD** |
| 5 | 74.28 | | | | | | **74.28** | **ND** |
| 15 | 179.02 | | | | | | **179.02** | **ND** |
| 30 | 148.40 | | | | | | **148.40** | **ND** |
| 45 | 58.80 | | | | | | **58.80** | **ND** |
| 90 | 6.84 | | | | | | **6.84** | **ND** |
| 120 | 2.88 | | | | | | **2.88** | **ND** |
| ND Parameter cannot be calculated. | | | | | | | | |

**Table 17: Pharmacokinetic Parameters of [Glu$^{22,25}$, Leu$^{23,28,31}$, Aib$^{29}$, Lys$^{26,30}$]hPTHrP(1-34)NH$_2$ Exposure in Rats Dosed with a Single [Glu$^{22,25}$, Leu$^{23,28,31}$, Aib$^{29}$, Lys$^{26,30}$]hPTHrP(1-34)NH$_2$-Microneedle Array Application**

| Study Number | Dose | Formulation | | $AUC_{0-t}$° | $C_{max}$ | $C_{max}$ (20 μg/kg Dose) | $T_{max}$ | $T_{1/2}$ | %F* |
|---|---|---|---|---|---|---|---|---|---|
| | (μg) | (% Peptide) | (% HEC) | (ng*min/mL) | (ng/mL) | (ng/mL) | (min) | (min) | (%) |
| 09RAD005 | 20.0 | 5.0 | 4.5 | 576 | 11.94 | 3.36 | 15 | 43 | 47 |
| 09RAD006 | 31.0 | 15.0 | 4.0 | 750 | 23.58 | 4.58 | 15 | 17 | 42 |
| | 22.0 | 10.0 | 4.5 | 465 | 17.13 | 4.51 | 5 | 19 | 36 |
| 09RAD010 | 26.2 | 12.5 | 4.5 | 1447 | 43.97 | 10.60 | 15 | 18 | 101 |
| | 57.7 | 12.5 | 4.5 | 2505 | 60.79 | 6.83 | 15 | 18 | 82 |
| 09RAD011 | 67.7 | 16.7 | 4.0 | 2890 | 82.74 | 7.29 | 15 | 22 | 74 |
| | 45.6 | 20.5 | 3.5 | 2920 | 88.25 | 11.24 | 5 | 20 | 108 |
| 09RAD017 | 27.0 | 16.7 | 4.0 | 1022 | 31.02 | 7.95 | 15 | 20 | 76 |
| | 27.0 | 16.7 | 4.0 | 1277 | 41.83 | 10.59 | 15 | 20 | 94 |
| | 27.0 | 16.7 | 4.0 | 1508 | 40.36 | 10.09 | 15 | 26 | 109 |
| | 32.0 | 20.0 | 3.5 | 1647 | 49.24 | 10.48 | 15 | 25 | 102 |
| 09RAD018 | 141.9 | 59.3 | 0.0 | 10369 | 229.92 | 10.60 | 15 | 21 | 139 |
| | 386.5 | 59.3 | 0.0 | 4533 | 118.39 | 2.24 | 15 | 18 | 22 |

(continued)

| Study Number | Dose | Formulation | (% HEC) | AUC$_{0\text{-}t}$° | C$_{max}$ | C$_{max}$ (20 μg/kg Dose) | T$_{max}$ | T$_{1/2}$ | %F* |
|---|---|---|---|---|---|---|---|---|---|
| | (μg) | (% Peptide) | | (ng*min/mL) | (ng/mL) | (ng/mL) | (min) | (min) | (%) |
| 09RAD030 | 1 minute groups adjusted to 20 μg/kg | | | 166 | 7.29 | 7.29 | 10 | 18 | 69 |
| | 5 minute groups adjusted to 20 μg/kg | | | 166 | 7.15 | 7.15 | 15 | 14 | 69 |
| 09RAD048 | 9.8 | 49.0 | 0 | 343 | 12.00 | 6.49 | 5 | 22 | 49 |
| | 54.0 | 49.0 | 0. 0 | 1378 | 49.79 | 5.59 | 15 | 24 | 37 |
| 09RAD053 | 141.9 | 59.3 | 0. 0 | 5199 | 153.74 | 5.81 | 15 | 17 | 51 |
| | 386.5 | 59.3 | 0. 0 | 6899 | 179.02 | 2.44 | 15 | 16 | 24 |

° AUC from 0-90 minutes for all studies except 09RAD030, 09RAD048, and 09RAD053. AUC for 09RAD030 is from 0-45 minutes. AUC for 09RAD048 Group 1 and 09RAD053 is 0-120 minutes and 09RAD048 Group 2 is 0-180 minutes.

**Evaluation of different array materials and array contact times in Sprague Dawley rats**

**Study Design**

Several additional single dose pharmacokinetic studies were performed in Sprague-Dawley rats. The studies investigated the effect of different skin contact times for polycarbonate (PC) and liquid crystal polymer (LCP) microneedle arrays and the effect of different microneedle array dose loads. Microneedle arrays were coated with aqueous formulations of 40 to 60 wt-% [Glu$^{22,25}$, Leu$^{23,28,31}$, Aib$^{29}$, Lys$^{26,30}$]PTHrP(1-34)NH$_2$ and phosphate buffered saline.

General characteristics of PC and LCP microneedles are shown below in Table 18, and present results from depth of penetration tests performed with uncoated microneedle patches (i.e., arrays did not contain any peptide drug loading).

## Table 18: Polycarbonate ("PC") Microneedle Arrays and Liquid Crystal Polymer ("LCP") Microneedle Arrays

| Microneedle Arrays | | |
|---|---|---|
| Material of Construction | Polycarbonate | Liquid Crystal Polymer |
| Number of Microneedles | 366 | 316 |
| Flexural Modulus (by ISO 178) | 2300 | 9100 |
| Grade | Class VI, medical grade polymer | Class VI, medical grade polymer |
| Surface area | 5.5 cm$^2$ or ~27 mm in diameter | 5.5 cm$^2$ or ~27 mm in diameter |
| Depth of Penetration (DOP) | 250+/-10μm | 250+/-10μm |
| Height of Microneedles | 500μm | 500μm |
| Spacing between Microneedles | 550μm apart (tip to tip) | 550μm apart (tip to tip) |

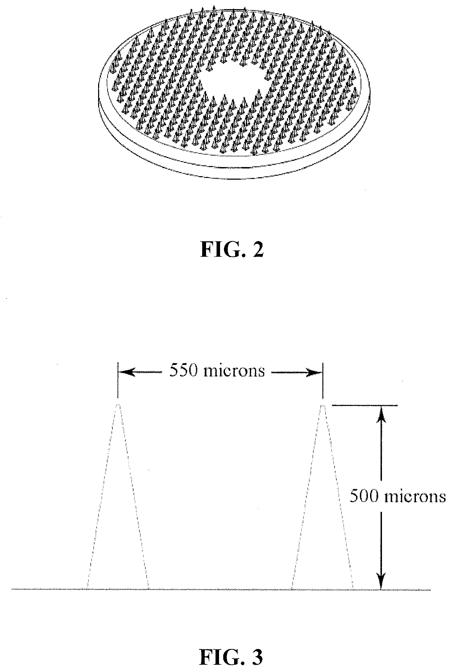

The LCP microneedle array is injection molded USP Class VI rated liquid crystal polymer resin (Ticona, Vectra® MT1300). The array is a circular disc with an overall surface area of 1.27 cm$^2$ or ~12.7 mm in diameter, containing approximately 316 pyramid-shaped microstructures on one side of the disc. An image of the LCP microarray is set forth in Figure 2.

For the LCP array, each microstructure is approximately 500μm tall. The microstructures are spaced approximately 550μm apart (tip to tip) in a geometric pattern. As side view with dimension of the microstructures is set forth in Figure 3.

## Dose Administration

Typically, one day prior to dosing, an area just above the hind legs on the dorsal side of the rat was shaved using electric clippers. Nair® hair removal lotion was then applied to the area for 5 to 8 minutes to remove remaining hair stubble. The Nair lotion was removed completely using a cloth dampened with water. The next day the microneedle array was applied using the supplied spring loaded applicator. The microneedle array was left in contact with the skin for either five minutes before removal or removed almost immediately (typically 2 - 3 seconds after skin contact). During microneedle application and contact, the rats were manually restrained.

**Table 19: Studies RAD021, RAD022, RAD024**

| Study Number | Number of Animals | Lot Number | Array Material | Peptide Content§ (mcg) | Skin Contact Time (min) |
|---|---|---|---|---|---|
| 10RAD021 | 6 | 155342-016 | LCP | 103 | 5 |
| 10RAD022 | 6 | 155342-041 | LCP | 124 | 5 |
| 10RAD024 | 6 | 155342-064 | LCP | 56 | 5 |
| 10RAD026 | 6 | 155342-016 | LCP | 103 | 0.05 |
| 10RAD026 | 6 | 155342-041 | LCP | 124 | 0.05 |
| 10RAD026 | 6 | 155342-064 | LCP | 56 | 0.05 |
| 10RAD021 | 5 | 152986-035 | PC | 13.6 | 5 |
| 10RAD021 | 5 | 152986-035 | PC | 13.6 | 0.05 |
| 10RAD021 | 6 | 155342-033 | PC | 211 | 5 |
| 10RAD021 | 6 | 155342-033 | PC | 211 | 0.05 |

§ Peptide ([Glu$^{22,25}$, Leu$^{23,28,31}$, Aib$^{29}$, Lys$^{26,30}$]hPTHrP(1-34)NH$_2$) content calculated based on total peptide content including water and acetic acid. Actual peptide content is approximately 80% - 90% of the stated amount.

## Serum Sample Collection

Blood was collected from each rat at 5, 15, 30, 45 and 90 minutes after application of microneedle arrays, for LCP-microneedle arrays and at 1, 5, 15 and 30 minutes after application for PC-microneedle arrays. Approximately 600 $\mu$L of blood was collected via the catheter from the jugular vein using a syringe and needle from rats for their first four blood draws. For the last blood collection, animals were euthanized via CO$_2$ chamber and approximately 600 $\mu$L of blood was collected via cardiac puncture. The blood was immediately transferred to a serum separator tube that contained 12 $\mu$L of a 2.5 mg/ml aprotinin (Sigma) solution.

Surprisingly, application of the 155342-041 [Glu$^{22,25}$, Leu$^{23,28,31}$, Aib$^{29}$, Lys$^{26,30}$]hPTHrP(I-34)NH$_2$-LCP-micro-needle arrays to the rat for either 5 minutes or approximately 3 seconds (0.05 minutes) resulted in a similar PK profile, based on C$_{max}$, T$_{max}$, AUC and T$_{1/2}$. Similar results comparing a 5 minute application time with 0.05 minutes were obtained with two other LCP arrays (155342-016 and 155342-064) and two PC-microneedle arrays (155342-033 and 152986-035). Furthermore, comparison of individual animal data values between 0.05 and 5 minute wear suggests that variability is not necessarily increased with the short application time. This indicates that times of patch application wherein the patch is left in place after administration are useful through a wide range of drug doses.

In the following tables, BLQ means Below the Limit of Quantitation.

**Table 20: Concentrations of [Glu$^{22,25}$, Leu$^{23,28,31}$, Aib$^{29}$, Lys$^{26,30}$]hPTHrP(1-34)NH2 (ng/mL) in Serum after Single Microneedle Array Application (lot 155342-041; 124 mcg)**

| Time (min) | 5 minute Microneedle Array Skin Contact Time | | | | | | Mean | SD |
|---|---|---|---|---|---|---|---|---|
| | Rat 1 | Rat 2 | Rat 3 | Rat 4 | Rat 5 | Rat 6 | | |
| 5 | 42.4 | 20.3 | 33.6 | 62.7 | <BLQ | 12.1 | 34.2 | 19.7 |
| 15 | 96.2 | 63.3 | 98.8 | 92.4 | <BLQ | 73.9 | 84.9 | 15.6 |
| 30 | 92.5 | 30.1 | 43.1 | 59.7 | <BLQ | 41.4 | 53.4 | 24.3 |
| 45 | 38.2 | 14.2 | 24 | 27.5 | <BLQ | 19.9 | 24.8 | 9 |
| 90 | 18.9 | 4.8 | 8.5 | 12.8 | <BLQ | 7.7 | 10.5 | 5.5 |

(continued)

| Time (min) | 5 minute Microneedle Array Skin Contact Time | | | | | | | |
| | Rat 1 | Rat 2 | Rat 3 | Rat 4 | Rat 5 | Rat 6 | Mean | SD |
|---|---|---|---|---|---|---|---|---|
| $C_{max}$ | 96.2 | 63.3 | 98.8 | 92.4 | | 73.9 | 84.9 | 15.6 |
| $T_{max}$ | 15 | 15 | 15 | 15 | | 15 | 15 | 0 |
| $AUC_{5-90}$ | 4374 | 1878 | 2961 | 3478 | | 2375 | 3013 | 970 |
| $T_{1/2}$ | 30.1 | 20.8 | 22.3 | 26.5 | | 23.4 | 24.6 | 3.7 |
| | | | | | | | | |
| Time (min) | 0.05 minute Microneedle Array Skin Contact Time | | | | | | | |
| | Rat 1 | Rat 2 | Rat 3 | Rat 4 | Rat 5 | Rat 6 | Mean | SD |
| 5 | 87.9 | 41.1 | 53.6 | 18.3 | 15.9 | 33.4 | 41.8 | 26.5 |
| 15 | 197.5 | 76 | 117.4 | 52.2 | 33.4 | 88.8 | 94.1 | 58.4 |
| 30 | 119.1 | 52 | 80.4 | 37.9 | 18.8 | 82.3 | 65.1 | 36.1 |
| 45 | 61.5 | 22.3 | 41.6 | 23.8 | 11.2 | 55.9 | 36 | 20.1 |
| 90 | 10.6 | 3.6 | NA | NA | 1.5 | 10.9 | 6.6 | 4.8 |
| $C_{max}$ | 197.5 | 76 | 117.4 | 52.2 | 33.4 | 88.8 | 94.2 | 58.4 |
| $T_{max}$ | 15 | 15 | 15 | 15 | 15 | 15 | 15 | 0 |
| $AUC_{5-90}$ | 6779 | 2686 | 3253 | 1496 | 1146 | 4432 | 3299 | 2081 |
| $T_{1/2}$ | 17.6 | 16.6 | 20 | 26.5 | 16.6 | 23.4 | 20.1 | 4.1 |
| NA No serum sample was collected. | | | | | | | | |

Table 21: Concentrations of [Glu$^{22,25}$, Leu$^{23,28,31}$, Aib$^{29}$, Lys$^{26,30}$]hPTHrP(1-34)NH2 (ng/mL) in Serum after Single Microneedle Array Application (lot 155342-016; 103 mcg)

| Time (min) | 5 minute Microneedle Array Skin Contact Time | | | | | | | |
| | Rat 1 | Rat 2 | Rat 3 | Rat 4 | Rat 5 | Rat 6 | Mean | SD |
|---|---|---|---|---|---|---|---|---|
| 5 | 42.4 | 20.3 | 33.6 | 62.7 | <BLQ | 12.1 | 34.2 | 19.7 |
| 15 | 96.2 | 63.3 | 98.8 | 92.4 | <BLQ | 73.9 | 84.9 | 15.6 |
| 30 | 92.5 | 30.1 | 43.1 | 59.7 | <BLQ | 41.4 | 53.4 | 24.3 |
| 45 | 38.2 | 14.2 | 24 | 27.5 | <BLQ | 19.9 | 24.8 | 9 |
| 90 | 18.9 | 4.8 | 8.5 | 12.8 | <BLQ | 7.7 | 10.5 | 5.5 |
| $C_{max}$ | 96.2 | 63.3 | 98.8 | 92.4 | | 73.9 | 84.9 | 15.6 |
| $T_{max}$ | 15 | 15 | 15 | 15 | | 15 | 15 | 0 |
| $AUC_{5-90}$ | 4374 | 1878 | 2961 | 3478 | | 2375 | 3013 | 970 |
| $T_{1/2}$ | 30.1 | 20.8 | 22.3 | 26.5 | | 23.4 | 24.6 | 3.7 |
| Time (min) | 0.05 minute Microneedle Array Skin Contact Time | | | | | | | |
| | Rat 1 | Rat 2 | Rat 3 | Rat 4 | Rat 5 | Rat 6 | Mean | SD |
| 5 | 87.9 | 41.1 | 53.6 | 18.3 | 15.9 | 33.4 | 41.8 | 26.5 |
| 15 | 197.5 | 76 | 117.4 | 52.2 | 33.4 | 88.8 | 94.1 | 58.4 |
| 30 | 119.1 | 52 | 80.4 | 37.9 | 18.8 | 82.3 | 65.1 | 36.1 |
| 45 | 61.5 | 22.3 | 41.6 | 23.8 | 11.2 | 55.9 | 36 | 20.1 |
| 90 | 10.6 | 3.6 | NA | NA | 1.5 | 10.9 | 6.6 | 4.8 |
| $C_{max}$ | 197.5 | 76 | 117.4 | 52.2 | 33.4 | 88.8 | 94.2 | 58.4 |
| $T_{max}$ | 15 | 15 | 15 | 15 | 15 | 15 | 15 | 0 |
| $AUC_{5-90}$ | 6779 | 2686 | 3253 | 1496 | 1146 | 4432 | 3299 | 2081 |

(continued)

| Time (min) | 0.05 minute Microneedle Array Skin Contact Time | | | | | | | |
| | Rat 1 | Rat 2 | Rat 3 | Rat 4 | Rat 5 | Rat 6 | Mean | SD |
|---|---|---|---|---|---|---|---|---|
| $T_{1/2}$ | 17.6 | 16.6 | 20 | 26.5 | 16.6 | 23.4 | 20.1 | 4.1 |
| NA No serum sample was collected. | | | | | | | | |

Table 22: Concentrations of [Glu$^{22,25}$, Leu$^{23,28,31}$, Aib$^{29}$, Lys$^{21,30}$]hPTHrP(1-34)NH$_2$ (ng/mL) in Serum after Single Microneedle Array Application (lot 155342-064; 56 mcg)

| Time (min) | 5 minute Microneedle Array Skin Contact Time | | | | | | | |
| | Rat 1 | Rat 2 | Rat 3 | Rat 4 | Rat 5 | Rat 6 | Mean | SD |
|---|---|---|---|---|---|---|---|---|
| 5 | 31.8 | 32.3 | 39.2 | 29.7 | 14.8 | 30.1 | 29.7 | 8.0 |
| 15 | 31.3 | 57.2 | 58.5 | 34.7 | 20.6 | 57.1 | 43.2 | 16.4 |
| 30 | 26.8 | 49.5 | 55.9 | 21.0 | 10.7 | 26.6 | 31.7 | 17.4 |
| 45 | 8.5 | 21.1 | 15.4 | 9.0 | 4.3 | 7.9 | 11.0 | 6.1 |
| 90 | 4.6 | 5.2 | 3.9 | 2.5 | 0.9 | 2.5 | 3.3 | 1.6 |
| $C_{max}$ | 31.8 | 57.2 | 58.5 | 34.7 | 20.6 | 57.1 | 43.3 | 16.3 |
| $T_{max}$ | 5 | 15 | 15 | 15 | 15 | 15 | 13 | 4 |
| $AUC_{6-90}$ | 1309 | 2352 | 2317 | 1225 | 643 | 1556 | 1567 | 666 |
| $T_{1/2}$ | 27.5 | 20.7 | 18.1 | 19.8 | 16.9 | 16.9 | 20.0 | 4.0 |
| | 31.8 | 32.3 | 39.2 | 29.7 | 14.8 | 30.1 | 29.7 | 8.0 |
| Time (min) | 0.05 minute Microneedle Array Skin Contact Time | | | | | | | |
| | Rat 1 | Rat 2 | Rat 3 | Rat 4 | Rat 5 | Rat 6 | Mean | SD |
| 5 | 25.3 | 23.6 | 25.7 | 14.5 | 16.8 | 25.6 | 21.9 | 5.0 |
| 15 | 30.8 | 29.8 | 57.0 | 24.7 | 33.9 | 44.7 | 36.8 | 11.9 |
| 30 | 16.6 | 16.1 | 33.8 | 11.3 | 19.2 | 28.3 | 20.9 | 8.5 |
| 45 | 9.9 | 10.2 | 20.0 | 7.2 | 9.6 | 13.4 | 11.7 | 4.5 |
| 90 | 1.6 | 1.5 | 5.2 | 1.9 | 2.6 | 2.3 | 2.5 | 1.4 |
| $C_{max}$ | 30.8 | 29.8 | 57.0 | 24.7 | 33.9 | 44.7 | 36.8 | 11.9 |
| $T_{max}$ | 15 | 15 | 15 | 15 | 15 | 15 | 15 | 0 |
| $AUC_{5-90}$ | 1092 | 1073 | 2067 | 810 | 1142 | 1563 | 1291 | 451 |
| $T_{1/2}$ | 17.8 | 17.5 | 21.9 | 21.0 | 20.3 | 17.1 | 19.3 | 2.0 |

Table 23: Concentrations of [Glu$^{22,25}$, Leu$^{23,28,31}$, Aib$^{29}$, Lys$^{21,30}$]hPTHrP(1-34)NH2 (ng/mL) in Serum after Single Microneedle Array Application (lot 155342-033; 211 mcg)

| Time (min) | 5 minute Microneedle Array Skin Contact Time | | | | | | | |
| | Rat 1 | Rat 2 | Rat 3 | Rat 4 | Rat 5 | Rat 6 | Mean | SD |
|---|---|---|---|---|---|---|---|---|
| 5 | 98.5 | 58.9 | 36.4 | 39.8 | <BLQ | 49.1 | 48.8 | 29.4 |
| 15 | 149.5 | 55.5 | 55.6 | 128.1 | <BLQ | 66.0 | 77.4 | 51.8 |
| 30 | 211.6 | 41.4 | 36.8 | 135.0 | 18.9 | 86.1 | 88.3 | 73.6 |
| $C_{max}$ | 211.6 | 58.9 | 55.6 | 135 | 18.9 | 86.1 | 94.4 | 69.2 |
| $T_{max}$ | 30 | 5 | 15 | 30 | 30 | 30 | 23 | 11 |
| $AUC_{5-30}$ | 4195 | 1446 | 1244 | 2912 | 338 | 1839 | 1996 | 1365 |
| | | | | | | | | |

(continued)

| Time (min) | 0.05 minute Microneedle Array Skin Contact Time | | | | | | Mean | SD |
|---|---|---|---|---|---|---|---|---|
| | Rat 1 | Rat 2 | Rat 3 | Rat 4 | Rat 5 | Rat 6 | | |
| 1 | 11.3 | 16.5 | 12.8 | 34.9 | 49.5 | 26.7 | **25.3** | 14.9 |
| 5 | 33.4 | 34.8 | 21.3 | 72.1 | 54.1 | 40.2 | **42.6** | 17.9 |
| 15 | 51.9 | 47.7 | 43.0 | 164.3 | 156.9 | 71.9 | **89.3** | 56.1 |
| 30 | 75.3 | 50.2 | 42.4 | 195.1 | 143.1 | 64.2 | **95.0** | 60.8 |
| $C_{max}$ | 75.3 | 50.2 | 43.0 | 195.1 | 156.9 | 71.9 | **98.7** | 62.3 |
| $T_{max}$ | 30 | 30 | 15 | 30 | 15 | 15 | **23** | 8 |
| $AUC_{5-30}$ | 1475 | 1258 | 1036 | 4108 | 3537 | 1729 | **2190** | 1298 |

Table 24: Concentrations of [Glu$^{22,25}$, Leu$^{23,28,31}$, Aib$^{29}$, Lys$^{26,30}$]hPTHrP(1-34)NH2 (ng/mL) in Serum after Single Microneedle Array Application (lot 152986-035; 13.6 mcg)

| Time (min) | 5 minute Microneedle Array Skin Contact Time | | | | | Mean | SD |
|---|---|---|---|---|---|---|---|
| | Rat 1 | Rat 2 | Rat 3 | Rat 4 | Rat 5 | | |
| 1 | 1.45 | 2.68 | 5.18 | 1.82 | 1.33 | **2.5** | 1.6 |
| 5 | 9.55 | 7.90 | 11.16 | 9.72 | 5.91 | **8.8** | 2.0 |
| 15 | 10.81 | 7.10 | 14.40 | 7.81 | 3.04 | **8.6** | 4.3 |
| 30 | 8.08 | 4.47 | 10.73 | 4.45 | 2.35 | **6.0** | 3.3 |
| $C_{max}$ | 10.8 | 7.9 | 14.4 | 9.7 | 5.9 | **9.7** | 3.2 |
| $T_{max}$ | 15 | 5 | 15 | 5 | 5 | **9** | 5 |
| $AUC_{5-90}$ | 266 | 184 | 352 | 204 | 100 | **221** | 94 |

| Time | 0.05 minute Microneedle Array Skin Contact Time | | | | | | |
|---|---|---|---|---|---|---|---|
| (min) | Rat 1 | Rat 2 | Rat 3 | Rat 4 | Rat 5 | Mean | SD |
| 1 | 7.68 | 5.49 | 4.76 | 7.15 | 5.91 | **6.2** | 1.2 |
| 5 | 12.11 | 12.21 | 11.28 | 12.13 | 11.23 | **11.8** | 0.5 |
| 15 | 8.90 | 10.00 | 7.71 | 14.41 | 11.25 | **10.5** | 2.6 |
| 30 | 7.39 | 7.97 | 4.66 | 9.38 | 6.11 | **7.1** | 1.8 |
| $C_{max}$ | 12.1 | 12.2 | 11.3 | 14.4 | 11.3 | **12.3** | 1.3 |
| $T_{max}$ | 5 | 5 | 5 | 15 | 15 | **9** | 5 |
| $AUC_{5-90}$ | 271 | 284 | 222 | 353 | 280 | **282** | 47 |

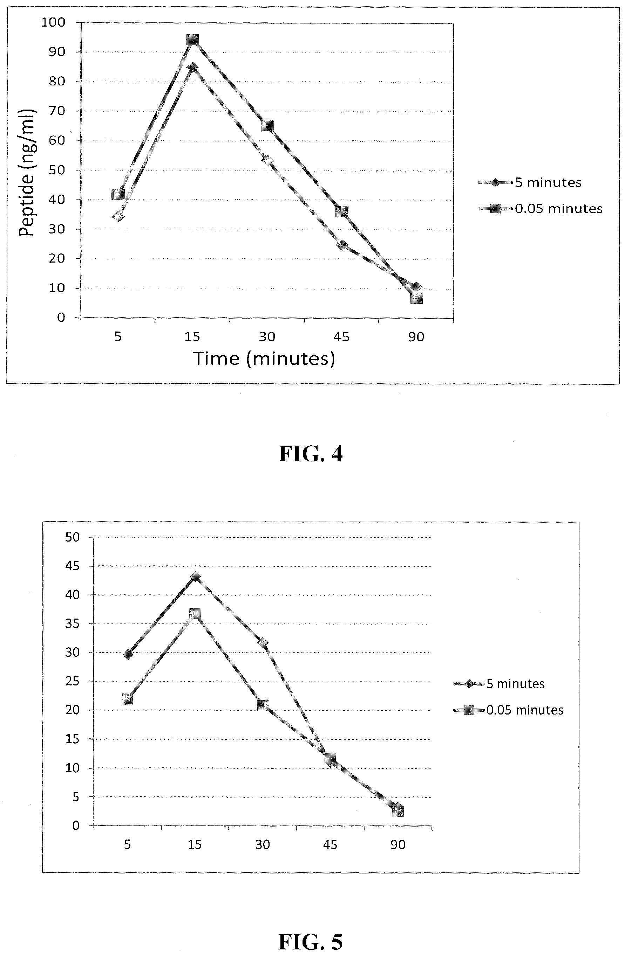

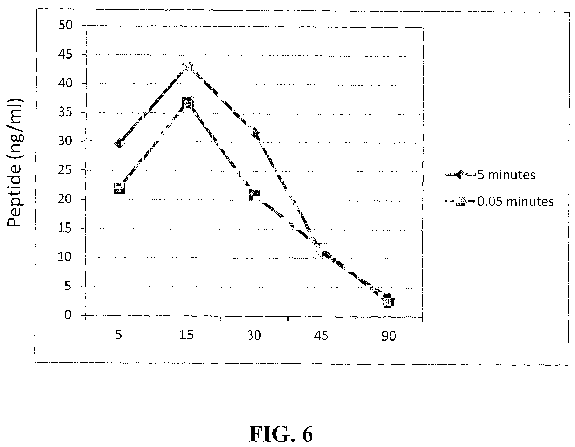

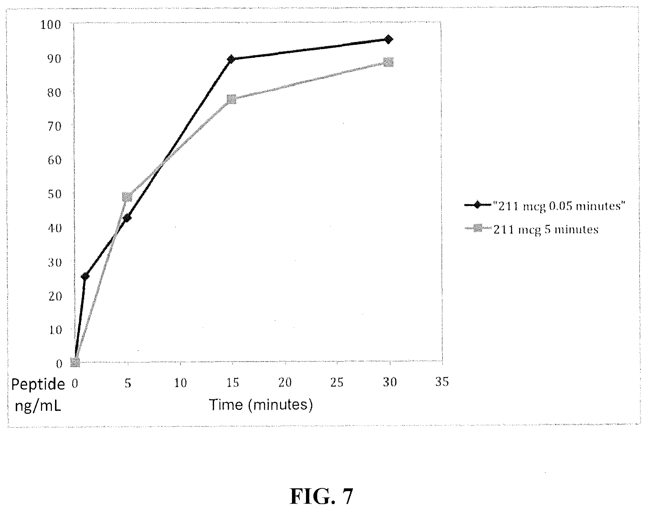

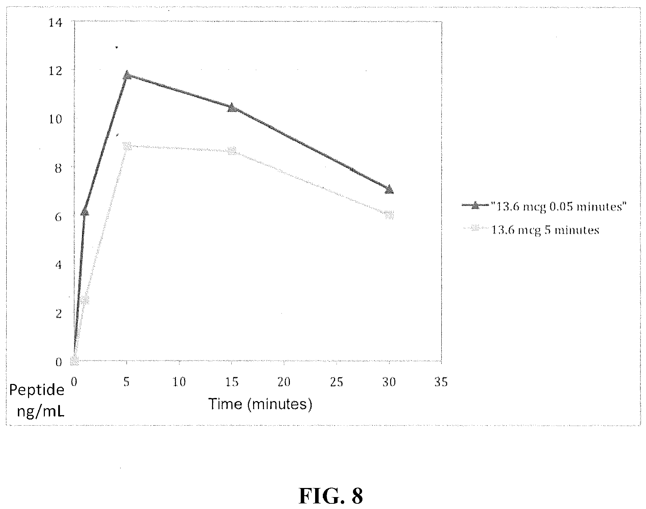

Graphs showing the mean concentrations of [Glu$^{22,25}$, Leu$^{23,28,31}$, Aib$^{29}$, Lys$^{21,30}$]hPTHrP(1-34)NH$_2$ (ng/mL) in serum versus time after single microneedle array application for the data in Tables 20-24 is presented in Figures 4-8.

**Evaluation of changes in bone mineral density and bone microstructure after repeat application of [Glu$^{22,25}$, Leu$^{23,28,31}$, Aib$^{29}$, Lys$^{21,30}$]hPTHrP(1-34)NH$_2$ PC Microneedle Arrays and [Glu$^{22,25}$, Leu$^{23,28,31}$, Aib$^{29}$, Lys$^{26,30}$]hP-THrP(1-34)NH2 LCP-Microneedle Arrays to osteopenic rats Study Design**

This study investigated the effect of [Glu$^{22,25}$, Leu$^{23,28,31}$, Aib$^{29}$, Lys$^{26,30}$]hPTHrP(1-34)NH$_2$-PC and LCP-microneedle arrays in an ovariectomy (OVX)-induced bone loss model in Sprague Dawley rats. Effects on the skeleton were assessed by measurement of changes in bone mineral density (BMD), using dual energy x-ray absorptiometry (DEXA) and bone micro-architecture, by micro-computed tomography (microCT).

Microneedle arrays were coated with aqueous formulations of [Glu$^{22,25}$, Leu$^{23,28,31}$, Aib$^{29}$, Lys$^{26,30}$]PTHrP(1-34)NH$_2$ and phosphate buffered saline.

**Table 25: Study treatment groups**

| Surgery | Dose ($\mu$g) | Number of Rats | Route of Dose Adminsitration |
|---|---|---|---|
| SHAM | 0 (Placebo) | 11 | - |
| OVX | 0 (Placebo) | 10 | PC microneedle array |
| OVX | 13.6 | 10 | PC microneedle array |
| OVX | 8.8 | 6 | LCP microneedle array |
| OVX | 0 (Placebo) | 11 | SC Injection |
| OVX | 12.7 | 11 | SC Injection |

*Animals*

Fifty nine female Sprague Dawley rats (CRL:CD; Charles River Laboratories) were singly housed in polycarbonate ventilated (45 ACH) cages. All rats were provided certified rodent diet (2918 from Harlan Teklad) and water *ad libitum.* The housing environment was maintained between 18-26°C with 30-70% relative humidity and a 12 hour light: 12 hour dark cycle. Rats underwent either ovariectomized or sham ovariectomy surgery at approximately 18 weeks of age.

*Dose Administration*

All rats starting approximately 6-weeks post surgery were acclimated to the experimental procedures by daily handling and restraint to simulate microneedle array application. Acclimation was continued for 4 weeks before a baseline assessment of BMD by DEXA and randomization into treatment groups based on femur BMD. Daily dose administration of either [Glu$^{22,25}$, Leu$^{23,28,31}$, Aib$^{29}$, Lys$^{26,30}$]hPTHrP(1-34)NH$_2$-PC-microneedle arrays, or [Glu$^{22,25}$, Leu$^{23,28,31}$, Aib$^{29}$, Lys$^{26,30}$]hPTHrP(1-34)NH$_2$-LCP-microneedle arrays or placebo microneedle arrays, or [Glu$^{22,25}$, Leu$^{23,28,31}$, Aib$^{29}$, Lys$^{26,30}$]hPTHrP(1-34)NH$_2$ by subcutaneous injection or placebo subcutaneous injection as outlined in Table 25. Microarrays were left in contact with the skin for one minute before being removed. Dosing was performed for 14 days, and then all rats were euthanized for sample collection.

*Serum Sample Collection*

On day 14 of dosing approximately 3 mL of blood was collected 15 minutes post dose. The blood was immediately transferred to a serum separator tube that contained 60 $\mu$L of a 2.5 mg/mL aprotinin (Sigma) solution. The blood was kept at room temperature for approximately 45 minutes to allow it to clot. Once clotted, the blood was centrifuged at 2500 rpm for 10 minutes. Serum was stored at -80°C. Prior to quantification [Glu$^{22,25}$, Leu$^{23,28,31}$, Aib$^{29}$, Lys$^{26,30}$]hPTHrP(1-34)NH$_2$ by radioimmunoassay.

*Bone Mineral Density (BMD)*

On the first day of test article dosing and on the day of sacrifice, BMD for all animals was assessed by DEXA (PIXImus, Lunar Corp/GE). The images were analyzed using the provided software to determine the BMD of the L3-L5 region of the spine and the left femur. The baseline and end of study scans were used to calculate the percent change in BMD after 14-days of treatment.

*Micro-Computed Tomography*

At necropsy the left femur and the L4-L5 vertebrae were removed and dissected of free soft tissue. The bones were stored in 70% ethanol at -80C. Prior to microCT analysis the femurs were cut through the midshaft and loaded into the sample analysis tube. Additionally, one vertebra from each rat was wrapped in ethanol soaked gauze and stacked in the sample tube for scanning. Qualitative 3D evaluation was preformed using the Scanco mCT40 system (Scanco, CH). For analysis of femur trabecular bone 250 slices of the distal femur metaphysis were scanned. 150 of these slices were contoured for evaluation. Analysis was started at the first slice where the right and left condyles were no loner visible. This ensured that there is no contribution from cortical bone or growth plate. Analysis continued towards the midshaft of the bone. For analysis of lumbar spine trabecular bone sections were analyzed starting at the first slice where the growth plate was no longer visible and continued until the growth plate appeared on the other side of the vertebra. Trabecular parameters analyzed included bone volume density (BV/TV), connectivity density (ConnD.), trabecular number (Tb.N), trabecular thickness (Tb.Th), trabecular spacing (Tb.Sp), and apparent bone density (ABD).

**Results**

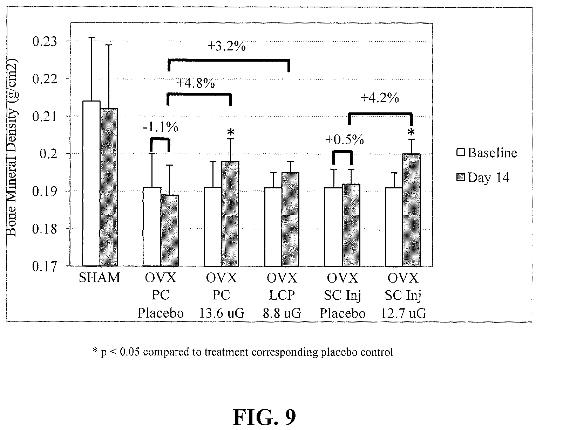

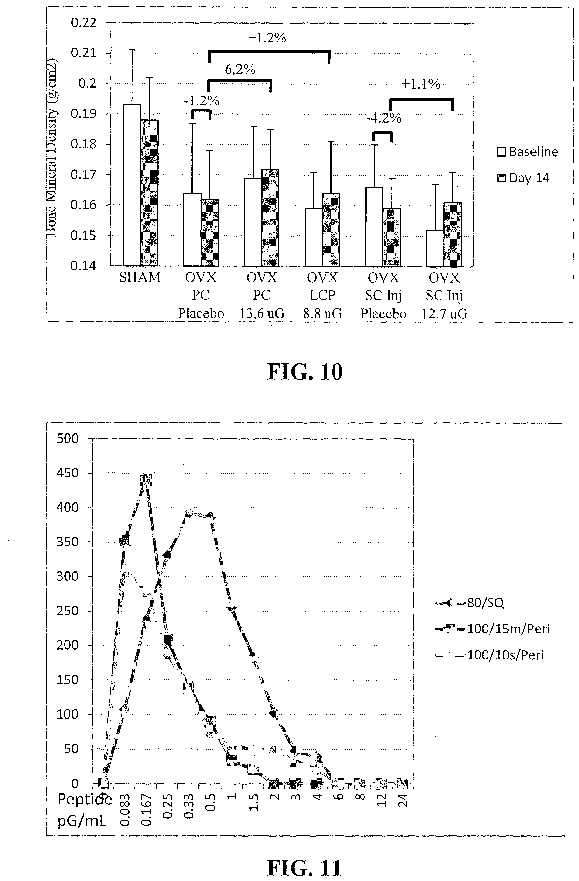

Ovariectomy of female rats resulted in an approximately 10% decrease in whole femur BMD at baseline, relative to sham surgery controls, and approximately a 15% decrease in lumbar spine BMD, confirming the effect of ovariectomy to induce osteopenia in rats (Figure 9). Repeat daily application of [Glu$^{22,25}$, Leu$^{23,28,31}$, Aib$^{29}$, Lys$^{26,30}$]hPTHrP(1-34)NH$_2$ PC microneedle arrays or [Glu$^{22,25}$, Leu$^{23,28,31}$, Aib$^{29}$, Lys$^{26,30}$]hPTHrP(1-34)NH$_2$ LCP microneedle arrays resulted in a marked increase in whole femur BMD (Figure 9) and lumbar spine BMD (Figure 10) after 14 days, compared to the corresponding placebo microneedle array control. Similar increase in femur and lumbar spine BMD was observed with [Glu$^{22,25}$, Leu$^{23,28,31}$, Aib$^{29}$, Lys$^{26,30}$] hPTHrP(1-34)NH$_2$ subcutaneous injections (Figures 9 and 10). The rapid recovery in bone mineral density clearly indicate the utility of the arrays containing [Glu$^{22,25}$, Leu$^{23,28,31}$, Aib$^{29}$, Lys$^{26,30}$] hP-THrP(1-34)NH$_2$ for the prevention and treatment of disorders relating to decreased bone mineral density such as osteoporosis and due to the particular rapidity of the effect and the anabolic nature of the product, the healing of bone fractures and/or breaks.

Trabecular bone microstructure parameters evaluated by microCT, including BV/TV, Tb.N and Tb.Th are decreased, while Tb. Sp is increased at baseline in the femoral metaphysis of OVX rats compared to Sham controls (Table 26). Similar changes in baseline OVX rats are observed in bone microstructure parameters in the lumber spine (Table 27). Repeat daily application for 14 days of [Glu$^{22,25}$, Leu$^{23,28,31}$, Aib$^{29}$, Lys$^{26,30}$] hPTHrP(1-34)NH$_2$ PC or [Glu$^{22,25}$, Leu$^{23,28,31}$, Aib$^{29}$, Lys$^{26,30}$] hPTHrP(1-34)NH$_2$ LCP microneedle arrays partially reversed these changes with increases in BV/TV, TB. N and Tb Th, while Tb. Sp was decreased in both the femoral metaphysis and lumbar spine (Tables 26 and 27). The magnitude of changes on these bone microstructure parameters was similar to those observed with repeat daily administration of [Glu$^{22,25}$, Leu$^{23,28,31}$, Aib$^{29}$, Lys$^{26,30}$] hPTHrP(1-34)NH$_2$ by subcutaneous injection (Tables 26 and 27). Additionally bone density measured by microCT was also increased following application of [Glu$^{22,25}$, Leu$^{23,28,31}$, Aib$^{29}$, Lys$^{26,30}$] hPTHrP(1-34)NH$_2$ PC or [Glu$^{22,25}$, Leu$^{23,28,31}$, Aib$^{29}$, Lys$^{26,30}$] hPTHrP(1-34)NH$_2$ LCP microneedle arrays in bone the femoral metaphysis and lumbar spine (Tables 26 and 27).

Serum concentration of [Glu$^{22,25}$, Leu$^{23,28,31}$, Aib$^{29}$, Lys$^{26,30}$] hPTHrP(1-34)NH$_2$ was measured 15 minutes post dose and for rats treated with [Glu$^{22,25}$, Leu$^{23,28,31}$, Aib$^{29}$, Lys$^{26,30}$] hPTHrP(1-34)NH$_2$ PC microneedle arrays the serum concentration was 17.2 $\pm$ 5.9 pg/ml, for rats treated with [Glu$^{22,25}$, Leu$^{23,28,31}$, Aib$^{29}$, Lys$^{26,30}$] hPTHrP(1-34)NH$_2$ LCP microneedle arrays the serum concentration was 14.0 $\pm$ 9.2 pg/ml and for [Glu$^{22,25}$, Leu$^{23,28,31}$, Aib$^{29}$, Lys$^{26,30}$] hPTHrP(1-34)NH$_2$ subcutaneous injection was 10.8 $\pm$ 3.6 pg/ml.

**Table 26: Change in Trabecular Bone Microstructure in the Osteopenic Rat Distal Femur Metaphysis Following Repeat Application of [Glu$^{22,25}$, Leu$^{23,28,31}$, Aib$^{29}$, Lys$^{26,30}$]hPTHrP(1-34)NH$_2$-Microneedle Arrays**

| | SHAM | OVX PC | OVX PC | OVX LCP | OVX SC | OVX SC |
|---|---|---|---|---|---|---|
| Parameters | - | Placebo | 13.6 $\mu$g | 8.8 $\mu$g | Placebo | 12.7 $\mu$g |
| BV/TV (ratio) | 0.554 $\pm$ 0.14 | 0.172 $\pm$ 0.04 | 0.227 $\pm$ 0.06* | 0.240 $\pm$ 0.05* | 0.203 $\pm$ 0.04 | 0.253 $\pm$ 0.05* |
| Tb.Th (mm) | 0.130 $\pm$ 0.03 | 0.089 $\pm$ 0.01 | 0.101 $\pm$ 0.01* | 0.104 $\pm$ 0.01* | 0.095 $\pm$ 0.01 | 0.108 $\pm$ 0.01* |
| Tb.N (#/mm) | 5.51 $\pm$ 0.85 | 1.72 $\pm$ 0.30 | 2.21 $\pm$ 0.70 | 2.14 $\pm$ 0.37* | 2.13 $\pm$ 0.60 | 2.33 $\pm$ 0.53 |
| Tb.Sp (mm) | 0.156 $\pm$ 0.04 | 0.632 $\pm$ 0.12 | 0.513 $\pm$ 0.16 | 0.507 $\pm$ 0.12 | 0.532 $\pm$ 0.16 | 0.471 $\pm$ 0.11 |
| Conn.D (#/mm$^3$) | 130 $\pm$ 23 | 126 $\pm$ 9 | 122 $\pm$ 6 | 124 $\pm$ 15 | 122 $\pm$ 11 | 122 $\pm$ 8 |
| ABD (mgHA/mm$^2$) | 466 $\pm$ 112 | 152 $\pm$ 39 | 202 $\pm$ 54* | 215 $\pm$ 46* | 179 $\pm$ 31 | 222 $\pm$ 44* |

* $p < 0.05$ compared to treatment corresponding placebo control

**Table 27: Change in Trabecular Bone Microstructure in the Osteopenic Rat Lumbar Spine Following Repeat Application of [Glu$^{22,25}$, Leu$^{23,28,31}$, Aib$^{29}$, Lys$^{21,31}$]hPTHrP(1-34)NH$_2$-Microneedle Arrays**

| | SHAM | OVX PC | OVX PC | OVX LCP | OVX SC | OVX SC |
|---|---|---|---|---|---|---|
| Parameters | - | Placebo | 13.6 µg | 8.8 µg | Placebo | 12.7 µg |
| BV/TV (ratio) | 0.604 ± 0.07 | 0.472 ± 0.07 | 0.520 ± 0.04 | 0.500 ± 0.03 | 0.470 ± 0.04 | 0.520 ± 0.05* |
| Tb.Th (mm) | 0.134 ± 0.02 | 0.119 ± 0.01 | 0.131 ± 0.01* | 0.125 ± 0.01 | 0.117 ± 0.01 | 0.132 ± 0.01* |
| Tb.N (#/mm) | 4.86 ± 0.39 | 3.94 ± 0.39 | 4.00 ± 0.38 | 4.00 ± 0.37 | 4.00 ± 0.28 | 4.02 ± 0.39 |
| Tb.Sp (mm) | 0.186 ± 0.02 | 0.231 ± 0.03 | 0.223 ± 0.02 | 0.225 ± 0.02 | 0.227 ± 0.02 | 0.224 ± 0.03 |
| Conn.D | 44 ± 12 | 49 ± 7 | 44 ± 10 | 48 ± 6 | 48 ± 9 | 46 ± 8 |
| (#/mm$^3$) ABD (mgHA/mm$^2$) | 507 ± 57 | 391 ± 53 | 451 ± 28* | 432 ± 14 | 400 ± 30 | 451 ± 43* |

* $p < 0.05$ compared to treatment corresponding placebo control

**Clinical study evaluation of pharmacokinetics of [Glu$^{22,25}$, Leu$^{23,28,31}$, Aib$^{29}$, Lys$^{26,30}$] hPTHrP(1-34)NH$_2$ polycarbonate-coated microarrays in postmenopausal women**

**Study Design**

**Table 28: Polycarbonate arrays used in clinical study**

| Arrays | Polycarbonate arrays |
|---|---|
| Material of Construction | Polycarbonate |
| Number of Microneedles | 366 |
| Flexural Modulus (by ISO 178) | 2300 |
| Grade | Class VI, medical grade polymer |
| Surface area | 5.5 cm$^2$ or ~27 mm in diameter |
| Depth of Penetration (DOP) | 250+/-10µm |
| Height of Microneedles | 500µm |
| Spacing between Microneedles | 550µm apart (tip to tip) |

Arrays were prepared using aqueous formulations of 54 to 58 wt-% [Glu$^{22,25}$, Leu$^{23,28,31}$, Aib$^{29}$, Lys$^{26,30}$]PTHrP(1-34)NH$_2$ and phosphate buffered saline.

Array loading dosages tested
Array 1:      100 µg per array +/- 15 µg per array (90 µg per array mean)
Array 2:      150 µg per array +/- 22.5 µg per array (149 µg per array mean)
Array 3:      200 µg per array +/- 30 µg per array (211 µg per array mean)

**Study Design and Methodology:**

This is a randomized, double-blind, placebo-controlled, ascending single-dose safety, pharmacokinetic and tolerability study of [Glu$^{22,25}$, Leu$^{23,28,31}$, Aib$^{29}$, Lys$^{26,30}$]hPTHrP(1-34)NH$_2$ presented as a coated transdermal microarray in healthy postmenopausal women. Enrolled subjects will undergo up to 3 single dose exposures to [Glu$^{22,25}$, Leu$^{23,28,31}$, Aib$^{29}$, Lys$^{26,30}$]hPTHrP(1-34)NH$_2$ or [Glu$^{22,25}$, Leu$^{23,28,31}$, Aib$^{29}$, Lys$^{26,30}$]hPTHrP(1-34)NH$_2$-sMTS-Placebo or [Glu$^{22,25}$, Leu$^{23,28,31}$, Aib$^{29}$, Lys$^{26,30}$]hPTHrP(1-34)NH$_2$ 80 µg subcutaneous (sc) injection over the course of the study.
Three study Periods and 13 study Groups are planned, with subjects being randomized prior to each dosing.

In the first study Period, 4 wear time variable Groups will be completed, as will 6 subjects who will receive 80 $\mu$g [Glu$^{22,25}$, Leu$^{23,28,31}$, Aib$^{29}$, Lys$^{26,30}$]hPTHrP(1-34)NH$_2$ for injection administered subcutaneously. In study Periods 2 and 3 there will be three Groups receiving an escalating dose. Within the first study Group, 32 subjects will be randomized into one of four sub-Groups of varying wear time for the transdermal microarray. The potential wear times of the TD microarray are 5, 15, 30, and 60 minutes while the concentration of the [Glu$^{22,25}$, Leu$^{23,28,31}$, Aib$^{29}$, Lys$^{26,30}$]hPTHrP(1-34)NH$_2$ -microneedle array will remain constant at 100 $\mu$g. Within each of the 5 subgroups (Study Period 1), 6 subjects will receive [Glu$^{22,25}$, Leu$^{23,28,31}$, Aib$^{29}$, Lys$^{26,30}$]hPTHrP(1-34)NH$_2$-microneedle array via a transdermal microarray and two subjects will receive a corresponding [Glu$^{22,25}$, Leu$^{23,28,31}$, Aib$^{29}$, Lys$^{26,30}$]hPTHrP(1-34)NH$_2$ -microneedle array-Placebo, and six subjects will receive [Glu$^{22,25}$, Leu$^{23,28,31}$, Aib$^{29}$, Lys$^{26,30}$]hPTHrP(1-34)NH$_2$ 80 $\mu$g for injection, administered subcutaneously. Prior to proceeding to the next dose safety, tolerability, and pharmacokinetic data from subjects enrolled in earlier Groups will be reviewed for suitability to escalate to the next higher dose. In study Group 2 which will enroll 24 subjects, 18 will be randomly assigned to receive [Glu$^{22,25}$, Leu$^{23,28,31}$, Aib$^{29}$, Lys$^{21,31}$]hPTHrP(1-34)NH$_2$ -microneedle array via transdermal microarray, 4 will receive a corresponding [Glu$^{22,25}$, Leu$^{23,28,31}$, Aib$^{29}$, Lys$^{26,30}$]hPTHrP(1-34)NH$_2$ -microneedle array-Placebo, and 2 will receive [Glu$^{22,25}$, Leu$^{23,28,31}$, Aib$^{29}$, Lys$^{26,30}$]hPTHrP(1-34)NH$_2$ administered as a SC injection of [Glu$^{22,25}$, Leu$^{23,28,31}$, Aib$^{29}$, Lys$^{26,30}$]hPTHrP(1-34)NH$_2$ 80 $\mu$g for injection administered subcutaneously. In Group 3 which will enroll 16 subjects, 2 Groups of 6 will be randomly assigned to receive [Glu$^{22,25}$, Leu$^{23,28,31}$, Aib$^{29}$, Lys$^{26,30}$]hPTHrP(1-34)NH$_2$ -microneedle array applied to the periumbilical region or upper outer arm, while 2 will receive a corresponding [Glu$^{22,25}$, Leu$^{23,28,31}$, Aib$^{29}$, Lys$^{26,30}$]hPTHrP(1-34)NH$_2$-microneedle array-placebo, one at each of these sites. In addition, 2 additional subjects will receive a standard SC administration of [Glu$^{22,25}$, Leu$^{23,28,31}$, Aib$^{29}$, Lys$^{26,30}$]hPTHrP(1-34)NH$_2$ 80 $\mu$g for injection administered subcutaneously.

If the bioavailability of the 100 $\mu$g [Glu$^{22,25}$, Leu$^{23,28,31}$, Aib$^{29}$, Lys$^{26,30}$]hPTHrP(1-34)NH$_2$ -microneedle array is greater than 50%, the 200 $\mu$g dose will not be administered; if greater than 66%, the 150 $\mu$g dose will not be administered.

The doses and the number of subjects that are planned for enrollment per Period and Group are shown in Table 29.

**Table 29: Schedule of dosages, sites and wear time**

| | | | | | Number of Subjects Randomized | | | |
|---|---|---|---|---|---|---|---|---|
| Period 1 | | | | | Microarray | | Subcutaneous | |
| Study Group | Frequency of Dosing | Dose | Application or Injection Site | Wear Time | Peptide-sMTS | Microneedle Array-Placebo | 80 μg Peptide for injection | Total # |
| 1a | Once | 100 μg | Periumbilical | 5 min | 6 | 2 | N/A | 8 |
| 1b | Once | 100 μg | Periumbilical | 15 min | 6 | 2 | N/A | 8 |
| 1c | Once | 100 μg | Periumbilical | 30 min | 6 | 2 | N/A | 8 |
| 1d | Once | 100 μg | Periumbilical | 60 min | 6 | 2 | N/A | 8 |
| 1e | Once | 80 μg | Periumbilical | N/A | N/A | N/A | 6 | 6 |
| | | Total: | | | 24 | 8 | 6 | 38 |
| Period 2 | | | | | Microarray | | Subcutaneous | |
| Study Group | Frequency of Dosing | Dose | Application Site | Wear Time[1] | Peptide-microneed le array | Peptide-microneedle array-Placebo | 80 μg Peptide for injection | Total # |
| 2a | Once | 150 μg | Periumbilical | TBD | 6 | 1 | N/A | 7 |
| 2b | Once | 150 μg | Upper Anterior Thigh | TBD | 6 | 1 | N/A | 7 |
| 2c | Once | 100 μg | Periumbilical | 24 Hours | 6 | 2 | N/A | 8 |
| 2d | Once | 80 μg | Periumbilical | N/A | N/A | N/A | 2 | 2 |
| | | Total: | | | 18 | 4 | 2 | 24 |
| Period 3 | | | | | Microarray | | Subcutaneous | |
| Study Group | Frequency of Dosing | Dose | Application Site | Wear Time | Peptide-microneed le array | Peptide-micro needle array-Placebo | 80 μg Peptide for injection | Total # |
| 3a | Once | 200 μg | Periumbilical | TBD | 6 | 1 | N/A | 7 |

(continued)

| Period 3 | | | | | Microarray | | Subcutaneous | |
|---|---|---|---|---|---|---|---|---|
| Study Group | Frequency of Dosing | Dose | Application Site | Wear Time | Peptide-microneed le array | Peptide-micro needle array-Placebo | 80 $\mu$g Peptide for injection | Total # |
| 3b | Once | 200 $\mu$g | Upper Outer Arm (deltoid) | TBD | 6 | 1 | N/A | 7 |
| 3c | Once | 80 $\mu$g | Periumbilical | N/A | N/A | N/A | 2 | 2 |
| | | Total: | | | 12 | 2 | 2 | 16 |
| [1] The wear times in Study Periods 2 and 3 will be based upon the results obtained in Study Period 1 | | | | | | | | |

Standard safety assessments are included to ensure the safety of subjects. These safety evaluations include physical examinations, vital signs, 12-lead digital ECGs, clinical laboratory tests, and monitoring and recording of local tolerance and adverse events.

For pharmacokinetic (PK) analysis, a total of 15 venous blood samples across 24 hours will be taken to measure [Glu$^{22,25}$, Leu$^{23,28,31}$, Aib$^{29}$, Lys$^{26,30}$]hPTHrP(1-34)NH$_2$ plasma concentrations at the following times: pre-dose and 5, 10, 15, 20, 30, 60 minutes, 1.5, 2, 3, 4, 6, 8, and 12 hours post-dose. A final sample will be taken 24 hours after the last dose of study medication.

**Number of subjects:** A sufficient number of eligible subjects will be enrolled to achieve 38 subjects who complete treatment and study procedures.

**Treatments Administered:** [Glu$^{22,25}$, Leu$^{23,28,31}$, Aib$^{29}$, Lys$^{26,30}$]hPTHrP(1-34)NH$_2$-sMTS (100 $\mu$g, 150 $\mu$g and 200 $\mu$g) will be supplied as a coated, transdermal array attached to a self-adhesive patch for use with an applicator.

[Glu$^{22,25}$, Leu$^{23,28,31}$, Aib$^{29}$, Lys$^{26,30}$]hPTHrP(1-34)NH$_2$-microneedle array-Placebo will be similarly supplied in a coated, transdermal array attached to a self-adhesive patch for use with a spring-loaded applicator.

[Glu$^{22,25}$, Leu$^{23,28,31}$, Aib$^{29}$, Lys$^{26,30}$]hPTHrP(1-34)NH$_2$ Drug Product for Injection 80 $\mu$g is supplied as a multi-dose cartridge (1.5 mL) containing 2 mg/mL [Glu$^{22,25}$, Leu$^{23,28,31}$, Aib$^{29}$, Lys$^{26,30}$]hPTHrP(1-34)NH$_2$ (free base) in 5 mg/mL tri-hydrate sodium acetate and 5 mg/mL of phenol (preservative) adjusted at pH 5.1 with acetic acid.

The pen injector is a modified version of the Becton Dickinson Pen II device and has been validated for use with [Glu$^{22,25}$, Leu$^{23,28,31}$, Aib$^{29}$, Lys$^{26,30}$]hPTHrP(1-34)NH$_2$ in its prefilled cartridge.

**Data Analysis:**

Pharmacokinetic analysis:

Individual plasma concentrations of [Glu$^{22,25}$, Leu$^{23,28,31}$, Aib$^{29}$, Lys$^{26,30}$]hPTHrP(1-34)NH$_2$ will be tabulated separately for each dose Group and sampling time and summarized descriptively. Individual and summary profiles will also be plotted for each dose. The plasma concentration-time profiles of [Glu$^{22,25}$, Leu$^{23,28,31}$, Aib$^{29}$, Lys$^{26,30}$]hP-THrP(1-34)NH$_2$ will be analyzed using non-compartmental methods. For each dose level, relative bioavailability will be calculated as the ratio of dose normalized AUCinf values:

$$[AUCinf(transdermal)/Dose(transdermal)]/[AUCinf(SC)/Dose(SC)].$$

**Selection of Study Population**

**Number of Subjects**

A sufficient number of eligible subjects will be enrolled to achieve 38 subjects who complete treatment and study procedures.

**Inclusion Criteria**

Subjects must meet all of the following inclusion criteria to be eligible to participate in this study.

The subject is a healthy postmenopausal woman from 50 to 80 years of age, inclusive. For the purposes of this study, postmenopausal is defined as $\geq$ 24 months of spontaneous amenorrhea (not relating to eating disorders or other causes) or $\geq$ 6 months of spontaneous amenorrhea with serum follicle-stimulating hormone (FSH) levels $\geq$ 40 mIU/mL or 6 weeks postsurgical bilateral oophorectomy with or without hysterectomy.

The subject is in good general health as determined by medical history and physical examination (including vital signs) and without evidence of clinically significant abnormality, in the opinion of the Investigator.

The subject has a hemoglobin value greater than 12.0 g/dL during the screening Period.

The subject has a serum phosphorus, PTH(1-84) and a serum total calcium within the normal range during the screening Period.

The subject has a normal serum alkaline phosphatase during the screening visit or, if abnormal but not clinically significant, a normal serum bone-specific alkaline phosphatase The subject has a 25-hydroxyvitamin D of $\geq$ 9 ng/mL.

The subject has all other screening and baseline clinical laboratory tests without any clinically significant abnormality, in the opinion of the Investigator.

The resting 12-lead electrocardiogram obtained during screening shows no clinically significant abnormality of the following intervals: PR: $\geq$ 120 and $\leq$ 220 ms; QRS $\leq$ 120 ms: QTc (Bazett's correction) $\leq$ 470 ms. Incomplete right bundle branch block (IRBBB) and left anterior hemiblock (LAH) are acceptable.

The subject's systolic blood pressure is $\geq$ 100 and $\leq$ 155 mmHg, diastolic blood pressure is $\geq$ 40 and $\leq$ 95 mmHg, and heart rate is $\geq$ 45 and $\leq$ 90 bpm during screening.

The subject weighs at least 120 pounds (54.5 kg) and is within -25% and +30% of her ideal body weight (at screening) based on height and body frame according to the Metropolitan Life Insurance Company table.

The subject has read, understood, and signed the written informed consent form.

**Exclusion Criteria**

Subjects who meet any of the following exclusion criteria will not be eligible to participate in the study.

*General exclusion criteria:*

The subject has a history of clinically significant chronic or recurrent renal, hepatic, pulmonary, allergic, cardiovascular, gastrointestinal, endocrine, central nervous system, hematologic or metabolic diseases, or immunologic, emotional and/or psychiatric disturbances.

The subject has been diagnosed with osteoporosis, Paget's disease, or other metabolic bone diseases (e.g., vitamin D deficiency or osteomalacia) or has had a non-traumatic fracture that occurred within one year prior to the initial screening visit.

The subject has a history of urolithiasis within the past five years.

The subject has a history of gout or a uric acid value > 7.5 mg/dL during the Screening Period.

The patient has a decrease of 20 mmHg or more in systolic blood pressure or 10 mmHg or more in diastolic blood pressure from supine to standing (5 minutes lying and 3 minutes standing) and/or any symptomatic hypotension.

The subject has an acute illness which, in the opinion of the Investigator, could pose a threat or harm to the subject or obscure laboratory test results or interpretation of study data.

The subject has donated blood, or has had a blood loss of more than 50 mL within 8 weeks prior to study Day 1, or has had a plasma donation (apheresis) within 7 days prior to Day 1.

The subject is known to be positive for Hepatitis B, Hepatitis C, human immunodeficiency virus (HIV)-1 or HIV-2 or have positive results at screening for Hepatitis B surface antigen (HBsAg), Hepatitis C antibody (HCV-Ab), or HIV.

The subject has been previously randomized, dosed and discontinued in this study for any reason.

*Medication related exclusion criteria:*

The subject has a known history of hypersensitivity to any of the test materials or related compounds.

The subject uses any medication on a chronic basis, including bisphosphonates and estrogens or estrogen derivatives, with the exception of certain medications.

The subject received any medication, including over-the-counter, non-prescription preparations or herbal or homeopathic supplements, with the exception of certain medicines, within 72 hours prior to administration of the first dose of study medication.

The subject received a general anesthetic or an investigational other than [Glu$^{22,25}$, Leu$^{23,28,31}$, Aib$^{29}$, Lys$^{26,30}$]hPTHrP(1-34)NH$_2$ within 90 days prior to the initial dose of study medication.

Unwillingness or inability to understand study procedures or commitments as judged by the Medical Investigator.

*Lifestyle related exclusion criteria:*

The subject has an abnormal nutritional status (abnormal diets, excessive or unusual vitamin intakes, malabsorption, significant recent weight change).

The subject smokes more than 10 cigarettes per day. Subjects will not be allowed to consume any nicotine-containing products while they are confined to the clinical facility.

The subject has a history of alcohol abuse, illegal drug use or drug abuse within 24 months of the screening visit.

The subject has a positive urine drug/alcohol screen.

**Withdrawal of Subjects**

Subjects will be informed that they have the right to withdraw from the study at any time for any reason, without prejudice to their medical care. The Investigator also has the right to withdraw subjects from the study for any of the following reasons:

- Adverse events.

- Refusal of treatment.
- Subject request.
- Inability to complete study procedures.
- Lost to follow-up.
- Non-compliance.

If a subject is withdrawn or discontinued from the study, the reason for withdrawal from the study is to be recorded in the source documents and on the case report form. All subjects withdrawn prior to completing the study should be encouraged to complete postdose study evaluation scheduled for the Study Group. Subjects who withdraw from the study for administrative reasons after study medication has been administered may be replaced at the discretion of the Investigator after consultation with the Medical Monitor.

**Replacement of Subjects**

If there are insufficient subjects to achieve enrollment of 38, 24, and 16 subjects per dose Group respectively in Groups 1, 2, and 3, additional subjects may be recruited.

[Glu$^{22,25}$, Leu$^{23,28,31}$, Aib$^{29}$, Lys$^{26,30}$]hPTHrP(1-34)NH$_2$-microneedle array and [Glu$^{22,25}$, Leu$^{23,28,31}$, Aib$^{29}$, Lys$^{26,30}$]hPTHrP(1-34)NH$_2$-microneedle array-Placebo will be supplied. Transdermal microarrays, cartridges and needles for administration of study medications will also be supplied to the study site. Study drug will be prepared for individual patients by the pharmacist.

[Glu$^{22,25}$, Leu$^{23,28,31}$, Aib$^{29}$, Lys$^{26,30}$]hPTHrP(1-34)NH$_2$ has been formulated with phosphate buffered saline (PBS) alone to deliver either 100, 150, or 200 $\mu$g of [Glu$^{22,25}$, Leu$^{23,28,31}$, Aib$^{29}$, Lys$^{26,30}$]PTHrP(1-34)NH$_2$ per array for transdermal administration using a microneedle array. The microneedle array is a 366 microneedle (500$\mu$m tall) array designed to be drug coated and applied directly to the skin to achieve systemic delivery. The array patch has an overall surface area of 5.5 cm$^2$ or ~27 mm in diameter.

The [Glu$^{22,25}$, Leu$^{23,28,31}$, Aib$^{29}$, Lys$^{26,30}$]hPTHrP(1-34)NH$_2$-coated microneedle array ([Glu$^{22,25}$, Leu$^{23,28,31}$, Aib$^{29}$, Lys$^{26,30}$]hPTHrP(1-34)NH$_2$ - microneedle array) will be enclosed in a collar assembly for loading onto a spring loaded applicator. The [Glu$^{22,25}$, Leu$^{23,28,31}$, Aib$^{29}$, Lys$^{26,30}$]hPTHrP(1-34)NH$_2$ - microneedle array will be removed from refrigeration one hour prior to application. Then, the [Glu$^{22,25}$, Leu$^{23,28,31}$, Aib$^{29}$, Lys$^{26,30}$]hPTHrP(1-34)NH$_2$ microneedle array will be loaded onto the applicator by the pharmacist or study personnel for subject dosing. Each [Glu$^{22,25}$, Leu$^{23,28,31}$, Aib$^{29}$, Lys$^{26,30}$]hPTHrP(1-34)NH$_2$ microneedle array is coated with either 100 $\mu$g, 150 $\mu$g or 200 $\mu$g of [Glu$^{22,25}$, Leu$^{23,28,31}$, Aib$^{29}$, Lys$^{26,30}$]hPTHrP(1-34)NH$_2$.

[Glu$^{22,25}$, Leu$^{23,28,31}$, Aib$^{29}$, Lys$^{26,30}$]hPTHrP(1-34)NH$_2$-microneedle array-Placebo: Phosphate Buffered Saline (PBS) has been formulated as a placebo for transdermal administration using a microneedle array. The PBS-coated microneedle array (Placebo microneedle array) will be enclosed in a collar assembly for loading onto a spring loaded applicator. The Placebo microneedle array will be removed from refrigeration one hour prior to application. Then the Placebo microneedle array will be loaded onto the applicator by the pharmacist or study personnel for subject dosing.

**Study Medication Administration**

[Glu$^{22,25}$, Leu$^{23,28,31}$, Aib$^{29}$, Lys$^{26,30}$]hPTHrP(1-34)NH$_2$-microneedle array and [Glu$^{22,25}$, Leu$^{23,28,31}$, Aib$^{29}$, Lys$^{26,30}$]hPTHrP(1-34)NH$_2$-microneedle array-Placebo will be administered in a double-blinded fashion. Subjects will fast overnight for a minimum of 8 hours prior to receiving study medication.

In Group 1 at the appropriate time, each subject will be given study medication via a single application of the transdermal microarray or single subcutaneous injection into the periumbilical region by study personnel. Subjects participating in Group 1 will be randomized to receive [Glu$^{22,25}$, Leu$^{23,28,31}$, Aib$^{29}$, Lys$^{26,30}$]hPTHrP(1-34)NH$_2$-microneedle array or [Glu$^{22,25}$, Leu$^{23,28,31}$, Aib$^{29}$, Lys$^{26,30}$]hPTHrP(1-34)NH$_2$-microneedle array-Placebo administered transdermally or [Glu$^{22,25}$, Leu$^{23,28,31}$, Aib$^{29}$, Lys$^{26,30}$]hPTHrP(1-34)NH$_2$ 80 $\mu$g administered subcutaneously. The subjects randomized to the [Glu$^{22,25}$, Leu$^{23,28,31}$, Aib$^{29}$, Lys$^{26,31}$]hPTHrP(1-34)NH$_2$-microneedle array or [Glu$^{22,25}$, Leu$^{23,28,31}$, Aib$^{29}$, Lys$^{26,30}$]hPTHrP(1-34)NH$_2$-microneedle array-Placebo transdermal application will be assigned to one of 4 wear times (5, 15, 30, and 60 minutes, 6 active treatment and 2 placebo in each Group). Six subjects will also be randomized to [Glu$^{22,25}$, Leu$^{23,28,31}$, Aib$^{29}$, Lys$^{21,30}$]hPTHrP(1-34)NH$_2$ for injection 80 $\mu$g administered subcutaneously.

Subjects in Group 2a will be randomized to receive either [Glu$^{22,25}$, Leu$^{23,28,31}$, Aib$^{29}$, Lys$^{26,30}$]hPTHrP(1-34)NH$_2$-microneedle array delivered transdermally or [Glu$^{22,25}$, Leu$^{23,28,31}$, Aib$^{29}$, Lys$^{26,30}$]hPTHrP(1-34)NH$_2$ for injection 80 $\mu$g administered subcutaneously. If the bioavailability of the 100 $\mu$g [Glu$^{22,25}$, Leu$^{23,28,31}$, Aib$^{29}$, Lys$^{26,30}$]hPTHrP(1-34)NH$_2$-microneedle array is greater than 66%, the 150 $\mu$g dose will not be administered. Those randomized to the transdermal application will receive either [Glu$^{22,25}$, Leu$^{23,28,31}$, Aib$^{29}$, Lys$^{26,30}$]hP-

THrP(1-34)NH$_2$-microneedle array 150 $\mu$g or [Glu$^{22,25}$, Leu$^{23,28,31}$, Aib$^{29}$, Lys$^{26,30}$]hPTHrP(1-34)NH$_2$-microneedle array-Placebo in one of two anatomical locations. Six subjects will be randomized to wear the microarray in the periumbilical region (Group 2a), and 6 subjects will be randomized to receive the microarray on the upper anterior thigh (Group 2b). One placebo patient will be randomized to each of the anatomical sites, for a total of 12 active, 2 placebo subjects in Group 2a. Eight further subjects will be randomized to Group 2c, and of these subjects, six will receive either [Glu$^{22,25}$, Leu$^{23,28,31}$, Aib$^{29}$, Lys$^{26,30}$]hPTHrP(1-34)NH$_2$-microneedle array or [Glu$^{22,25}$, Leu$^{23,28,31}$, Aib$^{29}$, Lys$^{26,30}$]hP-THrP(1-34)NH$_2$-microneedle array-Placebo at a dose of 100 $\mu$g via transdermal delivery for 24 hours applied to the periumbilical region.

Prior to the administration of study drug, the application site should be examined in order to assure that the areas are not compromised. Each application site will be graded immediately upon removal of the transdermal device or post injection, at one hour and 24 hours after the microarray application or subcutaneous injection was performed. For patients in Group 2c, who were randomized to a wear time of 24 hours, the patient will need to return to the clinic for a final local tolerance assessment 24 hours after removal of the microarray. For any administration sites rated with a grade of 3, evaluations will continue at 24 hour intervals until the skin irritation has stabilized or resolved.

Before loading the transdermal microarray and collar onto the applicator, the microarray should be visibly inspected. If any of the microarrays or collars appear to be damaged that microarray should not be used and a new array should be chosen.

**Concomitant Medications**

Vitamin D ($\leq$ 800 IU/day), calcium supplements ($\leq$ 1000 mg/day), and low dose aspirin ($\leq$ 81 mg/daily for prophylaxis of cardiovascular disease) are acceptable as long as the subject has been on a stable dose for 1 month prior to the initial screening visit and remains on the same dose(s) throughout the study. Thyroid replacement therapy is allowed if the subject has been on a stable dose for at least 6 months and remains on the same dose throughout the study. Statins for lowering blood cholesterol levels are allowed as long as the subject has been on a stable dose for at least 3 months and remains on the same dose throughout the study.

Subjects should not take any other medications, including over-the-counter medications, herbal medications, or mega-doses of vitamins during the study without prior approval of the Investigator. The occasional use of over-the-counter medications (e.g., ibuprofen or acetaminophen) for headache or minor discomfort is allowed if discussed with the Investigator and recorded in the CRF.

If it becomes necessary for a subject to take any other medication during the study, the specific medication(s) and indication(s) must be discussed with the Investigator. All concomitant medications taken during the course of the study must be recorded in the source documents and transcribed into the subject's case report form.

**Prohibited Medications**

Subjects cannot take any medications, including over-the-counter, non-prescription medication, with the exception of those noted (Concomitant Medications), within 72 hours prior to dosing on Day 1.

In addition, subjects are ineligible for the study if they received general anesthesia within the past 3 months, received an investigational drug within 90 days prior to the initial dose of study medication, take any medications on a chronic basis (other than allowed in Section 6.1), or have an abnormal nutritional status (abnormal diets, excessive or unusual vitamin intakes, malabsorption).

**Blood Sample Collection**

A total of 15 venous blood samples will be taken to measure [Glu$^{22,25}$, Leu$^{23,28,31}$, Aib$^{29}$, Lys$^{26,30}$]hP-THrP(1-34)NH$_2$ plasma concentrations. PK blood samples should be collected as close to the exact time point as possible. Two 5 mL samples will be collected into vacutainer tubes and put into an ice water bath immediately after collection. Exact procedures for centrifuging, storage, and shipping of plasma samples will be detailed in a separate document. Plasma samples will be stored at -80°C before shipment to the bioanalytical laboratory. Venous blood samples will be taken as follows:

Days P1-D1, P2-D1 and P3-D1
Pre-dose and at 5, 10, 15, 20, 30, 60 min, 1.5, 2, 3, 4, 6, 8 and 12 hours post-dose.
Days P1-D2, P2-D2 and P3-D2
A single venous blood sample will be taken in the morning 24 hours after study medication administration.

The actual time of each blood collection will be recorded.

**Pharmacodynamic (PD) Assessments**

**Blood Sample Collection**

Venous blood samples will be collected for the determination of total calcium and phosphorous at the following time points:

Days P1-D1, P2-D1 and P3-D1
Pre-dose and at 0.5, 1, 2, 3, 4, 6, 8 and 12 hours post-dose.
Days P1-D2, P2-D2 and P3-D2
A single venous blood sample will be taken in the morning 24 hours after study medication administration.

Venous blood samples for determination of 1.25 hydroxyvitamin D at the following time points:

Days P1-D1, P2-D1 and P3-D1
Pre-dose and at 3 and 12 hours post-dose.
Days P1-D2, P2-D2 and P3-D2
A single venous blood sample will be taken in the morning 24 hours after study medication administration.

## PHARMACOKINETIC ANALYSIS

PK parameters will be derived using noncompartmental methods with WinNonlin™ Professional Version 5.01, or higher, (Pharsight Corp, Cary, North Carolina) and SAS™ Version 9.1, or higher (SAS Institute, Inc., Cary, North Carolina).

The following PK parameters will be estimated:

- The peak plasma concentration ($C_{max}$)

- The empirical time of $C_{max}$ ($T_{max}$) as well as the time of the last sample with quantifiable concentration of [Glu[22,25], Leu[23,28,31], Aib[29], Lys[26,30]]hPTHrP(1-34)NH$_2$ ($T_{last}$)

- The apparent elimination rate constant ($\lambda_z$), estimated by linear regression of the terminal phase of the semilogarithmic plasma level curve, when it is clearly defined

- The apparent elimination half-life ($t_{1/2z}$), determined as $\ln2/\lambda_z$

- The area under the plasma concentration time curve from time 0 to the last experimental point ($AUC_{0-t}$), estimated by the linear-log trapezoidal rule

- The area under the plasma concentration time curve from time 0 to $\infty$, ($AUC_{0-\infty}$) estimated by the linear-log trapezoidal rule. $AUC_{0-\infty}=AUC_{0-t}+C_t/\lambda_z$, where $C_t$ is the last measurable concentration

- The area under the plasma concentration time curve from time 0 to the 24 hr ($AUC_\tau$), estimated by the linear-log trapezoidal rule

- The extravascular plasma clearance (CL/F), calculated as: = Dose/ $AUC_{0-\infty}$

The extravascular volume of distribution (Vd/F), calculated as: = CL/F/$\lambda z$.
Moreover, for each dose level, relative bioavailability will be calculated as the ratio of dose normalized $AUC_{0-\infty}$ values: [$AUC_{0-\infty}$ (transdermal)/Dose(transdermal)]/[$AUC_{0-\infty}$ (SC)/Dose(SC)]

**Analytical Methods**

The quantification of [Glu[22,25], Leu[23,28,31], Aib[29], Lys[26,30]]hPTHrP(1-34)NH$_2$ in human plasma will be performed using a validated immunoassay method.

**Table 30: Results of study with [Glu$^{22,25}$, Leu$^{23,28,31}$, Aib$^{29}$, Lys$^{26,30}$]hPTHrP(1-34)NH$_2$ using PCS-transdermal system**

| Comparison | Pharmacokinetic Parameters | LS Means | | % Mean Ratio | P-Value |
|---|---|---|---|---|---|
| | | Test | Reference | | |
| Treatment 1A versus Peptide for Injection 80 µg | $C_{max}$ (pg/mL) | 298.19 | 504.01 | 59.16 | 0.0546 |
| | $AUC_{0-t}$ (pg*hr/mL) | 64.78 | 655.94 | 9.88 | <.0001 |
| | $AUC_{0-inf}$ (pg*hr/mL) | 88.54 | 699.06 | 12.67 | <.0001 |
| Treatment 1B versus Peptide for Injection 80 µg | $C_{max}$ (pg/mL) | 366.47 | 504.01 | 72.71 | 0.2346 |
| | $AUC_{0-t}$ (pg*hr/mL) | 112.14 | 655.94 | 17.10 | 0.0002 |
| | $AUC_{0-inf}$ (pg*hr/mL) | 122.48 | 699.06 | 17.52 | <.0001 |
| Treatment 1C versus Peptide for Injection 80 µg | $C_{max}$ (pg/mL) | 237.56 | 504.01 | 47.13 | 0.0074 |
| | $AUC_{0-t}$ (pg*hr/mL) | 62.04 | 655.94 | 9.46 | <.0001 |
| | $AUC_{0-inf}$ (pg*hr/mL) | 77.67 | 699.06 | 11.11 | <.0001 |
| Treatment 1D versus Peptide for Injection 80 µg | $C_{max}$ (pg/mL) | 292.78 | 504.01 | 58.09 | 0.0471 |
| | $AUC_{0-t}$ (pg*hr/mL) | 82.48 | 655.94 | 12.57 | <.0001 |
| | $AUC_{0-inf}$ (pg*hr/mL) | 127.57 | 699.06 | 18.25 | 0.0001 |
| Treatment 2C versus Peptide for Injection 80 µg | $C_{max}$ (pg/mL) | 226.31 | 504.01 | 44.90 | 0.0390 |
| | $AUC_{0-t}$ (pg*hr/mL) | 86.85 | 655.94 | 13.24 | 0.0017 |
| | $AUC_{0-inf}$ (pg*hr/mL) | 107.84 | 699.06 | 15.43 | 0.0013 |

Treatment 1A: 1 x 100 µg Peptide-array administered into the periumbilical region via a TD delivery system (TD microarray) with 5 minutes wear time (test)

Treatment 1B: 1 x 100 µg Peptide-array administered into the periumbilical region via a TD delivery system (TD microarray) with 15 minutes wear time (test)

Treatment 1C: 1 x 100 µg Peptide-array administered into the periumbilical region via a TD delivery system (TD microarray) with 30 minutes wear time (test)

Treatment 1D: 1 x 100 µg Peptide-array administered into the periumbilical region via a TD delivery system (TD microarray) with 60 minutes wear time (test)

Treatment 1E: 1 x 80 µg Peptide administered into the periumbilical region in a single SC injection (reference)

Treatment 2C: 1 x 100 µg Peptide-array administered into the periumbilical region via a TD delivery system (TD microarray) with 24 hours wear time (test)

Values for Treatments are the least-squares means (LS Means) from the ANOVA.

Parameters were ln-transformed prior to analysis.

LS Means are calculated by exponentiating the LS Means from the ANOVA.

% Mean Ratio = 100*(test/reference)

Data from all 10 subjects combined from the 3 periods were used for the SC dose (Treatment 1E).

Peak [Glu$^{22,25}$, Leu$^{23,28,31}$, Aib$^{29}$, Lys$^{26,30}$]hPTHrP(1-34)NH$_2$ exposure from [Glu$^{22,25}$, Leu$^{23,28,31}$, Aib$^{29}$, Lys$^{26,30}$]hPTHrP(1-34)NH$_2$-microneedle array 100 µg, as determined from $C_{max}$, ranged from 45% to 73% of the reference treatment ([Glu$^{22,25}$, Leu$^{23,28,31}$, Aib$^{29}$, Lys$^{26,30}$]hPTHrP(1-34)NH$_2$ for Injection 80 µg). Total exposure, as deter-

mined from $AUC_{0-\infty}$, was 11% to 18% of the reference treatment.

The differences in mean $C_{max}$, $AUC_{0-t}$, and $AUC_{0-\infty}$ values between the [Glu[22,25], Leu[23,28,31], Aib[29], Lys[26,30]]hP-THrP(1-34)NH$_2$-microneedle array treatments and the reference treatment were statistically significant (p-values < 0.05) in most cases.

**Table 31: Statistical Comparisons of Plasma [Glu[22,25], Leu[23,28,31], Aib[29], Lys[26,30]]hPTHrP(1-34) NH$_2$Pharmacokinetic Parameters Following [Glu[22,25], Leu[23,28,31], Aib[29], Lys[26,30]]hPTHrP(1-34)NH$_2$ Treatments (Period 2): Effect of Application Site**

| Comparison | Pharmacokinetic Parameters | LS Means | | % Mean Ratio | P-Value |
|---|---|---|---|---|---|
| | | Test | Reference | | |
| Treatment 2A versus Peptide for Injection 80 μg | $C_{max}$ (pg/mL) | 314.89 | 504.01 | 62.48 | 0.1034 |
| | $AUC_{0-t}$ (pg*hr/mL) | 106.91 | 655.94 | 16.30 | 0.0003 |
| | $AUC_{0-inf}$ (pg*hr/mL) | 118.72 | 699.06 | 16.98 | 0.0003 |
| Treatment 2B versus Treatment 2A | $C_{max}$ (pg/mL) | 311.97 | 314.89 | 99.07 | 0.9621 |
| | $AUC_{0-t}$ (pg*hr/mL) | 142.85 | 106.91 | 133.62 | 0.3246 |
| | $AUC_{0-inf}$ (pg*hr/mL) | 157.42 | 118.72 | 132.60 | 0.3202 |
| Treatment 2B versus Peptide for Injection 80 μg | $C_{max}$ (pg/mL) | 311.97 | 504.01 | 61.90 | 0.0972 |
| | $AUC_{0-t}$ (pg*hr/mL) | 142.85 | 655.94 | 21.78 | 0.0015 |
| | $AUC_{0-inf}$ (pg*hr/mL) | 157.42 | 699.06 | 22.52 | 0.0014 |
| Treatment 2A: 1 x 150 μg Peptide-microneedle array administered into the periumbilical region via a TD delivery system (TD microarray) with 15 minutes wear time<br><br>Treatment 2B: 1 x 150 μg Peptide-microneedle array administered into the upper anterior thigh region via a TD delivery system (TD microarray) with 15 minutes wear time<br><br>Treatment 2D: 1 x 80 μg Peptide administered into the periumbilical region in a single SC injection<br><br>Values for Treatments are the least-squares means (LS Means) from the ANOVA.<br>Parameters were ln-transformed prior to analysis.<br>LS Means are calculated by exponentiating the LS Means from the ANOVA.<br>% Mean Ratio = 100*(test/reference)<br>Data from all 10 subjects combined from the 3 periods were used for the SC dose (Treatment 2D). | | | | | |

Peak [Glu[22,25], Leu[23,28,31], Aib[29], Lys[26,30]]hPTHrP(1-34)NH$_2$ exposure from [Glu[22,25], Leu[23,28,31], Aib[29], Lys[26,30]]hPTHrP(1-34)NH$_2$-microneedle array 150 μg, as determined from $C_{max}$, was about 62% of the reference treatment ([Glu[22,25], Leu[23,28,31], Aib[29], Lys[26,30]]hPTHrP(1-34)NH$_2$ for Injection 80 μg). Total exposure, as determined from $AUC_{0-\infty}$, was 17% to 23% of the reference treatment.

The differences in mean $C_{max}$, $AUC_{0-t}$, and $AUC_{0-\infty}$ values between the [Glu[22,25], Leu[23,28,31], Aib[29], Lys[26,30]]hP-THrP(1-34)NH$_2$-microneedle array treatments and [Glu[22,25], Leu[23,28,31], Aib[29], Lys[26,30]]hPTHrP(1-34)NH$_2$ for Injection 80 μg were statistically significant (p-values < 0.05) in most cases.

Peak [Glu[22,25], Leu[23,28,31], Aib[29], Lys[26,30]]hPTHrP(1-34)NH$_2$ exposure from [Glu[22,25], Leu[23,28,31], Aib[29], Lys[26,30]]hPTHrP(1-34)NH$_2$- microneedle array 150 μg administered into the upper anterior thigh region, as determined from $C_{max}$, was about 99% of the reference treatment ([Glu[22,25], Leu[23,28,31], Aib[29], Lys[26,30]]hPTHrP(1-34)NH$_2$- microneedle array 150 μg administered into the periumbilical region). Total exposure, as determined from $AUC_{0-\infty}$, was 133% of the reference treatment.

The differences in mean $C_{max}$, $AUC_{0-t}$, and $AUC_{0-\infty}$ values between [Glu[22,25], Leu[23,28,31], Aib[29], Lys[26,30]]hP-THrP(1-34)NH$_2$- microneedle array 150 μg administered into the periumbilical region and [Glu[22,25], Leu[23,28,31], Aib[29], Lys[26,30]]hPTHrP(1-34)NH$_2$-microneedle array 150 μg administered into the upper anterior thigh region were not statistically significant (p-values > 0.05).

**Table 32: Statistical Comparisons of Plasma [Glu$^{22,25}$, Leu$^{23,28,31}$, Aib$^{29}$, Lys$^{26,30}$]hPTHrP(1-34) NH$_2$Pharmacokinetic Parameters Following [Glu$^{22,25}$, Leu$^{23,28,31}$, Aib29, Lys$^{21,31}$]hPTHrP(1-34)NH$_2$ Treatments (Period 3): Effect of Application Site**

| Comparison | Pharmacokinetic Parameters | LS Means | | % Mean Ratio | P-Value |
|---|---|---|---|---|---|
| | | Test | Reference | | |
| Treatment 3A versus Peptide for Injection 80 μg | $C_{max}$ (pg/mL) | 336.59 | 504.01 | 66.78 | 0.3255 |
| | $AUC_{0-t}$ (pg*hr/mL) | 97.45 | 655.94 | 14.86 | 0.0009 |
| | $AUC_{0-inf}$ (pg*hr/mL) | 109.50 | 699.06 | 15.66 | 0.0004 |
| Treatment 3B versus Treatment 3A | $C_{max}$ (pg/mL) | 489.45 | 336.59 | 145.42 | 0.2017 |
| | $AUC_{0-t}$ (pg*hr/mL) | 166.79 | 97.45 | 171.17 | 0.1267 |
| | $AUC_{0-inf}$ (pg*hr/mL) | 190.16 | 109.50 | 173.66 | 0.0802 |
| Treatment 3B versus Peptide for Injection 80 μg | $C_{max}$ (pg/mL) | 489.45 | 504.01 | 97.11 | 0.9423 |
| | $AUC_{0-t}$ (pg*hr/mL) | 166.79 | 655.94 | 25.43 | 0.0101 |
| | $AUC_{0-inf}$ (pg*hr/mL) | 190.16 | 699.06 | 27.20 | 0.0065 |

Treatment 3A: 1 x 200 μg Peptide-microneedle array administered into the periumbilical region via a TD delivery system (TD microarray) with 15 minutes wear time

Treatment 3B: 1 x 200 μg Peptide-microneedle array administered into the upper outer arm (deltoid) region via a TD delivery system (TD microarray) with 15 minutes wear time

Treatment 3C: 1 x 80 μg Peptide administered into the periumbilical region in a single SC injection

Values for Treatments are the least-squares means (LS Means) from the ANOVA.

Parameters were ln-transformed prior to analysis.

LS Means are calculated by exponentiating the LS Means from the ANOVA.

% Mean Ratio = 100*(test/reference)

Data from all 10 subjects combined from the 3 periods were used for the SC dose (Treatment 3C).

Peak [Glu$^{22,25}$, Leu$^{23,28,31}$, Aib$^{29}$, Lys$^{26,30}$]hPTHrP(1-34)NH$_2$ exposure from [Glu$^{22,25}$, Leu$^{23,28,31}$, Aib$^{29}$, Lys$^{26,30}$]hPTHrP(1-34)NH$_2$- microneedle array 200 μg, as determined from $C_{max}$, ranged from 67% to 97% of the reference treatment ([Glu$^{22,25}$, Leu$^{23,28,31}$, Aib$^{29}$, Lys$^{26,30}$]hPTHrP(1-34)NH$_2$ for Injection 80 μg). Total exposure, as determined from $AUC_{0-\infty}$, was 16% to 27% of the reference treatment.

The differences in mean $C_{max}$, $AUC_{0-t}$, and $AUC_{0-\infty}$ values between the [Glu$^{22,25}$, Leu$^{23,28,31}$, Aib$^{29}$, Lys$^{26,30}$]hPTHrP(1-34)NH$_2$- microneedle array treatments and [Glu$^{22,25}$, Leu$^{23,28,31}$, Aib$^{29}$, Lys$^{26,30}$]hPTHrP(1-34)NH$_2$ for Injection 80 μg were statistically significant (p-values < 0.05) in most cases.

Peak [Glu$^{22,25}$, Leu$^{23,28,31}$, Aib$^{29}$, Lys$^{26,30}$]hPTHrP(1-34)NH$_2$ exposure from [Glu$^{22,25}$, Leu$^{23,28,31}$, Aib$^{29}$, Lys$^{26,30}$]hPTHrP(1-34)NH$_2$- microneedle array 200 μg administered into the upper outer arm (deltoid) region, as determined from $C_{max}$, was about 145% of the reference treatment ([Glu$^{22,25}$, Leu$^{23,28,31}$, Aib$^{29}$, Lys$^{26,30}$]hPTHrP(1-34)NH$_2$- microneedle array 200 μg administered into the periumbilical region). Total exposure, as determined from $AUC_{0-\infty}$, was 174% of the reference treatment.

The differences in mean $C_{max}$, $AUC_{0-t}$, and $AUC_{0-\infty}$ values between [Glu$^{22,25}$, Leu$^{23,28,31}$, Aib$^{29}$, Lys$^{26,30}$]hPTHrP(1-34)NH$_2$- microneedle array 200 μg administered into the periumbilical region and [Glu$^{22,25}$, Leu$^{23,28,31}$, Aib$^{29}$, Lys$^{26,30}$]hPTHrP(1-34)NH$_2$- microneedle array 200 μg administered into the upper outer arm (deltoid) region were not statistically significant (p-values > 0.05).

**Relative Bioavailability ($F_{rel}$):**

The results of relative bioavailability ($F_{rel}$) of -sMTS treatments compared to [Glu$^{22,25}$, Leu$^{23,28,31}$, Aib$^{29}$,

Lys$^{21,30}$]hPTHrP(1-34)NH$_2$ for Injection 80 $\mu$g administered into the periumbilical region in a single SC injection are presented in the following table.

**Table 33: Relative Bioavailability of [Glu$^{22,25}$, Leu$^{23,28,31}$, Aib$^{29}$, Lys$^{26,30}$]hPTHrP(1-34)NH$_2$-Microneedle Array Treatments Compared to [Glu$^{22,25}$, Leu$^{23,28,31}$, Aib$^{29}$, Lys$^{26,30}$]hPTHrP(1-34)NH$_2$ for Injection 80 $\mu$g**

| | | Mean Dose Normalized AUC$_{0\text{-inf}}$ | | | | |
| | | sMTS Doses | | SC Dose | | |
| Treatment | Study Period | Mean | N | Mean | N | Frel |
| --- | --- | --- | --- | --- | --- | --- |
| 1A | 1 | 1.00 | 5 | 9.25 | 10 | 0.108 |
| 1B | 1 | 1.39 | 6 | 9.25 | 10 | 0.151 |
| 1C | 1 | 1.11 | 5 | 9.25 | 10 | 0.120 |
| 1D | 1 | 1.89 | 5 | 9.25 | 10 | 0.204 |
| 2A | 2 | 0.83 | 6 | 9.25 | 10 | 0.090 |
| 2B | 2 | 1.24 | 6 | 9.25 | 10 | 0.134 |
| 2C | 2 | 1.40 | 6 | 9.25 | 10 | 0.151 |
| 3A | 3 | 0.58 | 6 | 9.25 | 10 | 0.063 |
| 3B | 3 | 1.13 | 6 | 9.25 | 10 | 0.122 |
| Data from all 10 subjects combined from .he 3 periods were used for the SC dose. | | | | | | |

Relative bioavailability of [Glu$^{22,25}$, Leu$^{23,28,31}$, Aib$^{29}$, Lys$^{26,30}$]hPTHrP(1-34)NH$_2$-microneedle array treatments compared to [Glu$^{22,25}$, Leu$^{23,28,31}$, Aib$^{29}$, Lys$^{26,30}$]hPTHrP(1-34)NH$_2$ for Injection 80 $\mu$g administered into the periumbilical region in a single SC injection ranged from approximately 6% following 200 $\mu$g [Glu$^{22,25}$, Leu$^{23,28,31}$, Aib$^{29}$, Lys$^{26,30}$]hPTHrP(1-34)NH$_2$ administered to the periumbilical region with 15 minutes wear time (Treatment 3A) to about 20% following 100 $\mu$g [Glu$^{22,25}$, Leu$^{23,28,31}$, Aib$^{29}$, Lys$^{26,30}$]hPTHrP(1-34)NH$_2$ administered in the periumbilical region with 60 minutes wear time (Treatment 1D).

**Dose Proportionality Analysis**

The results of dose proportionality analysis of [Glu$^{22,25}$, Leu$^{23,28,31}$, Aib$^{29}$, Lys$^{26,30}$]hPTHrP(1-34)NH$_2$-sMTS treatments are presented in the following table.

**Table 34: Dose Proportionality Analysis of [Glu$^{22,25}$, Leu$^{23,28,31}$, Aib$^{29}$, Lys$^{26,30}$]hPTHrP(1-34) NH$_2$Pharmacokinetics Parameters Following 100, 150, and 200 $\mu$g [Glu$^{22,25}$, Leu$^{23,28,31}$, Aib$^{29}$, Lys$^{26,30}$]hPTHrP (1-34)NH$_2$-Microneedle Array Treatments**

| Pharmacokinetic Parameters | Slope | Standard *Error* | 95% *CI* |
| --- | --- | --- | --- |
| C$_{max}$ (pg/mL) | -.2522 | 0.2944 | (-0.88, 0.37) |
| AUC$_{0\text{-t}}$ (pg*hr/mL) | -.3069 | 0.3735 | (-1.10, 0.49) |
| AUC$_{0\text{-inf}}$ (pg*hr/mL) | -.2791 | 0.3563 | (-1.03, 0.48) |
| Period 1: 1 x 100 $\mu$g Peptide -microneedle array administered into the periumbilical region via a TD delivery system (TD microarray) with 15 minutes wear time<br>Period 2: 1 x 150 $\mu$g Peptide-microneedle array administered into the periumbilical region via<br>a TD delivery system (TD microarray) with 15 minutes wear time<br>Period 3: 1 x 200 $\mu$g Peptide-microneedle array administered into the periumbilical region via<br>a TD delivery system (TD microarray) with 15 minutes wear time<br><br>Parameters and dose were In-transformed prior to analysis.<br>Dose Proportionality is concluded if the CI for the In-transformed parameters includes the value of 1. | | | |

The 95% CIs for the PK parameters did not contain the value of 1, indicating lack of dose proportionality of the 3 treatments ([Glu$^{22,25}$, Leu$^{23,28,31}$, Aib$^{29}$, Lys$^{26,30}$]hPTHrP(1-34)NH$_2$-microneedle array 100 μg, Treatment 1B, [Glu$^{22,25}$, Leu$^{23,28,31}$, Aib$^{29}$, Lys$^{26,30}$]hPTHrP(1-34)NH$_2$-microneedle array 150 μg Treatment 2A, and [Glu$^{22,25}$, Leu$^{23,28,31}$, Aib$^{29}$, Lys$^{26,30}$]hPTHrP(1-34)NH$_2$-microneedle array 200 μg Treatment 3A) administered into periumbilical region with 15 minutes' wear time. Moreover, the dose ratio, the expected and observed exposure ratios, the negative slopes of the regression lines for the PK parameter, and the display of the PK parameters C$_{max}$, AUC$_{0-t}$, and AUC$_{0-∞}$ versus [Glu$^{22,25}$, Leu$^{21,28,31}$, Aib$^{29}$, Lys$^{26,30}$]hPTHrP(1-34)NH$_2$-microneedle array doses, indicate that the exposure to [Glu$^{22,25}$, Leu$^{23,28,31}$, Aib$^{29}$, Lys$^{26,30}$]hPTHrP(1-34)NH$_2$ was less than proportional to the administered [Glu$^{22,25}$, Leu$^{23,28,31}$, Aib$^{29}$, Lys$^{26,30}$]hPTHrP(1-34)NH$_2$-microneedle array doses.

**Pharmacodynamic Results:**

In presenting the results for the PD markers, the term baseline-adjusted is used to refer to change from baseline.

**Total Serum Calcium**

**Study Period 1 and [Glu$^{22,25}$, Leu$^{23,28,31}$, Aib$^{29}$, Lys$^{26,30}$]hPTHrP(1-34)NH$_2$-Microneedle Array 100 μg With 24 Hours Wear Time (Treatment 2C) From Study Period 2**

Baseline-adjusted total serum calcium concentrations following the [Glu$^{22,25}$, Leu$^{23,28,31}$, Aib$^{29}$, Lys$^{26,30}$]hP-THrP(1-34)NH$_2$-microneedle array 100 μg with wear times ranging from 5 minutes to 24 hours administered into the periumbilical region and [Glu$^{22,25}$, Leu$^{23,28,31}$, Aib$^{29}$, Lys$^{26,30}$]hPTHrP(1-34)NH$_2$ for Injection 80 μg (Treatment 1E), stayed above the baseline levels for most parts of the sampling interval and up to about 8 hours postdose above the placebo levels. Baseline-adjusted total serum calcium concentrations were highest following Treatment 1E compared to other treatments for about 8 hours postdose.
Mean baseline-adjusted total serum calcium concentration ranged from -0.1 to 0.3 mg/dL following [Glu$^{22,25}$, Leu$^{23,28,31}$, Aib$^{29}$, Lys$^{26,30}$]hPTHrP(1-34)NH$_2$-microneedle array treatments, ranged from 0.1 to 0.4 mg/dL following Treatment IE, and -0.1 to 0.3 mg/dL following placebo. The mean maximum change from baseline in total serum calcium concentrations (Δ$_{max}$) was 0.3 to 0.5 mg/dL following [Glu$^{22,25}$, Leu$^{23,28,31}$, Aib$^{29}$, Lys$^{26,30}$]hPTHrP(1-34)NH$_2$-micronee-dle array treatments, 0.5 mg/dL following Treatment 1E, and 0.0 following placebo.

**Study Period 2**

Baseline-adjusted total serum calcium concentrations following the [Glu$^{22,25}$, Leu$^{23,28,31}$, Aib$^{29}$, Lys$^{26,30}$]hP-THrP(1-34)NH$_2$-microneedle arrays 150 μg (administered into the periumbilical region [Treatments 2A] and into the upper anterior thigh region [Treatments 2B]) and [Glu$^{22,25}$, Leu$^{23,28,31}$, Aib$^{29}$, Lys$^{26,30}$]hPTHrP(1-34)NH$_2$ for Injection 80 μg (Treatment 2D) generally stayed above the baseline levels for up to about 8 hours postdose. The highest baseline-adjusted total serum calcium concentrations resulted following Treatment 2D. Baseline-adjusted total serum calcium concentration was generally higher following the administration of Treatment 2A compared to administration of Treatment 2B, both with 15 minute wear times.
Mean baseline-adjusted total serum calcium concentration ranged from 0.0 to 0.3 mg/dL following [Glu$^{22,25}$, Leu$^{23,28,31}$, Aib$^{29}$, Lys$^{26,30}$]hPTHrP(1-34)NH$_2$-microneedle arrays 150 μg, ranged from 0.2 to 0.5 mg/dL following Treatment 2D, and 0.0 to 0.3 mg/dL following placebo. The mean maximum change from baseline in total serum calcium concentrations (Δ$_{max}$) ranged from 0.3 to 0.4 mg/dL following [Glu$^{22,25}$, Leu$^{23,28,31}$, Aib$^{29}$, Lys$^{26,30}$]hP-THrP(1-34)NH$_2$-microneedle arrays 150 μg, 0.6 mg/dL following Treatment 2D and 0.2 mg/dL following placebo.
Mean baseline-adjusted total serum calcium concentration ranging from 0.0 to 0.3 mg/dL were similar following Treatments 2A and 2B. The mean Δ$_{max}$ value at 0.4 mg/dL following Treatment 2A was comparable to the mean Δ$_{max}$ value following Treatment 2B at 0.3 mg/dL.

**Study Period 3**

Baseline-adjusted total serum calcium concentrations following the [Glu$^{22,25}$, Leu$^{23,28,31}$, Aib$^{29}$, Lys$^{26,30}$]hP-THrP(1-34)NH$_2$-microneedle array 200 μg (administered into the periumbilical region [Treatments 3A] and into upper outer arm [deltoid] region, [Treatments 3B]) and [Glu$^{22,25}$, Leu$^{23,28,31}$, Aib$^{29}$, Lys$^{26,30}$]hPTHrP(1-34)NH$_2$ for injection 80 μg (Treatment 3C), mostly remained around the baseline levels throughout the sampling interval and for up to about 8 hours postdose above the placebo level. Baseline-adjusted total serum calcium concentrations were generally higher following the administration of Treatment 3B compared to the administration of Treatment 3A, both with 15 minute wear times.

Mean baseline-adjusted total serum calcium concentration ranged from 0.0 to 0.3 mg/dL following [Glu$^{22,25}$, Leu$^{23,28,31}$, Aib$^{29}$, Lys$^{26,30}$]hPTHrP(1-34)NH$_2$ -microneedle array 200 $\mu$g, -0.5 to 0.1 mg/dL following Treatment 3C, and -0.3 to 0.2 mg/dL following placebo. The mean maximum changes from baseline in total serum calcium concentrations ($\Delta_{max}$) were 0.0 and 0.3 mg/dL following [Glu$^{22,25}$, Leu$^{23,28,31}$, Aib$^{29}$, Lys$^{26,30}$]hPTHrP(1-34)NH$_2$-microneedle array 200 $\mu$g, -0.5 mg/dL following Treatment 3C, and 0.0 following placebo.

Mean baseline-adjusted total serum calcium concentrations ranged from -0.2 to 0.3 mg/dL following Treatment 3A and ranged from 0.0 to 0.3 mg/dL following Treatment 3B. Mean $\Delta_{max}$ values were 0.0 following Treatment 3A and 0.3 mg/dL following Treatment 3B.

**Serum Phosphorus**

Study Period 1 and [Glu$^{22,25}$, Leu$^{23,28,31}$, Aib$^{29}$, Lys$^{26,30}$]hPTHrP(1-34)NH$_2$-microneedle array 100 $\mu$g with 24 hours wear time (Treatment 2C) From Study Period 2
Baseline-adjusted serum phosphorus concentrations following the [Glu$^{22,25}$, Leu$^{23,28,31}$, Aib$^{29}$, Lys$^{26,30}$]hPTHrP(1-34)NH$_2$-microneedle array 100 $\mu$g with wear times ranging from 5 minutes to 24 hours administered into the periumbilical region and [Glu$^{22,25}$, Leu$^{23,28,31}$, Aib$^{29}$, Lys$^{26,30}$]hPTHrP(1-34)NH$_2$ for Injection 80 $\mu$g (Treatment 1E) fluctuated around baseline levels for about 8 hours postdose and rose above baseline levels thereafter. Baseline-adjusted serum phosphorus concentrations following the [Glu$^{22,25}$, Leu$^{23,28,31}$, Aib$^{29}$, Lys$^{26,30}$]hPTHrP(1-34)NH$_2$-microneedle arrays 100 $\mu$g and Treatment IE were generally above those of the placebo.

Mean baseline-adjusted serum phosphorus concentrations ranged from -0.2 to 0.8 mg/dL following [Glu$^{22,25}$, Leu$^{23,28,31}$, Aib$^{29}$, Lys$^{26,30}$]hPTHrP(1-34)NH$_2$-microneedle arrays 100 $\mu$g, -0.2 to 0.4 mg/dL following Treatment IE, and -0.3 to 0.5 mg/dL following placebo. The mean maximum change from baseline serum phosphorus concentrations ($\Delta_{max}$) ranged from 0.3 to 0.9 mg/dL following [Glu$^{22,25}$, Leu$^{23,28,31}$, Aib$^{29}$, Lys$^{26,30}$]hPTHrP(1-34)NH$_2$-microneedle arrays 100 $\mu$g, 0.3 mg/dL following Treatment IE, and 0.4 mg/dL following placebo.

**Study Period 2**

Baseline-adjusted serum phosphorus concentrations following the [Glu$^{22,25}$, Leu$^{23,28,31}$, Aib$^{29}$, Lys$^{26,30}$]hPTHrP(1-34)NH$_2$-microneedle arrays 150 $\mu$g (administered into the periumbilical region [Treatments 2A] and into the upper anterior thigh region [Treatments 2B]) and [Glu$^{22,25}$, Leu$^{23,28,31}$, Aib$^{29}$, Lys$^{26,30}$]hPTHrP(1-34)NH$_2$ for Injection 80 $\mu$g (Treatment 2D) mostly fluctuated around the baseline levels for about 8 hours postdose and rose above the baseline levels thereafter. Baseline-adjusted serum phosphorus concentrations following Treatment 2B and Treatment 2D were generally above the placebo levels. Moreover, baseline-adjusted serum phosphorus concentrations were higher following Treatment 2B compared to Treatment 2A, both with 15 minute wear times.

Mean baseline-adjusted serum phosphorus concentrations ranged from -0.1 to 1.0 mg/dL following [Glu$^{22,25}$, Leu$^{23,28,31}$, Aib$^{29}$, Lys$^{26,30}$]hPTHrP(1-34)NH$_2$-microneedle arrays 150 $\mu$g, -0.3 to 0.6 mg/dL following Treatment 2D, and -0.1 to 0.4 mg/dL following placebo. The mean maximum change from baseline serum phosphorus concentrations ($\Delta_{max}$) ranged from 0.2 to 1.0 mg/dL following [Glu$^{22,25}$, Leu$^{23,28,31}$, Aib$^{29}$, Lys$^{26,30}$]hPTHrP(1-34)NH$_2$-microneedle arrays 150 $\mu$g, 0.6 mg/dL following Treatment 2D, and 0.4 mg/dL following placebo.

Mean baseline-adjusted serum phosphorus concentrations ranged from -0.2 to 0.3 mg/dL following Treatment 2A and ranged from -0.1 to 1.0 mg/dL following Treatment 2B. The mean $\Delta_{max}$ values were 0.2 mg/dL following Treatment 2A and 1.0 mg/dL following Treatment 2B.

**Study Period 3**

Baseline-adjusted serum phosphorus concentrations following the [Glu$^{22,25}$, Leu$^{23,28,31}$, Aib$^{29}$, Lys$^{26,30}$]hPTHrP(1-34)NH$_2$-microneedle arrays 200 $\mu$g (administered into the periumbilical region [Treatments 3A] and into upper outer arm [deltoid] region, [Treatments 3B]) and [Glu$^{22,25}$, Leu$^{23,28,31}$, Aib$^{29}$, Lys$^{26,30}$]hPTHrP(1-34)NH$_2$ for Injection 80 $\mu$g (Treatment 3C), fluctuated around the baseline levels for about 8 hours postdose and rose above the baseline levels thereafter. Baseline-adjusted serum phosphorus concentrations following [Glu$^{22,25}$, Leu$^{23,28,31}$, Aib$^{29}$, Lys$^{26,30}$]hPTHrP(1-34)NH$_2$-microneedle arrays 200 $\mu$g and Treatment 3C were generally above the placebo level. Baseline-adjusted serum phosphorus concentrations were generally higher for Treatment 3B compared Treatment 3A.

Mean baseline-adjusted serum phosphorus concentrations ranged from -0.4 to 0.6 mg/dL following [Glu$^{22,25}$, Leu$^{23,28,31}$, Aib$^{29}$, Lys$^{26,30}$]hPTHrP(1-34)NH$_2$-microneedle arrays 200 $\mu$g, -0.7 to 0.4 mg/dL following Treatment 3C, and -0.3 to 0.3 following placebo. The mean maximum change from baseline serum phosphorus concentrations ($\Delta_{max}$) were 0.4 and 0.6 mg/dL following [Glu$^{22,25}$, Leu$^{23,28,31}$, Aib$^{29}$, Lys$^{26,30}$]hPTHrP(1-34)NH$_2$-Microneedle arrays 200 $\mu$g, -0.7 mg/dL following Treatment 3C, and 0.2 mg/dL following placebo.

Mean baseline-adjusted serum phosphorus concentrations ranged from -0.4 to 0.6 mg/dL following Treatment 3A and -0.1 to 0.6 mg/dL following Treatment 3B. Mean $\Delta_{max}$ values were 0.4 mg/dL following Treatment 3A and 0.6 mg/dL following Treatment 3B.

**1, 25-dihydroxyvitamin D Study Period 1 and [Glu$^{22,25}$, Leu$^{23,28,31}$, Aib$^{29}$, Lys$^{26,30}$]hPTHrP(1-34)NH$_2$-Microneedle Arrays 100 μg with 24 hours wear time (Treatment 2C) From Study Period 2**

Baseline-adjusted serum 1, 25-dihydroxyvitamin D concentrations following the [Glu$^{22,25}$, Leu$^{23,28,31}$, Aib$^{29}$, Lys$^{26,30}$]hPTHrP(1-34)NH$_2$-sMTS 100 μg with wear times ranging from 5 minutes to 24 hours administered into the periumbilical region and [Glu$^{22,25}$, Leu$^{23,28,31}$, Aib$^{29}$, Lys$^{26,30}$]hPTHrP(1-34)NH$_2$ for injection 80 μg (Treatment 1E) stayed above the baseline levels. The highest baseline-adjusted serum 1, 25-dihydroxyvitamin D concentrations resulted after 3 hours postdose following Treatment IE.

Mean baseline-adjusted serum 1, 25-dihydroxyvitamin D concentrations ranged from 0.5 to 16.1 pg/mL following [Glu$^{22,25}$, Leu$^{23,28,31}$, Aib$^{29}$, Lys$^{26,30}$]hPTHrP(1-34)NH$_2$-microneedle array 100 μg, -0.6 to 26.2 pg/mL following Treatment 1E, and 1.1 to 7.1 pg/mL following placebo. The mean maximum change from baseline serum 1,25-dihydroxyvitamin D concentration ($\Delta_{max}$) ranged from 2.9 to 27.1 pg/mL following [Glu$^{22,25}$, Leu$^{23,28,31}$, Aib$^{29}$, Lys$^{26,30}$]hPTHrP(1-34)NH$_2$-microneedle array 100 μg, 30.2 pg/mL following Treatment IE, and 8.0 pg/mL following placebo.

**Study Period 2**

Baseline-adjusted serum 1, 25-dihydroxyvitamin D concentrations following the [Glu$^{22,25}$, Leu$^{23,28,31}$, Aib$^{29}$, Lys$^{26,30}$]hPTHrP(1-34)NH$_2$- microneedle array 150 μg (administered into the periumbilical region [Treatments 2A] and into the upper anterior thigh region [Treatments 2B]) and [Glu$^{22,25}$, Leu$^{23,28,31}$, Aib$^{29}$, Lys$^{26,30}$]hPTHrP(1-34)NH$_2$ for injection 80 μg (Treatment 2D) mostly remained above the baseline levels. The highest baseline-adjusted serum 1, 25-dihydroxyvitamin D concentrations resulted following Treatment 2D.

Mean baseline-adjusted serum 1, 25-dihydroxyvitamin D concentrations ranged from -14.2 to 11.0 pg/mL following [Glu$^{22,25}$, Leu$^{23,28,31}$, Aib$^{29}$, Lys$^{26,30}$]hPTHrP(1-34)NH$_2$-microneedle array 150 μg, 7.6 to 32.2 pg/mL following Treatment 2D, and 3.4 to 14.9 pg/mL following placebo. The mean maximum change from baseline serum 1, 25-dihydroxyvitamin D level ($\Delta_{max}$) ranged from -4.5 to 0.3 pg/mL following [Glu$^{22,25}$, Leu$^{23,28,31}$, Aib$^{29}$, Lys$^{26,30}$]hPTHrP(1-34)NH$_2$- microneedle array 150 μg, 32.2 pg/mL following Treatment 2D, and 17.0 pg/mL following placebo.

Mean baseline-adjusted serum 1, 25-dihydroxyvitamin D concentrations ranged from -14.2 to 11.0 pg/mL and from -2.5 to 11 pg/mL following Treatments 2A and 2B, respectively. Mean $\Delta_{max}$ values were -4.5 pg/mL following Treatment 2A and 0.3 pg/mL following Treatment 2B.

**Study Period 3**

Baseline-adjusted serum 1, 25-dihydroxyvitamin D concentrations following the [Glu$^{22,25}$, Leu$^{23,28,31}$, Aib$^{29}$, Lys$^{26,30}$]hPTHrP(1-34)NH$_2$- microneedle array 200 μg (administered into the periumbilical region [Treatments 3A] and into upper outer arm [deltoid] region, [Treatments 3B]) and [Glu$^{22,25}$, Leu$^{23,28,31}$, Aib$^{29}$, Lys$^{26,30}$]hPTHrP(1-34)NH$_2$ for Injection 80 μg (Treatment 3C) mostly remained above the baseline levels and, after 8 hours postdose, above the placebo levels. Baseline-adjusted serum 1, 25-dihydroxyvitamin D concentrations were generally higher following Treatment 3B compared to Treatment 3A.

Mean baseline-adjusted serum 1, 25-dihydroxyvitamin D concentrations ranged from -5.1 to 22.5 pg/mL following [Glu$^{22,25}$, Leu$^{23,28,31}$, Aib$^{29}$, Lys$^{26,30}$]hPTHrP(1-34)NH$_2$-microneedle array 200 μg, -0.2 to 25.9 pg/mL following Treatment 3C, and -0.7 to 19.0 pg/mL following placebo. The mean maximum changes from baseline serum 1, 25-dihydroxyvitamin D concentrations ($\Delta_{max}$) were 9.0 and 22.6 pg/mL following [Glu$^{22,25}$, Leu$^{23,28,31}$, Aib$^{29}$, Lys$^{26,30}$]hPTHrP(1-34)NH$_2$- microneedle array 200 μg, 30.2 pg/mL following Treatment 3C, and 10.5 pg/mL following placebo.

Mean baseline-adjusted serum 1, 25-dihydroxyvitamin D concentrations ranged from -5.1 to 11.5 pg/mL following Treatment 3A and 7.2 to 22.5 pg/mL following Treatment 3B. Mean $\Delta_{max}$ values were 9.0 pg/mL following Treatment 3A and 22.6 pg/mL following Treatment 3B.

**Pharmacokinetics:**

Peak [Glu$^{22,25}$, Leu$^{23,28,31}$, Aib$^{29}$, Lys$^{26,30}$]hPTHrP(1-34)NH$_2$ exposure from [Glu$^{22,25}$, Leu$^{23,28,31}$, Aib$^{29}$, Lys$^{26,30}$]hPTHrP(1-34)NH$_2$- microneedle array 100 μg, as determined from $C_{max}$, ranged from 45% to 73% of the reference treatment ([Glu$^{22,25}$, Leu$^{23,28,31}$, Aib$^{29}$, Lys$^{26,30}$]hPTHrP(1-34)NH$_2$ for Injection 80 μg). Total exposure, as determined from $AUC_{0-\infty}$, was 11% to 18% of the reference treatment.

Peak [Glu$^{22,25}$, Leu$^{23,28,31}$, Aib$^{29}$, Lys$^{26,30}$]hPTHrP(1-34)NH$_2$ exposure from [Glu$^{22,25}$, Leu$^{23,28,31}$, Aib$^{29}$, Lys$^{26,30}$]hPTHrP(1-34)NH$_2$- microneedle array 150 μg, as determined from $C_{max}$, was about 62% of the reference treatment ([Glu$^{22,25}$, Leu$^{23,28,31}$, Aib$^{29}$, Lys$^{26,30}$]hPTHrP(1-34)NH$_2$ for Injection 80 $\mu$g). Total exposure, as determined from AUC$_{0-\infty}$, was 17% to 23% of the reference treatment.

Peak [Glu$^{22,25}$, Leu$^{23,28,31}$, Aib$^{29}$, Lys$^{26,30}$]hPTHrP(1-34)NH$_2$exposure from [Glu$^{22,25}$, Leu$^{23,28,31}$, Aib$^{29}$, Lys$^{26,30}$]hPTHrP(1-34)NH$_2$- microneedle array 200 $\mu$g, as determined from C$_{max}$, ranged from 67% to 97% of the reference treatment ([Glu$^{22,25}$, Leu$^{23,28,31}$, Aib$^{29}$, Lys$^{26,30}$]hPTHrP(1-34)NH$_2$ for Injection 80 $\mu$g). Total exposure, as determined from AUC$_{0-\infty}$, was 16% to 27% of the reference treatment.

Mean relative bioavailability of [Glu$^{22,25}$, Leu$^{23,28,31}$, Aib$^{29}$, Lys$^{26,30}$]hPTHrP(1-34)NH$_2$- microneedle array 100 $\mu$g ranged from about 11% to 20%, of [Glu$^{22,25}$, Leu$^{23,28,31}$, Aib$^{29}$, Lys$^{26,30}$]hPTHrP(1-34)NH$_2$- microneedle array 150 $\mu$g was 9% (when administered into the periumbilical region) and 13% (when administered into the upper anterior thigh region), and of [Glu$^{22,25}$, Leu$^{23,28,31}$, Aib$^{29}$, Lys$^{26,30}$]hPTHrP(1-34)NH$_2$- microneedle array 200 $\mu$g was about 6% (when administered into the periumbilical region) and 12% (when administered into the upper outer arm [deltoid] region) when compared to [Glu$^{22,25}$, Leu$^{23,28,31}$, Aib$^{29}$, Lys$^{26,30}$]hPTHrP(1-34)NH$_2$ for Injection 80 $\mu$g.

Relationship between wear time and exposure to [Glu$^{22,25}$, Leu$^{23,28,31}$, Aib$^{29}$, Lys$^{26,30}$]hPTHrP(1-34)NH$_2$ was not apparent from the results of this investigation. The relative bioavailability ranged from about 6% to 20% irrespective of wear time.

The mean relative bioavailability was comparable following [Glu$^{22,25}$, Leu$^{23,28,31}$, Aib$^{29}$, Lys$^{26,30}$]hP-THrP(1-34)NH$_2$- microneedle array 150 $\mu$g administration between the periumbilical region and the upper anterior thigh region. Peak [Glu$^{22,25}$, Leu$^{23,28,31}$, Aib$^{29}$, Lys$^{26,30}$]hPTHrP(1-34)NH$_2$ exposures from [Glu$^{22,25}$, Leu$^{23,28,31}$, Aib$^{29}$, Lys$^{26,30}$]hPTHrP(1-34)NH$_2$- microneedle array 150 $\mu$g administered into the periumbilical region and upper anterior thigh region, as determined from C$_{max}$, were about 62% of the reference treatment ([Glu$^{22,25}$, Leu$^{23,28,31}$, Aib$^{29}$, Lys$^{26,30}$]hPTHrP(1-34)NH$_2$ for Injection 80 $\mu$g). Total exposures, as determined from AUC$_{0-\infty}$, were 17% and 23% of the reference treatment, respectively.

The mean relative bioavailability was higher following [Glu$^{22,25}$, Leu$^{23,28,31}$, Aib$^{29}$, Lys$^{26,30}$]hPTHrP(1-34)NH$_2$-microneedle array 200 $\mu$g administration to the deltoid region than to the periumbilical region. Peak [Glu$^{22,25}$, Leu$^{23,28,31}$, Aib$^{29}$, Lys$^{26,30}$]hPTHrP(1-34)NH$_2$ exposures from [Glu$^{22,25}$, Leu$^{23,28,31}$, Aib$^{29}$, Lys$^{26,30}$]hPTHrP(1-34)NH$_2$- microneedle array 200 $\mu$g administered into the periumbilical and the deltoid regions, as determined from C$_{max}$, were about 67% and 97% of the reference treatment ([Glu$^{22,25}$, Leu$^{23,28,31}$, Aib$^{29}$, Lys$^{26,30}$]hPTHrP(1-34)NH$_2$ for Injection 80 $\mu$g), respectively. Total exposures, as determined from AUC$_{0-\infty}$, were 16% and 27% of the reference treatment, respectively.

**Pharmacodynamics:**

Baseline-adjusted total serum calcium concentrations either marginally or transiently increased following [Glu$^{22,25}$, Leu$^{23,28,31}$, Aib$^{29}$, Lys$^{26,30}$]hPTHrP(1-34)NH$_2$- microneedle array treatments and [Glu$^{22,25}$, Leu$^{23,28,31}$, Aib$^{29}$, Lys$^{26,30}$]hPTHrP(1-34)NH$_2$ for Injection 80 $\mu$g that remained within the normal laboratory range, or remained around the baseline levels. Baseline-adjusted total serum calcium concentrations rose above the placebo levels up to about 8 hours postdose and either fell below the placebo levels or overlapped with the placebo afterwards.

Baseline-adjusted total serum calcium concentrations were higher following the application of [Glu$^{22,25}$, Leu$^{23,28,31}$, Aib$^{29}$, Lys$^{26,30}$]hPTHrP(1-34)NH$_2$- microneedle array 150 $\mu$g to the periumbilical region compared to the upper anterior thigh region and were higher following the application of [Glu$^{22,25}$, Leu$^{23,28,31}$, Aib$^{29}$, Lys$^{26,30}$]hP-THrP(1-34)NH$_2$-microneedle array 200 $\mu$g to the upper outer arm (deltoid) region compared to the periumbilical region, indicating the effect of the site of [Glu$^{22,25}$, Leu$^{23,28,31}$, Aib$^{29}$, Lys$^{26,30}$]hPTHrP(1-34)NH$_2$ administration on total serum calcium concentrations.

Baseline-adjusted serum phosphorus concentrations following [Glu$^{22,25}$, Leu$^{23,28,31}$, Aib$^{29}$, Lys$^{26,30}$]hP-THrP(1-34)NH$_2$- microneedle array treatments and [Glu$^{22,25}$, Leu$^{23,28,31}$, Aib$^{29}$, Lys$^{26,30}$]hPTHrP(1-34)NH$_2$ for Injection 80 $\mu$g fluctuated around the baseline levels for approximately 8 hours postdose and rose above the baseline levels for the remainder of the sampling interval. Serum phosphorus concentrations were generally above the placebo levels and at times overlapped with the placebo.

Baseline-adjusted serum phosphorus concentrations were higher following the application of [Glu$^{22,25}$, Leu$^{23,28,31}$, Aib$^{29}$, Lys$^{26,30}$]hPTHrP(1-34)NH$_2$- microneedle array 150 $\mu$g to the upper anterior thigh region compared to the periumbilical region and were higher following the application of [Glu$^{22,25}$, Leu$^{23,28,31}$, Aib$^{29}$, Lys$^{26,30}$]hP-THrP(1-34)NH$_2$-microneedle array 200 $\mu$g to the upper outer arm (deltoid) region compared to the periumbilical region, indicating the effect of the site of [Glu$^{22,25}$, Leu$^{23,28,31}$, Aib$^{29}$, Lys$^{26,30}$]hPTHrP(1-34)NH$_2$administration on serum phosphorus concentrations.

Baseline-adjusted serum 1, 25-dihydroxyvitamin D concentrations increased following [Glu$^{22,25}$, Leu$^{23,28,31}$, Aib$^{29}$, Lys$^{26,30}$]hPTHrP(1-34)NH$_2$- microneedle array treatments and [Glu$^{22,25}$, Leu$^{23,28,31}$, Aib$^{29}$, Lys$^{26,30}$]hP-THrP(1-34)NH$_2$ for Injection 80 $\mu$g compared to baseline levels. Baseline-adjusted serum 1, 25-dihydroxyvitamin D concentrations were either above the placebo levels or overlapped with the placebo.

While there was no clear trend in serum 1, 25-dihydroxyvitamin D concentrations following the application of [Glu$^{22,25}$, Leu$^{23,28,31}$, Aib$^{29}$, Lys$^{26,30}$]hPTHrP(1-34)NH$_2$-microneedle array 150 $\mu$g to periumbilical region and the upper anterior thigh region, baseline-adjusted serum 1, 25-dihydroxyvitamin D concentrations were generally higher following application of [Glu$^{22,25}$, Leu$^{23,28,31}$, Aib$^{29}$, Lys$^{26,30}$]hPTHrP(1-34)NH$_2$- microneedle array 200 $\mu$g to the upper outer arm (deltoid) region and remained above the baseline level during the entire sampling time compared to the periumbilical region.

**Safety:**

Single-dose administration of up to 200 $\mu$g [Glu$^{22,25}$, Leu$^{23,28,31}$, Aib$^{29}$, Lys$^{26,30}$]hPTHrP(1-34)NH$_2$- microneedle array TD microarray patch and 80 $\mu$g [Glu$^{22,25}$, Leu$^{23,28,31}$, Aib$^{29}$, Lys$^{26,30}$]hPTHrP(1-34)NH$_2$ for Injection appeared to be safe and generally well tolerated by this group of healthy postmenopausal female volunteers.

[Glu$^{22,25}$, Leu$^{23,28,31}$, Aib$^{29}$, Lys$^{26,30}$]hPTHrP(1-34)NH$_2$- microneedle array was well tolerated at the application site, with minor irritation consisting mostly of mild erythema and swelling. Note that the comparison of the composite irritancy score between subjects receiving active TD microarray versus placebo indicated that irritancy was not associated with the amount of the active component [Glu$^{22,25}$, Leu$^{23,28,31}$, Aib$^{29}$, Lys$^{26,30}$]hPTHrP(1-34)NH$_2$.

**Clinical Study 2**

Clinical study evaluation of pharmacokinetics of [Glu$^{22,25}$, Leu$^{23,28,31}$, Aib$^{29}$, Lys$^{26,30}$]hPTHrP(1-34)NH$_2$ (ng/mL) LCP-coated microarrays in postmenopausal women.

**Study Design**

## Table 35: Arrays used

| Mmicroneedle Array | Description |
|---|---|
| Material of Construction | Liquid Crystal Polymer (LCP) |
| Number of Microneedles | 316 |
| Flexural Modulus (by ISO 178) | 9100 |
| Grade | Class VI, medical grade polymer |
| Surface area | 5.5 cm$^2$ or ~27 mm in diameter |
| Depth of Penetration (DOP) | 250+/-10$\mu$m |
| Height of Microneedles | 500$\mu$m |
| Spacing between Microneedles | 550$\mu$m apart (tip to tip) |

Array loading dosages tested

Array 1:    100 $\mu$g per array +/- 15 $\mu$g per array (104 $\mu$g per array mean)

Array 2:    150 $\mu$g per array +/- 22.5 $\mu$g per array (146 $\mu$g per array mean)

Arrays were prepared using aqueous formulations of 54 to 58 wt-% [Glu$^{22,25}$, Leu$^{23,28,31}$, Aib$^{29}$, Lys$^{26,30}$]PTHrP(1-34)NH$_2$ and phosphate buffered saline.

A second phase 1 clinical study was conducted utilizing [Glu$^{22,25}$, Leu$^{23,28,31}$, Aib$^{29}$, Lys$^{26,30}$]hPTHrP(1-34)NH$_2$ coated LCP microarrays in postmenopausal women. The study was designed to evaluate the utility of a new array material (LCP) and shorter application time (10 seconds and 15 minutes) as well as to evaluate the site of administration on relative bioavailability and C$_{max}$ values and pharmacodynamic parameters as occurred in the PCS study discussed above in the previous example.

This second study was a randomized, double-blind, placebo-controlled, single- and multiple-dose safety, PK, and tolerability study of [Glu$^{22,25}$, Leu$^{23,28,31}$, Aib$^{29}$, Lys$^{26,30}$]hPTHrP(1-34)NH$_2$ coated LCP arrays administered transdermally to healthy postmenopausal women.

This study was conducted at 1 study site and consisted of 3 study periods. In Study Period 1, subjects were to receive a single administration of the following: [Glu$^{22,25}$, Leu$^{23,28,31}$, Aib$^{29}$, Lys$^{26,30}$]hPTHrP(1-34)NH$_2$ coated LCP array 100 µg, [Glu$^{22,25}$, Leu$^{23,28,31}$, Aib$^{29}$, Lys$^{26,30}$]hPTHrP(1-34)NH$_2$ coated LCP array-Placebo, or a single SC administration of [Glu$^{22,25}$, Leu$^{23,28,31}$, Aib$^{29}$, Lys$^{26,30}$]hPTHrP(1-34)NH$_2$ for Injection 80 µg. Application sites were to be either periumbilical or upper thigh regions each with 2 wear times of 10 seconds and 15 minutes. Subjects enrolled in Study Period 2 were to receive [Glu$^{22,25}$, Leu$^{23,28,31}$, Aib$^{29}$, Lys$^{26,30}$]hPTHrP(1-34)NH$_2$ coated LCP array 100 or 150 µg or [Glu$^{22,25}$, Leu$^{23,28,31}$, Aib$^{29}$, Lys$^{26,30}$]hPTHrP(1-34)NH$_2$ coated LCP array -Placebo for 7 consecutive days. Application sites were to be either periumbilical with 10 second and 15 minute wear times or upper thigh regions with a 15 minute wear time. Subjects enrolled in Study Period 3 were to receive [Glu$^{22,25}$, Leu$^{23,28,31}$, Aib$^{29}$, Lys$^{26,30}$]hPTHrP(1-34)NH$_2$ coated LCP array 150 µg or [Glu$^{22,25}$, Leu$^{23,28,31}$, Aib$^{29}$, Lys$^{26,30}$]hPTHrP(1-34)NH$_2$ coated LCP array-Placebo over a range of application times for 7 consecutive days. Application times were to included 30 seconds, 1, 5, 15, 60 minutes, and 24 hours. New subjects were to be enrolled in each study period.

Standard safety evaluations were to be included in this study to ensure the safety of subjects. These safety evaluations were to include physical examinations, vital signs, 12-lead ECGs, clinical laboratory tests, and monitoring and recording of local tolerance and AEs. As a precaution and to ensure that the study procedures were to be performed according to protocol, subjects were to remain under direct supervision during the PK and PD assessment periods and were not to be released from the clinical facility until the Principal Investigator determined that it was safe to do so.

To facilitate safety and tolerability assessments and to reduce bias in interpretation of results, a randomized, double-blind, placebo-controlled design was utilized. A [Glu$^{22,25}$, Leu$^{23,28,31}$, Aib$^{29}$, Lys$^{26,30}$]hPTHrP(1-34)NH$_2$ coated LCP array group size of 6 or 8 subjects per dose level (6 active or 6 active/2 placebo) was chosen as appropriate for an early phase clinical trial of safety and tolerability in which clinical judgment was to be used to determine the enrollment of subjects into subsequent periods. The lowest dose, 100 µg, was administered in the first period. Subjects in subsequent periods were to receive 100 or 150 µg, subject to the safety and tolerability of [Glu$^{22,25}$, Leu$^{23,28,31}$, Aib$^{29}$, Lys$^{26,30}$]hPTHrP(1-34)NH$_2$ coated LCP array -Active in the preceding period.

**Table 36: Arrays used**

| Period 1 | | | | |
|---|---|---|---|---|
| Study Group | Frequency of Dosing | Dose | Application or Injection Site | WearTime |
| 1a | Once | 100 µg | Periumbilical | 10 seconds |
| 1b | Once | 100 µg | Periumbilical | 15 minutes |
| 1c | Once | 100 µg | Upper Thigh | 10 seconds |
| 1d | Once | 100 µg | Upper Thigh | 15 minutes |
| 1e | Once | 80 µg | Periumbilical | N/A |
| | | Total: | | |
| **Period 2** | | | | |
| Study Group | Frequency of Dosing | Dose | Application Site | Wear Time |
| 2a | Daily X 7 | 150 µg | Periumbilical | 10 seconds |
| 2b | Daily X 7 | 150 µg | Periumbilical | 15 minutes |
| 2c | Daily X 7 | 100 µg | Upper thigh | 15 minutes |
| | | Total: | | |
| **Period 3** | | | | |
| Study Group | Frequency of Dosing | Dose | Application Site | Wear Time |
| 3a | Daily X 7 | 150 µg | Upper Thigh | 5 minutes on Days 1 - 6, 30 seconds on Day 7 |
| 3b | Daily X 7 | 150 µg | Upper Thigh | 1 minute on Days 1 - 6, 60 minutes on Day 7 |
| 3c | Daily X 7 | 150 µg or Placebo | Upper Thigh | 150 µg: Day 1 24 hours, 15 minutes on Day 7. |

(continued)

| Period 3 | | | | |
|---|---|---|---|---|
| Study Group | Frequency of Dosing | Dose | Application Site | Wear Time |
| | | | | Placebo: Day 1 24 hours, Days 2, 3, 4, 5, and 6 for 60, 15 , 5 , and 1 minute, and 30 seconds, respectively |

Study Period 1 was to include 4 study groups (1A, 1B, 1C, and ID) receiving [Glu$^{22,25}$, Leu$^{23,28,31}$, Aib$^{29}$, Lys$^{26,30}$]hPTHrP(1-34)NH$_2$ coated LCP array 100 $\mu$g with embedded [Glu$^{22,25}$, Leu$^{23,28,31}$, Aib$^{29}$, Lys$^{26,30}$]hP-THrP(1-34)NH$_2$ coated LCP array placebo within 2 (1B and ID) of the 4 study groups and a fifth group (1E) to receive [Glu$^{22,25}$, Leu$^{23,28,31}$, Aib$^{29}$, Lys$^{26,30}$]hPTHrP(1-34)NH$_2$ for Injection 80 $\mu$g. The major purpose of the 4 [Glu$^{22,25}$, Leu$^{23,28,31}$, Aib$^{29}$, Lys$^{21,30}$]hPTHrP(1-34)NH$_2$ coated LCP array 100 $\mu$g study groups was to define the impact of wear time (10 seconds and 15 minutes) and application site (periumbilical and upper thigh) on relative bioavailability of [Glu$^{22,25}$, Leu$^{23,28,31}$, Aib$^{29}$, Lys$^{26,30}$]hPTHrP(1-34)NH$_2$ coated LCP array compared to [Glu$^{22,25}$, Leu$^{23,28,31}$, Aib$^{29}$, Lys$^{26,30}$]hP-THrP(1-34)NH$_2$ for Injection 80 $\mu$g. [Glu$^{22,25}$, Leu$^{23,28,31}$, Aib$^{29}$, Lys$^{26,30}$]hPTHrP(1-34)NH$_2$ for Injection 80 $\mu$g administered SC (fifth group, Study Group 1E) was to serve as a positive control group since this dose has been demonstrated to exhibit relevant *in vivo* activity. There were to be 6 active and 2 placebo subjects in Study Groups 1B and 1D and only 6 active subjects in each of Study Groups 1A, 1C, and [Glu$^{22,25}$, Leu$^{23,28,31}$, Aib$^{29}$, Lys$^{26,30}$]hPTHrP(1-34)NH$_2$ for Injection 80 $\mu$g (1E). Pooled placebo treatments from 2 [Glu$^{22,25}$, Leu$^{23,28,31}$, Aib$^{29}$, Lys$^{26,30}$]hPTHrP(1-34)NH$_2$ coated LCP array groups (1B and ID) were to serve as control for the safety evaluation.

Study Period 2 was to examine a 50% higher [Glu$^{22,25}$, Leu$^{23,28,31}$, Aib$^{29}$, Lys$^{26,30}$]hPTHrP(1-34)NH$_2$ coated LCP array dose (150 $\mu$g) in 2 of 3 study groups. Six (6) subjects in Study Group 2A were to receive [Glu$^{22,25}$, Leu$^{23,28,31}$, Aib$^{29}$, Lys$^{26,30}$]hPTHrP(1-34)NH$_2$ coated LCP array and 8 subjects in Study Group 2B were to receive [Glu$^{22,25}$, Leu$^{23,28,31}$, Aib$^{29}$, Lys$^{26,30}$]hPTHrP(1-34)NH$_2$ coated LCP array and [Glu$^{22,25}$, Leu$^{23,28,31}$, Aib$^{29}$, Lys$^{26,30}$]hP-THrP(1-34)NH$_2$ coated LCP array -Placebo for 7 consecutive days with a 6:2 allocation (randomized, double blind). The major goal was to compare 2 different wear times (10 seconds and 15 minutes, administered into the periumbilical region) following single (Day 1) and 7 consecutive days of [Glu$^{22,25}$, Leu$^{23,28,31}$, Aib$^{29}$, Lys$^{26,30}$]hPTHrP(1-34)NH$_2$ coated LCP array multiple dosing. Additionally, the relative bioavailability of [Glu$^{22,25}$, Leu$^{23,28,31}$, Aib$^{29}$, Lys$^{26,30}$]hP-THrP(1-34)NH$_2$ coated LCP array following Study Groups 2A and 2B was to be compared to [Glu$^{22,25}$, Leu$^{23,28,31}$, Aib$^{29}$, Lys$^{26,30}$]hPTHrP(1-34)NH$_2$ for Injection 80 $\mu$g (Study Group IE, Study Period 1). A third group [Study Group 2C] involved the [Glu$^{22,25}$, Leu$^{23,28,31}$, Aib$^{29}$, Lys$^{26,30}$]hPTHrP(1-34)NH$_2$ coated LCP array 100 $\mu$g dose (as in Study Period 1) with 6 subjects receiving [Glu$^{22,25}$, Leu$^{23,28,31}$, Aib$^{29}$, Lys$^{26,30}$]hPTHrP(1-34)NH$_2$ coated LCP array dose with a 15 minute wear time for 7 consecutive days.

In Study Period 3, the same 150 $\mu$g [Glu$^{22,25}$, Leu$^{23,28,31}$, Aib$^{29}$, Lys$^{26,30}$]hPTHrP(1-34)NH$_2$ coated LCP array dose as in Study Period 2 (Study Groups 2A and 2B) was further investigated but was administered in the upper thigh region (instead of periumbilical region) with varying wear times. The main goal was to describe the effect of application site and varying wear times on relative bioavailability of [Glu$^{22,25}$, Leu$^{23,28,31}$, Aib$^{29}$, Lys$^{26,30}$]hPTHrP(1-34)NH$_2$ coated LCP array compared to [Glu$^{22,25}$, Leu$^{23,28,31}$, Aib$^{29}$, Lys$^{26,30}$]hPTHrP(1-34)NH$_2$ for Injection 80 $\mu$g (Study Group IE, Study Period 1). Moreover, the effect of varying wear times was to be compared. Subjects in Study Period 3 (Study Group 3A [N = 6] and Study Group 3B [N = 6]) were to receive 150 $\mu$g [Glu$^{22,25}$, Leu$^{23,28,31}$, Aib$^{29}$, Lys$^{26,30}$]hP-THrP(1-34)NH$_2$ coated LCP array doses with 5 and 1 minute wear times, respectively on Days 1 through 6 and 30 second and 60 minute wear times, respectively, on Day 7. The 8 subjects in Study Group 3C were to receive [Glu$^{22,25}$, Leu$^{23,28,31}$, Aib$^{29}$, Lys$^{26,30}$]hPTHrP(1-34)NH$_2$ coated LCP array and [Glu$^{22,25}$, Leu$^{23,28,31}$, Aib$^{29}$, Lys$^{26,30}$]hP-THrP(1-34)NH$_2$ coated LCP array-Placebo with a 6:2 allocation (randomized, double blind) with a 24 hour wear time on Day 1. All 8 subjects were to receive [Glu$^{22,25}$, Leu$^{23,28,31}$, Aib$^{29}$, Lys$^{26,30}$]hPTHrP(1-34)NH$_2$ coated LCP array-Placebo on Days 2, 3, 4, 5, and 6 with 60, 15, 5, and 1 minute, and 30 second wear times, respectively. All 8 subjects were to receive 150 $\mu$g [Glu$^{22,25}$, Leu$^{23,28,31}$, Aib$^{29}$, Lys$^{26,30}$]hPTHrP(1-34)NH$_2$ coated LCP array doses on Day 7 with a 15 minute wear time.

Subjects were to meet all of the following inclusion criteria to be eligible to participate in this study.

1. The subject was to be a healthy postmenopausal woman from 50 to 80 years of age, inclusive. For the purposes of this study, postmenopausal was defined as $\geq$ 24 months of spontaneous amenorrhea (not relating to eating disorders or other causes), $\geq$ 6 months of spontaneous amenorrhea with serum follicle-stimulating hormone (FSH) levels $\geq$ 40 mIU/mL, or 6 weeks postsurgical bilateral oophorectomy with or without hysterectomy.
2. In the opinion of the Principal Investigator, the subject was to be in good general health as determined by medical history and physical examination (including vital signs) and without evidence of clinically significant abnormality.

3. The subject was to have a hemoglobin value > 12.0 g/dL during the screening period.

4. The subject was to have a serum phosphorus, PTH(1-84), and a serum total calcium within the normal range during the screening period.

5. The subject was to have a normal serum alkaline phosphatase (ALP) during the screening visit or, if abnormal but not clinically significant, a normal serum bone-specific ALP.

6. The subject was to have a 25-hydroxyvitamin D of > 20 ng/mL.

7. In the opinion of the Principal Investigator, the subject was to have all other screening and baseline clinical laboratory tests without any clinically significant abnormality.

8. The resting 12-lead ECG obtained during screening was to show no clinically significant abnormality of the following intervals: PR: $\geq 120$ and $\leq 220$ msec; QRS $\leq 120$ msec; QTc (Bazett's correction) $\leq 470$ msec. Incomplete right bundle branch block (IRBBB) and left anterior hemiblock (LAH) were acceptable.

9. The subject's systolic blood pressure (SBP) was to be $\geq 100$ and $\leq 155$ mmHg, diastolic blood pressure (DBP) was to be $\geq 40$ and $\leq 95$ mmHg, and heart rate was to be $\geq 45$ and $\leq 90$ bpm during screening.

10. The subject was to weigh at least 120 pounds (54.5 kg) and was to be within -25% and +30% of her ideal body weight (at screening) based on height and body frame.

11. The subject was to read, understand, and sign the written ICF.

Subjects who met any of the following exclusion criteria were not eligible to participate in the study.

General exclusion criteria:

1. The subject had a history of clinically significant chronic or recurrent renal, hepatic, pulmonary, allergic, cardiovascular, gastrointestinal, endocrine, CNS, hematologic or metabolic diseases, or immunologic, emotional, and/or psychiatric disturbances.

2. The subject was diagnosed with osteoporosis, Paget's disease, or other metabolic bone diseases (e.g., vitamin D deficiency or osteomalacia) or was to a non-traumatic fracture that occurred within 1 year prior to the initial screening visit.

3. The subject had a history of urolithiasis within the past 5 years.

4. The subject had a history of gout or a uric acid value > 7.5 mg/dL during the screening period.

5. The subject had a decrease of 20 mmHg or more in SBP or 10 mmHg or more in DBP from supine to standing (5 minutes lying and 3 minutes standing) and/or any symptomatic hypotension.

6. The subject had an acute illness which, in the opinion of the Principal Investigator, could have posed a threat or harm to the subject or obscure laboratory test results or interpretation of study data.

7. The subject had donated blood, had a blood loss of more than 50 mL within 8 weeks prior to study Day 1, or had a plasma donation (apheresis) within 7 days prior to Day 1.

8. The subject was known to be positive for hepatitis B, hepatitis C, human immunodeficiency virus (HIV)-1 or HIV-2 or had positive results at screening for hepatitis B surface antigen (HBsAg), hepatitis C antibody (HCV-Ab), or HIV.

9. The subject had been previously randomized, dosed, and discontinued in this study for any reason.

Medication related exclusion criteria:

10. The subject had a known history of hypersensitivity to any of the test materials or related compounds.

11. The subject used any medication on a chronic basis, including bisphosphonates and estrogens or estrogen derivatives.

12. The subject received any medication, including over-the-counter (OTC), non-prescription preparations or herbal or homeopathic supplements, within 72 hours prior to administration of the first dose of study medication.

13. The subject received a general anesthetic or an investigational product other than [Glu$^{22,25}$, Leu$^{23,28,31}$, Aib$^{29}$, Lys$^{26,30}$]hPTHrP(1-34)NH$_2$ within 90 days prior to the initial dose of study medication.

14. Unwillingness or inability to understand study procedures or commitments as judged by the Principal Investigator.

Lifestyle related exclusion criteria:

15. The subject had an abnormal nutritional status (abnormal diets, excessive or unusual vitamin intakes, malabsorption, or significant recent weight change).

16. The subject smoked more than 10 cigarettes per day. Subjects were not allowed to consume any nicotine-containing products while they were confined to the clinical facility.

17. The subject had a history of alcohol abuse, illegal drug use, or drug abuse within 24 months of the screening visit.

18. The subject had a positive urine drug/alcohol screen.

Subjects were informed that they had the right to withdraw from the study at any time for any reason, without prejudice to their medical care. The Principal Investigator also had the right to withdraw subjects from the study for any of the following reasons:

- Adverse events
- Refusal of treatment
- Subject request
- Inability to complete study procedures
- Lost to follow-up
- Non-compliance
- Administrative reasons

If a subject was withdrawn or discontinued from the study, the reason for withdrawal from the study was to be recorded in the source documents and on the case report form (CRF). All subjects withdrawn prior to completing the study were to be encouraged to complete the postdose study evaluation scheduled for the study group. All AEs were to be followed to resolution.

Subjects who withdrew from the study for administrative reasons after study medication had been administered may have been replaced at the discretion of the Principal Investigator after consultation with the Medical Monitor.

According to the study protocol, the term study group will be used instead of treatment in the tables, figures, and the text of the report.

The [Glu$^{22,25}$, Leu$^{23,28,31}$, Aib$^{29}$, Lys$^{26,30}$]hPTHrP(1-34)NH$_2$ coated LCP array ([Glu$^{22,25}$, Leu$^{23,28,31}$, Aib$^{29}$, Lys$^{26,30}$]hPTHrP(1-34)NH$_2$ coated LCP array, 100, 150, and 200 $\mu$g) ([Glu$^{22,25}$, Leu$^{23,28,31}$, Aib$^{29}$, Lys$^{26,30}$]hP-THrP(1-34)NH$_2$ coated LCP array) was to be supplied in an enclosed collar assembly for loading onto a spring loaded applicator.

The phosphate buffered saline (PBS)-coated array (Placebo-array) was to be similarly supplied in an enclosed collar assembly for loading onto a spring loaded applicator.

[Glu$^{22,25}$, Leu$^{23,28,31}$, Aib$^{29}$, Lys$^{26,30}$]hPTHrP(1-34)NH$_2$ for Injection 80 $\mu$g was to be supplied as a multi-dose cartridge (1.5 mL) containing 2 mg/mL [Glu$^{22,25}$, Leu$^{23,28,31}$, Aib$^{29}$, Lys$^{26,30}$]hPTHrP(1-34)NH$_2$ (free base) in 5 mg/mL tri-hydrate sodium acetate and 5 mg/mL of phenol (preservative) adjusted at pH 5.1 with acetic acid.

The pen injector is a modified version of the Becton Dickinson Pen II device and has been validated for use with [Glu$^{22,25}$, Leu$^{23,28,31}$, Aib$^{29}$, Lys$^{26,30}$]hPTHrP(1-34)NH$_2$ in its prefilled cartridge.

**Study Period 1**

Study Group 1A = 1 x 100 $\mu$g [Glu$^{22,25}$, Leu$^{23,28,31}$, Aib$^{29}$, Lys$^{26,30}$]hPTHrP(1-34)NH$_2$ -sMTS was to be administered into the periumbilical region via a TD delivery system (TD microarray) with 10 second wear time.

Study Group 1B = 1 x 100 $\mu$g [Glu$^{22,25}$, Leu$^{23,28,31}$, Aib$^{29}$, Lys$^{26,30}$]hPTHrP(1-34)NH$_2$ -sMTS was to be administered into the periumbilical region via a TD delivery system (TD microarray) with 15 minute wear time.

Study Group 1C = 1 x 100 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 10 second wear time.

Study Group ID = 1 x 100 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 15 minute wear time.

Study Group IE = 1 x 80 $\mu$g [Glu$^{22,25}$, Leu$^{23,28,31}$, Aib$^{29}$, Lys$^{26,30}$]hPTHrP(1-34)NH$_2$ was to be administered into the periumbilical region in a single SC injection.

Placebo = placebo was to be administered into the periumbilical/upper thigh region via a TD delivery system (TD microarray) with 15 minute wear time.

**Study Period 2**

Study Group 2A = 1 x 150 $\mu$g [Glu$^{22,25}$, Leu$^{23,28,31}$, Aib$^{29}$, Lys$^{26,30}$]hPTHrP(1-34)NH$_2$ -sMTS was to be administered into the periumbilical region via a TD delivery system (TD microarray) with 10 second wear time daily for 7 days.

Study Group 2B = 1 x 150 $\mu$g [Glu$^{22,25}$, Leu$^{23,28,31}$, Aib$^{29}$, Lys$^{26,30}$]hPTHrP(1-34)NH$_2$ -sMTS was to be administered into the periumbilical region via a TD delivery system (TD microarray) with 15 minute wear time daily for 7 days.

Study Group 2C = 1 x 100 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 15 minute wear time daily for 7 days.

Placebo = placebo was to be administered into the periumbilical region via a TD delivery system (TD microarray) with 15 minute wear time daily for 7 days.

**Study Period 3**

Study Group 3A = 1 x 150 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 5 minute wear time on Days 1 through 6 and 30 second wear time on Day 7.

Study Group 3B = 1 x 150 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 1 minute wear time on Days 1 through 6 and 60 minute wear time on Day 7.

Study Group 3C = 1 x 150 $\mu$g [Glu$^{22,25}$, Leu$^{23,28,31}$, Aib$^{29}$, Lys$^{26,30}$]hPTHrP(1-34)NH$_2$ -sMTS was to be administered into the upper thigh region via a TD delivery system (TD microarray) with 24 hour wear time on Day 1 and 15 minute wear time on Day 7.

Placebo = placebo was to be administered into the upper thigh region via a TD delivery system (TD microarray) with 24 hour wear time on Day 1 and 60, 15, 5, 1 minute, and 30 second wear times on Days 2, 3, 4, 5, 6, respectively.

**Method of Assigning Patients to Treatment Groups**

The study employed a double randomization procedure. A specific study group was assigned to subjects according to the subject number and randomization code. This assignment was not blinded. Secondly, the subject was assigned to active drug versus placebo and this assignment was double blind.

A total of 34 subjects planned for Study Period 1 were assigned to 5 study groups. The study groups included 4 groups (1A, 1B, 1C, and ID) who received [Glu$^{22,25}$, Leu$^{23,28,31}$, Aib$^{29}$, Lys$^{26,30}$]hPTHrP(1-34)NH$_2$ -sMTS 100 $\mu$g with varying wear times (10 seconds and 15 minutes) and applications sites (periumbilical and upper thigh). Six subjects were randomly assigned to each of Study Groups 1A and 1C and 8 subjects were randomly assigned to each of Study Groups 1B and ID. While 6 subjects in each of Study Groups 1B and 1D were randomized to receive [Glu$^{22,25}$, Leu$^{23,28,31}$, Aib$^{29}$, Lys$^{26,30}$]hPTHrP(1-34)NH$_2$-sMTS 100 $\mu$g, 2 subjects in each group were randomly assigned to receive [Glu$^{22,25}$, Leu$^{23,28,31}$, Aib$^{29}$, Lys$^{26,30}$]hPTHrP(1-34)NH$_2$ -sMTS-Placebo. A fifth group (Study Group IE, N = 6) was randomized to receive [Glu$^{22,25}$, Leu$^{23,28,31}$, Aib$^{29}$, Lys$^{26,30}$]hPTHrP(1-34)NH$_2$ for Injection 80 $\mu$g.

Twenty (20) subjects planned for Study Period 2 were randomly assigned to 3 study groups. Six (6) subjects were assigned to Study Group 2A to receive [Glu$^{22,25}$, Leu$^{23,28,31}$, Aib$^{29}$, Lys$^{26,30}$]hPTHrP(1-34)NH$_2$ -sMTS 150 $\mu$g in the periumbilical region with a 10 second wear time for 7 consecutive days. Eight (8) subjects were assigned to Study Group 2B, 6 subjects received [Glu$^{22,25}$, Leu$^{23,28,31}$, Aib$^{29}$, Lys$^{26,30}$]hPTHrP(1-34)NH$_2$ -sMTS 150 $\mu$g in the periumbilical region with a 15 minute wear time and 2 subjects received a corresponding [Glu$^{22,25}$, Leu$^{23,28,31}$, Aib$^{29}$, Lys$^{26,30}$]hP-THrP(1-34)NH$_2$ -sMTS-Placebo for 7 consecutive days. Six (6) subjects were assigned to Study Group 2C to receive [Glu$^{22,25}$, Leu$^{23,28,31}$, Aib$^{29}$, Lys$^{26,30}$]hPTHrP(1-34)NH$_2$ -sMTS 100 $\mu$g in the upper thigh with a 15 minute wear time for 7 consecutive days.

Twenty (20) subjects planned for Study Period 3 were randomly assigned to 3 study groups. Six (6) subjects were assigned to Study Group 3A to receive [Glu$^{22,25}$, Leu$^{23,28,31}$, Aib$^{29}$, Lys$^{26,30}$]hPTHrP(1-34)NH$_2$ -sMTS 150 $\mu$g in the upper thigh for 7 consecutive days with a 5 minute wear time (Days 1 - 6) and a 30 second wear time (Day 7). Six (6) subjects were assigned to Study Group 3B to receive [Glu$^{22,25}$, Leu$^{23,28,31}$, Aib$^{29}$, Lys$^{26,30}$]hPTHrP(1-34)NH$_2$ -sMTS 150 $\mu$g in the upper thigh for 7 consecutive days with a 1 minute wear time (Days 1 - 6) and 60 minute wear time (Day 7). Subjects in Study Group 3C (N = 8) were randomized to receive 1 application of [Glu$^{22,25}$, Leu$^{23,28,31}$, Aib$^{29}$, Lys$^{26,30}$]hPTHrP(1-34)NH$_2$ -sMTS 150 $\mu$g (N = 6) or [Glu$^{22,25}$, Leu$^{23,28,31}$, Aib$^{29}$, Lys$^{26,30}$]hPTHrP(1-34)NH$_2$ -sMTS-Placebo (N = 2) with a wear time of 24 hours administered to the upper thigh on Day 1. These 8 subjects subsequently received 5 consecutive days of [Glu$^{22,25}$, Leu$^{23,28,31}$, Aib$^{29}$, Lys$^{26,30}$]hPTHrP(1-34)NH$_2$ -sMTS-Placebo application administered to the upper thigh over a range of wear times (60, 15, 5, 1 minutes and 30 seconds on Days 2 - 6, respectively), followed by a single dose of [Glu$^{22,25}$, Leu$^{23,28,31}$, Aib$^{29}$, Lys$^{26,30}$]hPTHrP(1-34)NH$_2$ -sMTS 150 $\mu$g (N = 8) with a wear time of 15 minutes applied to the upper thigh on Day 7.

Study Periods 1 and 2 were separated by approximately 28 days to allow for a safety review, analysis of PK samples, and calculation of bioavailability. Study Periods 2 and 3 were separated by an approximately 7-day interval to review safety. New subjects were to be enrolled for each period. All study subjects for Study Periods 2 and 3 had a maximum of 7 study drug administrations.

**Selection of Doses in the Study**

[Glu$^{22,25}$, Leu$^{23,28,31}$, Aib$^{29}$, Lys$^{26,30}$]hPTHrP(1-34)NH$_2$ -sMTS Active and [Glu$^{22,25}$, Leu$^{23,28,31}$, Aib$^{29}$, Lys$^{26,30}$]hP-THrP(1-34)NH$_2$ -sMTS-Placebo

[Glu$^{22,25}$, Leu$^{23,28,31}$, Aib$^{29}$, Lys$^{26,30}$]hPTHrP(1-34)NH$_2$ -sMTS

The [Glu$^{22,25}$, Leu$^{23,28,31}$, Aib$^{29}$, Lys$^{26,30}$]hPTHrP(1-34)NH$_2$ -coated sMTS microneedle array was enclosed in a collar assembly for loading onto a spring loaded applicator. The [Glu$^{22,25}$, Leu$^{23,28,31}$, Aib$^{29}$, Lys$^{26,30}$]hPTHrP(1-34)NH$_2$ -sMTS was removed from refrigeration 1 hour prior to application and was loaded onto the applicator by the pharmacist or qualified study personnel for subject dosing. Each [Glu$^{22,25}$, Leu$^{23,28,31}$, Aib$^{29}$, Lys$^{26,30}$]hPTHrP(1-34)NH$_2$ -sMTS was coated with 100 or 150 $\mu$G[Glu$^{22,25}$, Leu$^{23,28,31}$, Aib$^{29}$, Lys$^{26,30}$]hPTHrP(1-34)NH$_2$.

[Glu$^{22,25}$, Leu$^{23,28,31}$, Aib$^{29}$, Lys$^{26,30}$]hPTHrP(1-34)NH$_2$ -sMTS-Placebo

The PBS was formulated as a placebo for TD administration using an sMTS. The PBS-coated sMTS (Placebo-sMTS) was enclosed in a collar assembly for loading onto a spring loaded applicator. The Placebo-sMTS was removed from refrigeration 1 hour prior to application and was loaded onto the applicator by qualified study personnel for subject dosing.

[Glu$^{22,25}$, Leu$^{23,28,31}$, Aib$^{29}$, Lys$^{26,30}$]hPTHrP(1-34)NH$_2$ for Injection 80 $\mu$g

Each multi-dose cartridge contained 2 mg/mL [Glu$^{22,25}$, Leu$^{23,28,31}$, Aib$^{29}$, Lys$^{26,30}$]hPTHrP(1-34)NH$_2$ (free base) in 5 mg/mL tri-hydrate sodium acetate and 5 mg/mL of phenol (preservative) adjusted at pH 5.1 with acetic acid. [Glu$^{22,25}$, Leu$^{23,28,31}$, Aib$^{29}$, Lys$^{26,30}$]hPTHrP(1-34)NH$_2$ for Injection 80 $\mu$g was supplied as a liquid in a 1.5 mL Type 1 glass cartridge and was stored refrigerated at 5 $\pm$ 3°C. The multi-dose cartridge was designed to deliver a dose of 80 $\mu$g of [Glu$^{22,25}$, Leu$^{23,28,31}$, Aib$^{29}$, Lys$^{26,30}$]hPTHrP(1-34)NH$_2$ in 40 mL of fluid when inserted into the pen injector device (BD Pen II). The multi-dose cartridge was designed to deliver a dose of 80 $\mu$g of [Glu$^{22,25}$, Leu$^{23,28,31}$, Aib$^{29}$, Lys$^{26,30}$]hP-THrP(1-34)NH$_2$ in 40 mL of fluid when inserted into the pen injector device (BD Pen II). The 80 $\mu$g cartridge was removed from refrigeration 1 hour prior to application.

**Selection and Timing of Dose for Each Patient**

In Study Period 1, 34 subjects were randomized into 1 of 5 study groups of varying application sites and wear times for [Glu$^{22,25}$, Leu$^{23,28,31}$, Aib$^{29}$, Lys$^{26,30}$]hPTHrP(1-34)NH$_2$-sMTS 100 $\mu$g, or into a study group that received [Glu$^{22,25}$, Leu$^{23,28,31}$, Aib$^{29}$, Lys$^{26,30}$]hPTHrP(1-34)NH$_2$ for Injection 80 $\mu$g. The application sites were in the periumbilical region and the upper anterior thigh and the wear times for [Glu$^{22,25}$, Leu$^{23,28,31}$, Aib$^{29}$, Lys$^{26,30}$]hPTHrP(1-34)NH$_2$-sMTS 100 $\mu$g were 10 seconds and 15 minutes. For all subjects in this period randomized to the Glu$^{22,25}$, Leu$^{23,28,31}$, Aib$^{29}$, Lys$^{26,30}$]hPTHrP(1-34)NH$_2$-sMTS groups, there was a single application and the dose of Glu$^{22,25}$, Leu$^{23,28,31}$, Aib$^{29}$, Lys$^{26,30}$]hPTHrP(1-34)NH$_2$-sMTS was to remain constant at 100 $\mu$g. In Study Group 1A, 6 subjects were administered Glu$^{22,25}$, Leu$^{23,28,31}$, Aib$^{29}$, Lys$^{26,30}$]hPTHrP(1-34)NH$_2$-sMTS 100 $\mu$g in the periumbilical region for 10 seconds. In Study Group 1B, 6 subjects were randomized to receive Glu$^{22,25}$, Leu$^{23,28,31}$, Aib$^{29}$, Lys$^{26,30}$]hPTHrP(1-34)NH$_2$-sMTS 100 $\mu$g applied in the periumbilical region for 15 minutes and 2 subjects received a corresponding sMTS-Placebo, also administered in the periumbilical region for 15 minutes. In Study Group 1C, 6 subjects were administered Glu$^{22,25}$, Leu$^{23,28,31}$, Aib$^{29}$, Lys$^{26,30}$]hPTHrP(1-34)NH$_2$ 100 $\mu$g in the upper thigh for 10 seconds. In Study Group ID, 6 subjects were randomized to receive Glu$^{22,25}$, Leu$^{23,28,31}$, Aib$^{29}$, Lys$^{26,30}$]hPTHrP(1-34)NH$_2$-sMTS 100 $\mu$g applied to the upper thigh for 15 minutes and 2 subjects received a corresponding Glu$^{22,25}$, Leu$^{23,28,31}$, Aib$^{29}$, Lys$^{26,30}$]hPTHrP(1-34)NH$_2$-sMTS-Placebo, also administered in the upper thigh for 15 minutes. In addition, 6 subjects were to receive Glu$^{22,25}$, Leu$^{23,28,31}$, Aib$^{29}$, Lys$^{26,30}$]hPTHrP(1-34)NH$_2$ for Injection 80 $\mu$g, administered SC (Study Group 1E) into the periumbilical region.

Prior to proceeding to the next dose, safety and tolerability data from subjects enrolled in earlier periods were reviewed for suitability to escalate to the next higher dose. If the bioavailability of the single-dose administration of Glu$^{22,25}$, Leu$^{23,28,31}$, Aib$^{29}$, Lys$^{26,30}$]hPTHrP(1-34)NH$_2$ sMTS 100 $\mu$g was greater than 66% of the SC 80 $\mu$g dose in Study Period 1, the 150 $\mu$g dose was not administered.

In Study Period 2, 20 subjects were dosed once daily for 7 consecutive days with Glu$^{22,25}$, Leu$^{23,28,31}$, Aib$^{29}$, Lys$^{26,30}$]hPTHrP(1-34)NH$_2$-sMTS 100 or 150 $\mu$g. In Study Group 2A, 6 subjects were randomized to receive Glu$^{22,25}$, Leu$^{23,28,31}$, Aib$^{29}$, Lys$^{26,30}$]hPTHrP(1-34)NH$_2$-sMTS 150 $\mu$g applied to the periumbilical region with a wear time of 10 seconds. In Study Group 2B, 6 subjects were randomized to receive Glu$^{22,25}$, Leu$^{23,28,31}$, Aib$^{29}$, Lys$^{26,30}$]hP-THrP(1-34)NH$_2$-sMTS 150 $\mu$g applied to the periumbilical region for 15 minutes and 2 subjects received a corresponding

Glu$^{22,25}$, Leu$^{23,28,31}$, Aib$^{29}$, Lys$^{26,30}$]hPTHrP(1-34)NH$_2$-sMTS-Placebo, also administered in the periumbilical region for 15 minutes. In addition, 6 subjects in Study Group 2C were to be randomly assigned to receive Glu$^{22,25}$, Leu$^{23,28,31}$, Aib$^{29}$, Lys$^{26,30}$]hPTHrP(1-34)NH$_2$-sMTS 100 $\mu$g at an application site (either periumbilical or upper thigh) and for a wear time (either 10 seconds or 15 minutes) to be determined by the PK results obtained from Study Period 1.

Prior to proceeding to the next dose, safety and tolerability from subjects enrolled in earlier periods were reviewed for suitability to escalate to the next higher dose. If the bioavailability of the single-dose administration of Glu$^{22,25}$, Leu$^{23,28,31}$, Aib$^{29}$, Lys$^{26,30}$]hPTHrP(1-34)NH$_2$-sMTS 100 $\mu$g was greater than 50% of the SC 80 $\mu$g dose in Study Period 1, the 200 $\mu$g dose was not to be administered.

Protocol Amendment 4 was enacted to conduct a time-course study to optimize the duration of Glu$^{22,25}$, Leu$^{23,28,31}$, Aib$^{29}$, Lys$^{26,30}$]hPTHrP(1-34)NH$_2$-sMTS application within the Glu$^{22,25}$, Leu$^{23,28,31}$, Lys$^{26,30}$]hP-THrP(1-34)NH$_2$-sMTS 150 $\mu$g dose groups.

Study Period 3 was to dose a total of 20 subjects. Subjects randomized to Study Group 3A (N = 6) were to receive Glu$^{22,25}$, Leu$^{23,28,31}$, Aib$^{29}$, Lys$^{26,30}$]hPTHrP(1-34)NH$_2$ 150 $\mu$g administered in the upper thigh with a wear time of 5 minutes for 6 consecutive days, followed by a single administration of Glu$^{22,25}$, Leu$^{23,28,31}$, Aib$^{29}$, Lys$^{26,30}$]hP-THrP(1-34)NH$_2$ 150 $\mu$g with a wear time of 30 seconds, also applied to the upper thigh on Day 7. Subjects randomized to Study Group 3B (N = 6) were to receive Glu$^{22,25}$, Leu$^{23,28,31}$, Aib$^{29}$, Lys$^{26,30}$]hPTHrP(1-34)NH$_2$-sMTS 150 $\mu$g administered to the upper thigh with a wear time of 1 minute for 6 consecutive days followed by a single administration of Glu$^{22,25}$, Leu$^{23,28,31}$, Aib$^{29}$, Lys$^{26,30}$]hPTHrP(1-34)NH$_2$-sMTS 150 $\mu$g with a wear time of 60 minutes, also applied to the upper thigh on Day 7. Subjects randomized to Study Group 3C (N = 8) received 1 application of Glu$^{22,25}$, Leu$^{23,28,31}$, Aib$^{29}$, Lys$^{26,30}$]hPTHrP(1-34)NH$_2$-sMTS 150 $\mu$g (N = 6) or sMTS-Placebo (N = 2) with a wear time of 24 hours applied to the upper thigh on Day 1. These 8 subjects subsequently received 5 consecutive days of placebo application over a range of wear times (30 seconds and 1, 5, 15, and 60 minutes), followed by a single dose of Glu$^{22,25}$, Leu$^{23,28,31}$, Aib$^{29}$, Lys$^{26,30}$]hPTHrP(1-34)NH$_2$-sMTS 150 $\mu$g (N = 8) with a wear time of 15 minutes applied to the upper thigh on Day 7.

**Prior and Concomitant Therapy**

Vitamin D ($\leq$ 800 IU/day), calcium supplements ($\leq$ 1000 mg/day), and low-dose aspirin ($\leq$ 81 mg/daily for prophylaxis of cardiovascular disease) were acceptable as long as the subject had been on a stable dose for 1 month prior to the initial screening visit and remained on the same dose(s) throughout the study. Thyroid replacement therapy was allowed if the subject had been on a stable dose for at least 6 months and remained on the same dose throughout the study. Statins for lowering blood cholesterol levels were allowed as long as the subject had been on a stable dose for at least 3 months and remained on the same dose throughout the study.

Subjects were not to take any other medications, including OTC medications, herbal medications, or mega-doses of vitamins during the study without prior approval of the Principal Investigator. The occasional use of OTC medications (e.g., ibuprofen or acetaminophen) for headache or minor discomfort was allowed if discussed with the Principal Investigator and recorded in the CRF.

If it became necessary for a subject to take any other medication during the study, the specific medication(s) and indication(s) were to be discussed with the Principal Investigator. All concomitant medications taken during the course of the study were to be recorded in the source documents and transcribed into the subject's CRF.

In addition, subjects were ineligible for the study if they received general anesthesia within the past 3 months, received an investigational drug within 90 days prior to the initial dose of study medication, took any medications on a chronic basis, or had an abnormal nutritional status (abnormal diets, excessive or unusual vitamin intakes, or malabsorption).

**Treatment Compliance**

In order to evaluate the safety, tolerability, and PK of the study drug, it was critical that subjects received each dose of study medication as directed. The date and time that each dose of study drug was administered was to be recorded. All doses of study medication were to be administered at the clinical facility by qualified personnel under direct observation.

If a subject did not wear the microarray for the intended duration or take all study medication, the reason for the missed dosing was to be recorded on the CRF and in the source documents.

**Primary Pharmacokinetic and Pharmacodynamic Parameters**

**Pharmacokinetics**

The following PK parameters were to be calculated from individual plasma concentration-time Glu$^{22,25}$,

Leu[23,28,31], Aib[29], Lys[26,30]]hPTHrP(1-34)NH$_2$ data based on actual time using noncompartmental methods using Win-Nonlin Version 5.0.1 and SAS® Version 9.1:

## Study Periods 1, 2, and 3 - Day 1 (Single Dose)

| | |
|---|---|
| AUC$_{0-t}$ | Area under the drug concentration-time curve, calculated using linear trapezoidal summation from time zero to time t, where t was the time of the last measurable concentration ($C_t$). |
| AUC$_{0-\infty}$ | Area under the drug concentration-time curve from time zero to infinity. AUC$_{0-\infty}$ = AUC$_{0-t}$ + $C_t/\lambda_z$, where $\lambda_z$ was the terminal elimination rate constant. The parameter was be displayed as AUC$_{0-inf}$ in SAS. |
| AUCR | Ratio of AUC$_{0-t}$ to AUC$_{0-\infty}$ |
| C$_{max}$ | Maximum observed drug concentration |
| T$_{max}$ | Time of the observed maximum drug concentration |
| T$_{last}$ | Time of the last quantifiable drug concentration |
| $\lambda_z$ | Apparent elimination rate constant, estimated by linear regression of the terminal linear portion of the log concentration versus time curve. The parameter was displayed as Lambda_z in SAS. |
| t$_{1/2}$ | Apparent elimination half-life, calculated as ln(2)/$\lambda_z$ |
| CL/F | Apparent clearance, calculated as Dose/AUC$_{0-\infty}$ |
| V$_d$/F | Apparent volume of distribution (V$_d$/F), calculated as CL/F/$\lambda_z$ |
| Relative bioavailability (F$_{rel}$) | Relative bioavailability was to be calculated as the ratio of dose normalized AUC$_{0-\infty}$ values: [AUC$_{0-\infty}$ (transdermal)/Dose(transdermal)]/[Mean AUC$_{0-\infty}$ (SC)/Dose(SC)]-Study Periods 1, 2, and 3 - Day 1. |

## Study Periods 2 and 3 - Day 7 (Multiple Dose)

In addition to the above parameters (except AUC$_{0-\infty}$), the following PK parameters were to be computed using the same method:

| | |
|---|---|
| AUC$_{0-\tau}$ | Area under the drug concentration-time curve, calculated using linear trapezoidal summation from time zero to time $\tau$, where t was the dosing interval (24 hr). |
| AR$_1$ | Accumulation ratio (AR$_1$), calculated as C$_{max}$, Day 7/C$_{max}$, Day 1 - Study Period 2 (2a, 2b, and 2c) only. |
| AR$_2$ | Accumulation ratio (AR$_2$), calculated as AUC$_{0-\tau}$, Day 7/AUC$_{0-\tau}$, Day 1 - Study Period 2 (2a, 2b, and 2c) only. |
| LF | Linearity factor (LF) = AUC$_{0-\tau}$ Day 7/AUC$_{0-\infty}$ Day 1 - Study Period 2 (2a, 2b, and 2c) |

Moreover, CL$_{ss}$/F and V$_{ss}$/F were to be calculated following multiple dosing for Day 7, wherever applicable but were to be presented as CL/F and V$_d$/F, respectively.
The following footnotes were to be added, wherever applicable, on Day 7 PK parameter tables.

CL/F following multiple dosing was computed as Dose/AUC$_{0-\tau}$

Vd/F following multiple dosing was computed as MRT$_\infty$*CL$_{ss}$

## Pharmacodynamics

The following PD parameters were to be computed for total serum calcium and serum phosphorus using SAS® Version 9.1:

**Study Periods 1, 2 and 3 - Days 1 and 7**

| Original/Change From Baseline Data | Parameter | Description |
|---|---|---|
| Original | $C_{min}/_{max}$ | Minimum and maximum observed serum concentration |
| Original | $T_{min}/_{max}$ | Time of the first occurrence of the minimum or maximum serum concentration |
| Change From Baseline | $\Delta_{max}$ | Maximum change from predose (0 hour). Note: Day 1 predose was to be used as baseline. The parameter was to be displayed as Delta Max in SAS. |
| Change From Baseline | $T_{max}$ | Time of maximum change from predose (0 hour). The parameter was to be displayed as $T_{max}$ in SAS. |
| Note: For 1,25-dihydroxyvitamin D, CTX, and P1NP, Day 1 predose was to be used to compute change from baseline for Days 3 and 7. | | |

**Table 37 Summary of Plasma Glu$^{22,25}$, Leu$^{23,28,31}$, Aib$^{29}$, Lys$^{26,30}$]hPTHrP(1-34)NH$_2$Pharmacokinetic Parameters Following Glu$^{22,25}$, Leu$^{23,28,31}$, Aib$^{29}$, Lys$^{26,30}$]hPTHrP(1-34)NH$_2$Study Groups and Glu$^{22,25}$, Leu$^{23,28,31}$, Aib$^{29}$, Lys$^{26,30}$]hPTHrP(1-34)NH$_2$ for Injection 80 $\mu$g (Study Period 1)**

| | Study Group 1A | Study Group 1B | Study Group 1C | Study Group 1D | Study Group 1E |
|---|---|---|---|---|---|
| Pharmacokinetic Parameters | Mean $\pm$ SD (N) | Mean $\pm$ SD (N) | Mean $\pm$ SD (N) | Mean $\pm$ SD (N) | Mean $\pm$ SD (N) |
| $C_{max}$ (pg/mL) | 292 $\pm$ 167 | 401 $\pm$ 212 | 303 $\pm$ 139 | 676 $\pm$ 257 | 452 $\pm$ 189 |
| | (6) | (6) | (6) | (6) | (6) |
| $T_{max}$ (hr) | 0.130 (0.0775, 0.164) | 0.163 (0.0856, 0.175) | 0.160 (0.0808, 0.166) | 0.163 (0.161, 0.170) | 0.422 (0.246, 1.01) |
| | (6) | (6) | (6) | (6) | (6) |
| $T_{last}$ (hr) | 1.42 $\pm$ 1.32 | 1.09 $\pm$ 0.363 | 1.26 $\pm$ 0.693 | 1.85 $\pm$ 0.701 | 3.51 $\pm$ 0.546 |
| | (6) | (6) | (6) | (6) | (6) |
| $AUC_{0-t}$ (pg*hr/mL) | 126 $\pm$ 89.9 | 132 $\pm$ 66.6 | 134 $\pm$ 105 | 247 $\pm$ 66.4 | 584 $\pm$ 219 |
| | (6) | (6) | (6) | (6) | (6) |
| $AUC_{0-inf}$ (pg*hr/mL) | 142.1 $\pm$ 101.7 | 142.4 $\pm$ 67.94 | 150.8 $\pm$ 116.4 | 268.9 $\pm$ 74.31 | 633.3 $\pm$ 226.2 |
| | (6) | (6) | (6) | (6) | (6) |
| $AUC_{0-tau}$ (pg*hr/mL) | 142.1 $\pm$ 101.6 | 142.4 $\pm$ 67.92 | 150.7 $\pm$ 116.3 | 268.8 $\pm$ 74.16 | 633.1 $\pm$ 226.0 |
| | (6) | (6) | (6) | (6) | (6) |
| $AUC_{0-inf}$/dose (pg*hr/mL/$\mu$g) | 1.42 $\pm$ 1.02 | 1.42 $\pm$ 0.679 | 1.51 $\pm$ 1.16 | 2.69 $\pm$ 0.743 | 7.92 $\pm$ 2.83 |
| | (6) | (6) | (6) | (6) | (6) |
| $AUC_{0-tau}$/dose (pg*hr/mL/$\mu$g) | 1.42 $\pm$ 1.02 | 1.42 $\pm$ 0.679 | 1.51 $\pm$ 1.16 | 2.69 $\pm$ 0.742 | 7.91 $\pm$ 2.83 |
| | (6) | (6) | (6) | (6) | (6) |
| $t_{1/2}$ (hr) | 0.466 $\pm$ 0.417 | 0.302 $\pm$ 0.0821 | 0.405 $\pm$ 0.196 | 0.571 $\pm$ 0.297 | 0.970 $\pm$ 0.185 |
| | (6) | (6) | (6) | (6) | (6) |
| Lambda_z (1/hr) | 2.38 $\pm$ 1.49 | 2.53 $\pm$ 1.05 | 2.29 $\pm$ 1.60 | 1.46 $\pm$ 0.621 | 0.744 $\pm$ 0.185 |

(continued)

| Pharmacokinetic Parameters | Study Group 1A Mean ± SD (N) | Study Group 1B Mean ± SD (N) | Study Group 1C Mean ± SD (N) | Study Group 1D Mean ± SD (N) | Study Group 1E Mean ± SD (N) |
|---|---|---|---|---|---|
|  | (6) | (6) | (6) | (6) | (6) |
| AUCR | 0.877 ± 0.0317 | 0.915 ± 0.0309 | 0.871 ± 0.0616 | 0.919 ± 0.0138 | 0.916 ± 0.0471 |
|  | (6) | (6) | (6) | (6) | (6) |
| CL/F (L/hr) | 1143 ± 999.5 | 869.5 ± 457.3 | 1114 ± 819.5 | 402.6 ± 138.0 | 140.6 ± 49.21 |
|  | (6) | (6) | (6) | (6) | (6) |
| Vd/F (L) | 491.1 ± 207.7 | 337.4 ± 96.41 | 467.3 ± 159.7 | 297.9 ± 86.25 | 197.0 ± 86.21 |
|  | (6) | (6) | (6) | (6) | (6) |

$T_{max}$ is presented as Median (Minimum, Maximum)

Study Group 1A: 1 x 100 $\mu$g Glu$^{22,25}$, Leu$^{23,28,31}$, Aib$^{29}$, Lys$^{26,30}$]hPTHrP(1-34)NH$_2$sMTS, 10 Second Wear Time (Periumbilical)

Study Group 1B: 1 x 100 $\mu$g Glu$^{22,25}$, Leu$^{23,28,31}$, Aib$^{29}$, Lys$^{26,30}$]hPTHrP(1-34)NH$_2$sMTS, 15 Minute Wear Time (Periumbilical)

Study Group 1C: 1 x 100 $\mu$g Glu$^{22,25}$, Leu$^{23,28,31}$, Aib$^{29}$, Lys$^{26,30}$]hPTHrP(1-34)NH$_2$ sMTS), 10 Second Wear Time (Upper Thigh)

Study Group 1D: 1 x 100 $\mu$g Glu$^{22,25}$, Leu$^{23,28,31}$, Aib$^{29}$, Lys$^{26,30}$]hPTHrP(1-34)NH$_2$ sMTS, 15 Minute Wear Time (Upper Thigh)

Study Group 1E: 1 x 80 $\mu$g Glu$^{22,25}$, Leu$^{23,28,31}$, Aib$^{29}$, Lys$^{26,30}$]hPTHrP(1-34)NH$_2$ SC injection (Periumbilical)

Glu$^{22,25}$, Leu$^{23,28,31}$, Aib$^{29}$, Lys$^{26,30}$]hPTHrP(1-34)NH$_2$ was characterized by a rapid absorption as mean $C_{max}$ was achieved within 0.163 hours (~10 minutes) following Glu$^{22,25}$, Leu$^{23,28,31}$, Aib$^{29}$, Lys$^{26,30}$]hPTHrP(1-34)NH$_2$-sMTS 100 $\mu$g study groups and at 0.422 hours (~25 minutes) following Glu$^{22,25}$, Leu$^{23,28,31}$, Aib$^{29}$, Lys$^{26,30}$]hPTHrP(1-34)NH$_2$ for Injection 80 $\mu$g (Study Group 1E). Moreover, [Glu$^{22,25}$, Leu$^{23,28,31}$, Aib$^{29}$, Lys$^{26,30}$]hPTHrP(1-34)NH$_2$ had a short half-life with mean $t_{1/2}$, ranging from 0.302 hours (~18 minutes) to 0.571 hours (~34 minutes) following the Glu$^{22,25}$, Leu$^{23,28,31}$, Aib$^{29}$, Lys$^{26,30}$]hPTHrP(1-34)NH$_2$-sMTS study groups and at 0.970 hours (~58 minutes) following Study Group IE.

Mean peak exposure as measured by $C_{max}$ at 401 pg/mL following Glu$^{22,25}$, Leu$^{23,28,31}$, Aib$^{29}$, Lys$^{26,30}$]hP-THrP(1-34)NH$_2$-sMTS 100 $\mu$g administered to periumbilical region with 15 minute wear time (Study Groups 1B) was relatively comparable to mean peak exposure following Glu$^{22,25}$, Leu$^{23,28,31}$, Aib$^{29}$, Lys$^{26,30}$]hPTHrP(1-34)NH$_2$ for Injection 80 $\mu$g (Study Group 1E) at 452 pg/mL, but was higher compared to Glu$^{22,25}$, Leu$^{23,28,31}$, Aib$^{29}$, Lys$^{26,30}$]hP-THrP(1-34)NH$_2$-sMTS 100 $\mu$g applied to periumbilical or upper thigh regions with 10 second wear time (Study Groups 1A and 1C) at 292 pg/mL and 303 pg/mL, respectively.

The highest mean peak exposure at 676 pg/mL was observed following Glu$^{22,25}$, Leu$^{23,28,31}$, Aib$^{29}$, Lys$^{26,30}$]hP-THrP(1-34)NH$_2$-sMTS 100 $\mu$g administered to upper thigh region with 15 minutes wear time (Study Group ID). Subject 110 with a peak concentration of 1140 pg/mL (~2 times the average peak values of other subjects in this study group), probably contributed to the high $C_{max}$ value of Study Group ID.

The highest mean total exposure (as measured by $AUC_{0-\infty}$) resulted following Glu$^{22,25}$, Leu$^{23,28,31}$, Aib$^{29}$, Lys$^{26,30}$]hPTHrP(1-34)NH$_2$ for Injection 80 $\mu$g (Study Group 1E) at 633.3 pg*hr/mL followed by Study Group ID, at about 268.9 pg*hr/mL, Study Group 1C, at 150.8 pg*hr/mL, and Study Groups 1A and 1B at approximately 142 pg*hr/mL. The lower clearance value for Study Group IE might been the result of higher total exposure for this study group as compared to Glu$^{22,25}$, Leu$^{23,28,31}$, Aib$^{29}$, Lys$^{26,30}$]hPTHrP(1-34)NH$_2$-sMTS study groups.

Mean time to the last detectable plasma Glu$^{22,25}$, Leu$^{23,28,31}$, Aib$^{29}$, Lys$^{26,30}$]hPTHrP(1-34)NH$_2$ concentrations ranged from 1.09 to 1.86 hours following Glu$^{22,25}$, Leu$^{23,28,31}$, Aib$^{29}$, Lys$^{26,30}$]hPTHrP(1-34)NH$_2$-sMTS study groups and was 3.51 hours following Study Group IE.

Apparent total body clearance ranged from 402.6 to 1143 L/hr following Glu$^{22,25}$, Leu$^{23,28,31}$, Aib$^{29}$, Lys$^{26,30}$]hP-THrP(1-34)NH$_2$-sMTS study groups and was lower at 140.6 L/hr following Study Group IE (SC injection).

Plasma Glu$^{22,25}$, Leu$^{23,28,31}$, Aib$^{29}$, Lys$^{26,30}$]hPTHrP(1-34)NH$_2$ PK parameters following Glu$^{22,25}$, Leu$^{23,28,31}$, Aib$^{29}$, Lys$^{26,30}$]hPTHrP(1-34)NH$_2$-sMTS study groups on Days 1 and 7 in Study Period 2 are summarized in Tables 38 and 39.

**Table 38 Summary of Plasma Glu$^{22,25}$, Leu$^{23,28,31}$, Aib$^{29}$, Lys$^{26,30}$]hPTHrP(1-34)NH$_2$ Pharmacokinetic Parameters Following Glu$^{22,25}$, Leu$^{23,28,31}$, Aib$^{29}$, Lys$^{26,30}$]hPTHrP(1-34)NH$_2$Study Groups (Study Period 2) - Day 1**

| | | Study Group 2A | Study Group 2B | Study Group 2C |
|---|---|---|---|---|
| Day | Pharmacokinetic Parameters | Mean ± SD (N) | Mean ± SD (N) | Mean ± SD (N) |
| 1 | $C_{max}$ (pg/mL) | 380 ± 191 | 470 ± 203 | 317 ± 80.6 |
| | | (6) | (5) | (6) |
| | $T_{max}$ (hr) | 0.164 (0.0836, 0.248) | 0.179 (0.164, 0.242) | 0.201 (0.0856, 0.348) |
| | | (6) | (5) | (6) |
| | $T_{last}$ (hr) | 1.69 ± 1.27 | 2.83 ± 1.92 | 1.28 ± 0.424 |
| | | (6) | (5) | (6) |
| | $AUC_{0-t}$ (pg*hr/mL) | 236 ± 229 | 462 ± 577 | 154 ± 69.3 |
| | | (6) | (5) | (6) |
| | $AUC_{0-inf}$ (pg*hr/mL) | 268.6 ± 264.7 | 236.8 ± 102.3 | 176.9 ± 86.89 |
| | | (6) | (4) | (4) |
| | $AUC_{0-tau}$ (pg*hr/mL) | 268.3 ± 264.2 | 236.8 ± 102.3 | 176.8 ± 86.84 |
| | | (6) | (4) | (4) |
| | $AUC_{0-inf}$/dose (pg*hr/mL/$\mu$g) | 1.79 ± 1.76 | 1.58 ± 0.682 | 1.77 ± 0.869 |
| | | (6) | (4) | (4) |
| | $AUC_{0-tau}$/dose (pg*hr/mL/$\mu$g) | 1.79 ± 1.76 | 1.58 ±0.682 | 1.77 ± 0.868 |
| | | (6) | (4) | (4) |
| | $t_{1/2}$ (hr) | 0.568 ± 0.471 | 0.761 ± 0.361 | 0.402 ± 0.126 |
| | | (6) | (4) | (4) |
| | Lambda_z (1/hr) | 2.09 ± 1.57 | 1.06 ± 0.436 | 1,84 ± 0.495 |
| | | (6) | (4) | (4) |
| | AUCR | 0.883 ± 0.0201 | 0.873 ± 0.0332 | 0.898 ± 0.0169 |
| | | (6) | (4) | (4) |
| | CL/F (L/hr) | 1167 ± 1046 | 716.3 ± 264.9 | 666.2 ± 284.4 |
| | | (6) | (4) | (4) |
| | Vd/F (L) | 516.4 ± 112.7 | 718.2 ± 224.1 | 365.8 ± 127.8 |
| | | (6) | (4) | (4) |

$T_{max}$ is presented as Median (Minimum, Maximum)

Study Group 2A = 1 x 150 $\mu$g Glu$^{22,25}$, Leu$^{23,28,31}$, Aib$^{29}$, Lys$^{26,30}$]hPTHrP(1-34)NH$_2$-- sMTS, 10 Second Wear Time Daily for 7 Days (Periumbilical)

Study Group 2B = 1 x 150 $\mu$g Glu$^{22,25}$, Leu$^{23,28,31}$, Aib$^{29}$, Lys$^{26,30}$]hPTHrP(1-34)NH$_2$ -sMTS, 15 min Wear Time Daily for 7 Days (Periumbilical)

Study Group 2C = 1 x 100 $\mu$g Glu$^{22,25}$, Leu$^{23,28,31}$, Aib$^{29}$, Lys$^{26,30}$]hPTHrP(1-34)NH$_2$ -sMTS, 15 min Wear Time Daily for 7 Days (upper thigh)

Subject 204 was excluded from summary statistics for having un-measurable and missing concentration values.

(continued)

|  | | Study Group 2A | Study Group 2B | Study Group 2C |
|---|---|---|---|---|
| Day | Pharmacokinetic Parameters | Mean ± SD (N) | Mean ± SD (N) | Mean ± SD (N) |
| . = Value missing or not reportable. | | | | |

**Table 39 Summary of Plasma Glu$^{22,25}$, Leu$^{23,28,31}$, Aib$^{29}$, Lys$^{26,30}$]hPTHrP(1-34)NH$_2$ Pharmacokinetic Parameters Following Glu$^{22,25}$, Leu$^{23,28,31}$, Aib$^{29}$, Lys$^{26,30}$]hPTHrP(1-34)NH$_2$Study Groups (Study Period 2) - Day 7**

|  | | Study Group 2A | Study Group 2B | Study Group 2C |
|---|---|---|---|---|
| Day | Pharmacokinetic Parameters | Mean ± SD (N) | Mean ± SD (N) | Mean ± SD (N) |
| 7 | $C_{max}$ (pg/mL) | 144 ± 180 | 412 ± 172 | 359 ± 125 |
|  |  | (6) | (6) | (6) |
|  | $T_{max}$ (hr) | 0.166 (0.161, 0.179) | 0.172 (0.0978, 0.203) | 0.159 (0.0839, 0.220) |
|  |  | (5) | (6) | (6) |
|  | $T_{last}$ (hr) | 1.09 ± 1.19 | 2.37 ± 1.37 | 1.51 ± 0.526 |
|  |  | (5) | (6) | (6) |
|  | $AUC_{0-t}$ (pg*hr/mL) | 99.2 ± 168 | 260 ± 209 | 165 ± 67.4 |
|  |  | (6) | (6) | (6) |
|  | $AUC_{0-tau}$ (pg*hr/mL) | 219.3 ± 215.2 | 318.1 ± 283.3 | 184.3 ± 69.95 |
|  |  | (3) | (4) | (6) |
|  | $AUC_{0-tau}$/dose (pg*hr/mL/µg) | 1.46 ± 1.43 | 2.12 ± 1.89 | 1.84 ± 0.699 |
|  |  | (3) | (4) | (6) |
|  | $t_{1/2}$ (hr) | 0.572 ± 0.292 | 0.593 ± 0.419 | 0.489 ± 0.169 |
|  |  | (3) | (4) | (6) |
|  | Lambda_z (1/hr) | 1.47 ± 0.796 | 1.78 ± 1.38 | 1.64 ± 0.810 |
|  |  | (3) | (4) | (6) |
|  | CL/F (L/hr) | 1778 ± 2027 | 809.9 ± 597.1 | 679.2 ± 462.2 |
|  |  | (3) | (4) | (6) |
|  | Vd/F (L) | 957.5 ± 697.6 | 455.2 ± 146.9 | 406.5 ± 106.5 |
|  |  | (3) | (4) | (6) |
|  | AR1 | 0.2939 ± 0.2544 | 1.175 ± 0.9195 | 1.188 ± 0.5306 |
|  |  | (6) | (5) | (6) |
|  | AR2 | 0.4485 ± 0.2636 | 2.097 ± 1.620 | 1.076 ± 0.6683 |
|  |  | (3) | (3) | (4) |
|  | LF | 0.4482 ± 0.2633 | 2.097 ± 1.622 | 1.075 ± 0.6684 |
|  |  | (3) | (3) | (4) |
| $T_{max}$ is presented as Median (Minimum, Maximum) <br> CL/F following multiple dosing was computed as Dose/$AUC_{0-tau}$ <br> Vd/F following multiple dosing was computed as MRT*CLss | | | | |

(continued)

|  | | Study Group 2A | Study Group 2B | Study Group 2C |
|---|---|---|---|---|
| Day | Pharmacokinetic Parameters | Mean ± SD (N) | Mean ± SD (N) | Mean ± SD (N) |
| Study Group 2A = 1 x 150 μg Glu$^{22,25}$, Leu$^{23,28,31}$, Aib$^{29}$, Lys$^{26,30}$]hPTHrP(1-3 4)NH$_2$ - sMTS, 10 Second Wear Time Daily for 7 Days (Periumbilical) | | | | |
| Study Group 2B = 1 x 150 μg Glu$^{22.25}$, Leu$^{21,28,31}$, Aib$^{29}$, Lys$^{26,30}$]hPTHrP(1-34)NH$_2$-sMTS, 10 Second Wear Time Daily for 7 Days (Periumbilical) | | | | |
| Study Group 2C = 1 x 150 μg Glu$^{22,25}$, Leu$^{23,28,31}$, Aib$^{29}$, Lys$^{26,30}$]hPTHrP(1-34)NH$_2$ - sMTS, 10 Second Wear Time Daily for 7 Days (Periumbilical) | | | | |
| Subject 204 was excluded from summary statistics for having un-measurable and missing concentration values. | | | | |
| . = Value missing or not reportable. | | | | |

Mean peak exposure (at 470 pg/mL and 412 pg/mL on Days 1 and 7, respectively) was higher following Glu$^{22,25}$, Leu$^{23,28,31}$, Aib$^{29}$, Lys$^{26,30}$]hPTHrP(1-34)NH$_2$-sMTS 150 μg applied to the periumbilical region with 15 minute wear time (Study Group 2B) compared to the Glu$^{22,25}$, Leu$^{23,28,31}$, Aib$^{29}$, Lys$^{26,30}$]hPTHrP(1-34)NH$_2$-sMTS 150 μg administered to periumbilical region with 10 second wear time (Study Group 2A) at 380 pg/mL and 144 pg/mL on Days 1 and 7, respectively. The lowest mean peak exposure at 317 pg/mL resulted following Glu$^{22,25}$, Leu$^{23,28,31}$, Aib$^{29}$, Lys$^{26,30}$]hP-THrP(1-34)NH$_2$-sMTS 100 μg administered to upper thigh region with 15 minute wear time (Study Group 2C) on Day 1. The second highest mean peak exposure at 359 pg/mL resulted following Study Group 2C on Day 7. Mean total exposure values as measured by AUC$_{0-\infty}$ on Day 1 and AUC$_{0-\tau}$ on Day 7 were 268.8 and 219.3 pg*hr/mL, respectively, following Study Group 2A, were 236.8 and 318.1 pg*hr/mL, respectively, following Study Group 2B, and were 176.9 and 184.3 pg*hr/mL, respectively, following Study Group 2C.

The median T$_{max}$ occurred at approximately 11 minutes, was similar among the study groups. The mean t$_{1/2}$ value was 34 minutes following Study Group 2A, and ranged from 36 to 46 minutes following Study Group 2B, and 24 to 29 minutes following Study Group 2C.

The mean time to the last detectable plasma Glu$^{22,25}$, Leu$^{23,28,31}$, Aib$^{29}$, Lys$^{26,30}$]hPTHrP(1-34)NH$_2$ concentrations ranged from 1.28 hours following Study Group 2C to 2.83 hours following Study Group 2B on Day 1 and from 1.09 hours following Study Group 2A to 2.37 hours following Study Group 2B on Day 7.

The highest apparent total body clearance at 1167 and 1778 L/hr on Days 1 and 7, respectively, resulted following Study Group 2A followed by Study Group 2B at 716.3 and 809.9 L/hr, and Study Group 2C at 666.2 and 679.2 L/hr. Total body clearance values were relatively consistent between Days 1 and 7 following each study group.

Accumulation ratios (AR$_1$ and AR$_2$) and linearity factor (LF) values were 0.2939, 0.4485, and 0.4482, respectively, following Study Group 2A, 1.175, 2.097, and 2.097, respectively, following Study Group 2B, and 1.188, 1.076, and 1.075, respectively, following Study Group 2C.

Plasma Glu$^{22,25}$, Leu$^{23,28,31}$, Aib$^{29}$, Lys$^{26,30}$]hPTHrP(1-34)NH$_2$ PK parameters following Glu$^{22,25}$, Leu$^{23,28,31}$, Aib$^{29}$, Lys$^{26,30}$]hPTHrP(1-34)NH$_2$-sMTS study groups on Days 1 and 7 in Study Period 3 are summarized in Tables 40 and 41.

**Table 40 Summary of Plasma Glu$^{22,25}$, Leu$^{23,28,31}$, Aib$^{29}$, Lys$^{26,30}$]hPTHrP(1-34)NH$_2$Pharmacokinetic Parameters Following Glu$^{22,25}$, Leu$^{23,28,31}$, Aib$^{29}$, Lys$^{26,30}$]hPTHrP(1-34)NH$_2$ Study Groups (Study Period 3) - Day 1**

|  | | Study Group 3A | Study Group 3B | Study Group 3C |
|---|---|---|---|---|
| Day | Pharmacokinetic Parameters | Mean ±SD (N) | Mean ± SD (N) | Mean ± SD (N) |
| 1 | C$_{max}$ (pg/mL) | 347 ± 117 | 261 ± 135 | 345 ± 96.0 |
|  |  | (6) | (6) | (6) |
|  | T$_{max}$ (hr) | 0.163 (0.0817, 0.168) | 0.167 (0.0994, 0.178) | 0.169 (0.157, 0.216) |
|  |  | (6) | (6) | (6) |
|  | T$_{last}$ (hr) | 1.43 ± 0.499 | 1.42 ± 0.493 | 1.60 ± 0.880 |
|  |  | (6) | (6) | (6) |

(continued)

|  | Day | Pharmacokinetic Parameters | Study Group 3A<br>Mean ±SD (N) | Study Group 3B<br>Mean ± SD (N) | Study Group 3C<br>Mean ± SD (N) |
|---|---|---|---|---|---|
|  |  | $AUC_{0-t}$ (pg*hr/mL) | 153 ± 80.1 | 120 ± 66.2 | 165 ± 85.1 |
|  |  |  | (6) | (6) | (6) |
|  |  | $AUC_{0-inf}$ (pg*hr/mL) | 172.2 ± 79.69 | 137.3 ± 66.34 | 198.5 ± 98.76 |
|  |  |  | (6) | (6) | (5) |
|  |  | $AUC_{0-tau}$ (pg*hr/mL) | 172.1 ± 79.62 | 137.2 ± 66.32 | 198.4 ± 98.71 |
|  |  |  | (6) | (6) | (5) |
|  |  | $AUC_{0-inf}$/dose (pg*hr/mL/$\mu$g) | 1.15 ± 0.531 | 0.915 ± 0.442 | 1.32 ± 0.658 |
|  |  |  | (6) | (6) | (5) |
|  |  | $AUC_{0-tau}$/dose (pg*hr/mL/$\mu$g) | 1.15 ± 0.531 | 0.915 ± 0.442 | 1.32 ± 0.658 |
|  |  |  | (6) | (6) | (5) |
|  |  | $t_{1/2}$ (hr) | 0.484 ± 0.151 | 0.538 ± 0.171 | 0.570 ± 0.351 |
|  |  |  | (6) | (6) | (5) |
|  |  | Lambda z (1/hr) | 1.56 ± 0.520 | 1.42 ± 0.509 | 1.84 ± 1.51 |
|  |  |  | (6) | (6) | (5) |
|  |  | AUCR | 0.868 ± 0.104 | 0.846 ± 0.103 | 0.884 ± 0.0245 |
|  |  |  | (6) | (6) | (5) |
|  |  | CL/F (L/hr) | 1046 ± 478.8 | 1308 ± 567.0 | 921.4 ± 431.9 |
|  |  |  | (6) | (6) | (5) |
|  |  | Vd/F (L) | 701.9 ± 361.6 | 1013 ± 632.8 | 641.8 ± 317.5 |
|  |  |  | (6) | (6) | (5) |

$T_{max}$ is presented as Median (Minimum, Maximum)

Study Group 3A = 1 x 150 $\mu$g Glu$^{22,15}$, Leu$^{23,28,31}$, Aib$^{29}$, Lys$^{26,30}$]hPTHrP(1-34)NH$_2$ -sMTS, Wear Time: 5 Minute Days 1 - 6, 30 Second Day 7 (Upper Thigh)

Study Group 3B = 1 x 150 $\mu$g Glu$^{22,25}$, Leu$^{23,28,31}$, Aib$^{29}$, Lys$^{26,30}$]hPTHrP(1-34)NH$_2$ -sMTS, Wear Time: 1 Minute Days 1 - 6, 60 Minute Day 7 (Upper Thigh)

Study Group 3C = 1 x 150 $\mu$g Glu$^{22,25}$, Leu$^{23,28,31}$, Aib$^{29}$, Lys$^{26,30}$]hPTHrP(1-34)NH$_2$ - sMTS, Wear Time: 24 Hour Day 1, 15 Minute Day 7 (Upper Thigh)

. = Value missing or not reportable.

**Table 41 Summary of Plasma Glu$^{22,25}$, Leu$^{23,28,31}$, Aib$^{29}$, Lys$^{26,30}$]hPTHrP(1-34)NH$_2$Pharmacokinetic Parameters Following Glu$^{22,25}$, Leu$^{23,28,31}$, Aib$^{29}$, Lys$^{26,30}$]hPTHrP(1-34)NH$_2$ Study Groups (Study Period 3) - Day 7**

|  | Day | Pharmacokinetic Parameters | Study Group 3A<br>Mean ± SD (N) | Study Group 3B<br>Mean ± SD (N) | Study Group 3C<br>Mean ± SD (N) |
|---|---|---|---|---|---|
|  | 7 | $C_{max}$ (pg/mL) | 381 ± 174 | 319 ± 129 | 334 ± 222 |
|  |  |  | (6) | (6) | (8) |
|  |  | $T_{max}$ (hr) | 0.168 (0.0844, 0.186) | 0.171 (0.0942, 0.254) | 0.168 (0.0789, 0.189) |

(continued)

| Day | Pharmacokinetic Parameters | Study Group 3A Mean ± SD (N) | Study Group 3B Mean ± SD (N) | Study Group 3C Mean ± SD (N) |
|---|---|---|---|---|
| | | (6) | (6) | (8) |
| | $T_{last}$ (hr) | 2.18 ± 0.934 | 2.01 ± 0.550 | 1.44 ± 0.864 |
| | | (6) | (6) | (8) |
| | $AUC_{0-t}$ (pg*hr/mL) | 229 ± 121 | 179 ± 67.3 | 153 ± 107 |
| | | (6) | (6) | (8) |
| | $AUC_{0-inf}$ (pg*hr/mL) | . | . | 169.2 ± 113.5 |
| | | (.) | (.) | (8) |
| | $AUC_{0-tau}$ (pg*hr/mL) | 251.5 ± 125.5 | 205.3 ± 66.54 | 169.2 ± 113.4 |
| | | (6) | (6) | (8) |
| | $AUC_{0-inf}$/dose (pg*hr/mL/$\mu$g) | . | . | 1.13 ± 0.756 |
| | | 1.44(.) | (.) | (8) |
| | $AUC_{0-tau}$ /dose (pg*hr/mL/$\mu$g) | 1.676 ± 0.8367 | 1.369 ± 0.4436 | 1.13 ± 0.756 |
| | | (6) | (6) | (8) |
| | $t_{1/2}$ (hr) | 0.671 ± 0.280 | 0.737 ± 0.138 | 0.454 ± 0.224 |
| | | (6) | (6) | (8) |
| | Lambda_z (1/hr) | 1.13 ± 0.295 | 0.970 ± 0.196 | 1.95 ± 1.06 |
| | | (6) | (6) | (8) |
| | AUCR | . | . | 0.862 ± 0.102 |
| | | (.) | (.) | (8) |
| | CL/F (L/hr) | 723.1 ± 322.8 | 813.6 ± 332.1 | 1578 ± 1430 |
| | | (6) | (6) | (8) |
| | Vd/F (L) | 644.5 ± 241.6 | 878.9 ± 454.1 | 743.7 ± 439.8 |
| | | (6) | (6) | (8) |

$T_{max}$ is presented as Median (Minimum, Maximum)

CL/F following multiple dosing was computed as Dose/$AUC_{0-tau}$

Vd/F following multiple dosing was computed as MRT*CLss

Study Group 3A = 1 x 150 $\mu$g Glu[22.25], Leu[23,28,31], Aib[29], Lys[26,30]hPTHrP(1-34)NH$_2$ -sMTS, Wear Time: 5 Minute Days 1 - 6, 30 Second Day 7 (Upper Thigh)

Study Group 3B = 1 x 150 $\mu$g Glu[22,25], Leu[23,28,31], Aib[29], Lys[26,30]hPTHrP(1-34)NH$_2$-sMTS, Wear Time: 1 Minute Days 1 - 6, 60 Minute Day 7 (Upper Thigh)

Study Group 3C = 1 x 150 $\mu$g Glu[22,25], Leu[23,28,31], Aib[29], Lys[26,30]hPTHrP(1-34)NH$_2$-- sMTS, Wear Time: 24 Hour Day 1, 15 Minute Day 7 (Upper Thigh)

. = Value missing or not reportable.

**Day 1**

Peak and total exposure values were comparable between Glu[22,25], Leu[23,28,31], Aib[29], Lys[26,30]hP-THrP(1-34)NH$_2$-sMTS 150 $\mu$g applied to the upper thigh region with 5 minute and 24 hour wear times (Study Groups 3A and 3C, respectively), but were higher than the corresponding values of Glu[22,25], Leu[23,28,31], Aib[29], Lys[26,30]hP-

THrP(1-34)NH$_2$-sMTS 150 $\mu$g applied to the upper thigh with 1 minute wear time (Study Group 2B).

Median time to reach C$_{max}$ (i.e., T$_{max}$) at approximately 10 minutes and t$_{1/2}$ at approximately 30 to 35 minutes were similar or comparable among the 3 study groups. Furthermore, mean time to the last detectable plasma Glu$^{22,25}$, Leu$^{23,28,31}$, Aib$^{29}$, Lys$^{26,30}$]hPTHrP(1-34)NH$_2$ concentration (i.e., T$_{last}$) ranging from 1.42 to 1.60 hours were comparable among the 3 study groups.

Apparent total body clearance value of 1308 L/hr on Day 1 following the 1 minute wear time (Study Group 3B) was higher compared to those following the 5 minute wear time (Study Group 3A) and 24 hour wear time (Study Group 3C) which were 1046 and 921.4 L/hr, respectively.

**Day 7**

Mean peak and total exposure to Glu$^{22,25}$, Leu$^{23,28,31}$, Aib$^{29}$, Lys$^{26,30}$]hPTHrP(1-34)NH$_2$ were generally higher following Study Group 3A (30 second wear time), followed by Study Group 3B (60 minute wear time), and Study Group 3C (15 minute wear time). As on Day 1, median T$_{max}$ on Day 7 of about 10 minutes was similar and t$_{1/2}$ ranging from 27 to 44 minutes was relatively comparable among the 3 study groups. Time of the last detectable plasma Glu$^{22,25}$, Leu$^{23,28,31}$, Aib$^{29}$, Lys$^{26,30}$]hPTHrP(1-34)NH$_2$ concentration (i.e., T$_{last}$) was approximately 2 hours following 30 second and 60 minute wear times, which was somewhat later compared to the T$_{last}$ value following the 15 minute wear time of approximately 1.5 hours.

The apparent total body clearance value of 1578 L/hr following the 15 minute wear time (Study Group 3C) was approximately 2 times higher compared to those following the 30 second wear time (Study Group 3A) and 60 minute wear time (Study Group 3B) at 723 and 814 L/hr, respectively.

The results of the relative bioavailability (F$_{rel}$) of Glu$^{22,25}$, Leu$^{23,28,31}$, Aib$^{29}$, Lys$^{26,30}$]hPTHrP(1-34)NH$_2$-sMTS study groups for Day 1 in Study Periods 1, 2, and 3 compared to Glu$^{22,25}$, Leu$^{23,28,31}$, Aib$^{29}$, Lys$^{26,30}$]hPTHrP(1-34)NH$_2$ for Injection 80 $\mu$g are presented in Table 42.

**Table 42 Summary of relative bioavailability (F$_{rel}$) of Glu$^{22,25}$, Leu$^{23,28,31}$, Aib$^{29}$, Lys$^{26,30}$]hPTHrP(1-34) NH$_2$-sMTS study groups for Day 1 in Study Periods 1, 2, and 3 compared to Glu$^{22,25}$, Leu$^{23,28,31}$, Aib$^{29}$, Lys$^{26,30}$] hPTHrP(1-34)NH$_2$ for Injection 80 $\mu$g**

| | | Mean Dose Normalized AUC0-inf | | | | |
| | | sMTS Dose | | SC Dose | | |
| Treatment | Study Period | Mean | N | Mean | N | Frel |
|---|---|---|---|---|---|---|
| 1A | 1 | 1.42 | 6 | 7.92 | 6 | 0.180 |
| 1B | 1 | 1.42 | 6 | 7.92 | 6 | 0.180 |
| 1C | 1 | 1.51 | 6 | 7.92 | 6 | 0.190 |
| ID | 1 | 2.69 | 6 | 7.92 | 6 | 0.340 |
| 2A | 2 | 1.79 | 6 | 7.92 | 6 | 0.226 |
| 2B | 2 | 1.58 | 4 | 7.92 | 6 | 0.199 |
| 2C | 2 | 1.77 | 4 | 7.92 | 6 | 0.223 |
| 3A | 3 | 1.15 | 6 | 7.92 | 6 | 0.145 |
| 3B | 3 | 0.92 | 6 | 7.92 | 6 | 0.116 |
| 3C Day 1 | 3 | 1.32 | 5 | 7.92 | 6 | 0.167 |

(continued)

| 3C Day 7 | 3 | Mean Dose Normalized AUC0-inf | | | | 0.143 |
|---|---|---|---|---|---|---|
| | | sMTS Dose | | SC Dose | | |
| | | 1.13 | 8 | 7.92 | 6 | |

1A = 1 x 100 μg Glu$^{22,25}$, Leu$^{23,28,31}$, Aib$^{29}$, Lys$^{26,30}$]hPTHrP(1-34)NH$_2$-sMTS, 10 Second Wear Time (Periumbilical) 1B=1 x 100 μg Glu$^{22,25}$, Leu$^{23,28,31}$, Aib$^{29}$, Lys$^{26,30}$]hPTHrP(1-34)NH$_2$-sMTS, 15 Minute Wear Time (Periumbilical) 1C=1 x 100 μg Glu$^{22,25}$, Leu$^{23,28,31}$, Aib$^{29}$, Lys$^{26,30}$]hPTHrP(1-34)NH$_2$-sMTS, 10 Second Wear Time (Upper Thigh) 1D=1 x 100 μg Glu$^{22,25}$, Leu$^{23,28,31}$, Aib$^{29}$, Lys$^{26,30}$]hPTHrP(1-34)NH$_2$-sMTS, 15 Minute Wear Time (Upper Thigh) 2A = 1 x 150 μg Glu$^{22,25}$, Leu$^{23,28,31}$, Aib$^{29}$, Lys$^{26,30}$]hPTHrP(1-34)NH$_2$-sMTS, 10 Second Wear Time Daily for 7 Days (Periumbilical) 2B = 1 x 150 μg Glu$^{22,25}$, Leu$^{23,28,31}$, Aib$^{29}$, Lys$^{26,30}$]hPTHrP(1-34)NH$_2$-sMTS, 15 Minute Wear Time Daily for 7Days (Periumbilical) 2C= 1 x 100 μg Glu$^{22,25}$, Leu$^{23,28,31}$, Aib$^{29}$, Lys$^{26,30}$]hPTHrP(1-34)NH$_2$-sMTS, 15 Minute Wear Time Daily for 7 Days (Upper Thigh) 3A = 1 x 150 μg Glu$^{22,25}$, Leu$^{23,28,31}$, Aib$^{29}$, Lys$^{26,30}$]hPTHrP(1-34)NH$_2$-sMTS, Wear Time: 5 Minute Days 1-6,30 Second Day 7 (Upper Thigh) 3B = 1 x 150 μg Glu$^{22,25}$, Leu$^{23,28,31}$, Aib$^{29}$, Lys$^{26,30}$]hPTHrP(1-34)NH$_2$-sMTS, Wear Time: 1 Minute Days 1-6, 60 Minute Day 7 (Upper Thigh) 3C= 1 x 150 μg Glu$^{22,25}$, Leu$^{23,28,31}$, Aib$^{29}$, Lys$^{26,30}$]hPTHrP(1-34)NH$_2$-sMTS, Wear Time: 24 Hour Day 1,15 Minute Day 7 (Upper Thigh)

**Serum CTX (collagen type 1 cross-linked C-telopeptide)**

Predose samples were obtained on Days 1, 3, and 7 in Study Periods 2 (Study Groups 2A, 2B, 2C, and placebo) and 3 (Study Groups 3A and 3B) for the determination of serum CTX concentrations. Predose serum concentrations on Day 1 were used as baseline to compute the change from baseline concentrations for Days 3 and 7.

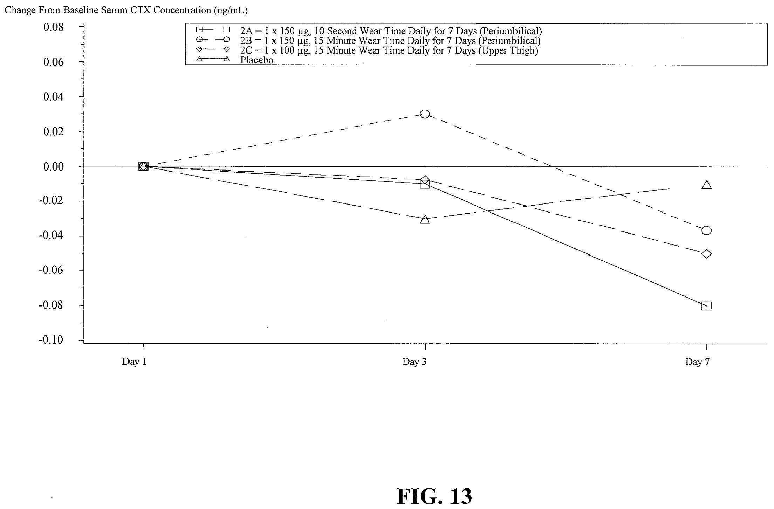

The mean change from baseline CTX concentrations following Glu$^{22,25}$, Leu$^{23,28,31}$, Aib$^{29}$, Lys$^{26,30}$]hP-THrP(1-34)NH$_2$-sMTS study groups on Days 1, 3, and 7 in Study Period 2 are presented in Figure 13.

With the exception of Glu$^{22,25}$, Leu$^{23,28,31}$, Aib$^{29}$, Lys$^{26,30}$]hPTHrP(1-34)NH$_2$ Glu$^{22,25}$, Leu$^{23,28,31}$, Aib$^{29}$, Lys$^{26,30}$]hPTHrP(1-34)NH$_2$-sMTS 150 μg applied to the periumbilical region with 15 minute wear time (Study Group 2B) on Day 3, mean CTX concentrations in serum following Glu$^{22,25}$, Leu$^{23,28,31}$, Aib$^{29}$, Lys$^{26,30}$]hPTHrP(1-34)NH$_2$-sMTS study groups remained below the baseline levels on Days 3 and 7. Mean CTX concentrations in serum were at or above the placebo levels on Day 3 and were below the placebo levels on Day 7.

Mean change from baseline serum CTX concentrations were 0.0 and -0.1 ng/mL on Days 3 and 7, respectively, following Glu$^{22,25}$, Leu$^{23,28,31}$, Aib$^{29}$, Lys$^{26,30}$]hPTHrP(1-34)NH$_2$-sMTS 150 μg applied into the periumbilical region with 10 second wear time (Study Group 2A); 0.0 and -0.1 ng/mL following Glu$^{22,25}$, Leu$^{23,28,31}$, Aib$^{29}$, Lys$^{26,30}$]hP-THrP(1-34)NH$_2$ Glu$^{22,25}$, Leu$^{23,28,31}$, Aib$^{29}$, Lys$^{26,30}$]hPTHrP(1-34)NH$_2$ Glu$^{22,25}$, Leu$^{23,28,31}$, Aib$^{29}$, Lys$^{26,30}$]hP-THrP(1-34)NH$_2$ Glu$^{22,25}$, Leu$^{23,28,31}$, Aib$^{29}$, Lys$^{26,30}$]hPTHrP(1-34)NH$_2$-sMTS 150 μg (Days 1 and 7, respectively) applied into the periumbilical region with 15 minute wear time (Study Group 2B); 0.0 and -0.1 ng/mL following Glu$^{22,25}$, Leu$^{23,28,31}$, Aib$^{29}$, Lys$^{26,30}$]hPTHrP(1-34)NH$_2$-sMTS 100 μg applied into the upper thigh region with 15 minute wear time (Study Group 2C); and 0.0 ng/mL following placebo.

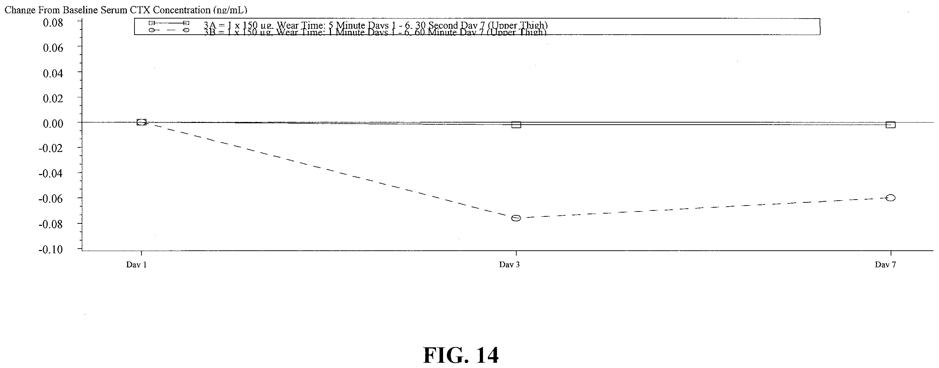

The mean change from baseline CTX concentrations following [Glu$^{22,25}$, Leu$^{23,28,31}$, Aib$^{29}$, Lys$^{26,30}$]hP-THrP(1-34)NH$_2$-sMTS study groups on Days 1, 3, and 7 in Study Period 3 are presented in the Figure 14

While mean CTX concentrations in serum following Glu$^{22,25}$, Leu$^{23,28,31}$, Aib$^{29}$, Lys$^{26,30}$]hP-THrP(1-34)NH$_2$-sMTS 150 μg applied to the upper thigh region with 5 minute wear time on Day 1 and 30 second wear time on Day 7 (Study Group 3A) remained at baseline levels on Days 3 and 7, those following Glu$^{22,25}$, Leu$^{23,28,31}$, Aib$^{29}$, Lys$^{26,30}$]hPTHrP(1-34)NH$_2$-sMTS 150 μg applied to the upper thigh region with 1 minute wear time on Day 1 and 60 minute wear time on Day 7 (Study Group 3B) decreased below the baseline levels on Days 3 and 7.

Mean change from baseline serum CTX concentration values were 0.0 ng/mL following Study Group 3A and -0.1 ng/mL following Study Group 3B.

The mean maximum change from baseline in serum CTX concentrations ($\Delta_{max}$) were 0.0 ng/mL following Study Group 3A and -0.1 ng/mL following Study Group 3B.

**Serum P1NP (procollagen type 1 amino-terminal propeptide)**

Predose samples were obtained on Days 1, 3, and 7 in Study Periods 2 (Study Groups 2A, 2B, 2C, and placebo) and 3 (Study Groups 3A and 3B) for the determination of serum P1NP concentrations. Predose serum concentrations on Day 1 were used as baseline to compute the change from baseline concentrations for Days 3 and 7.

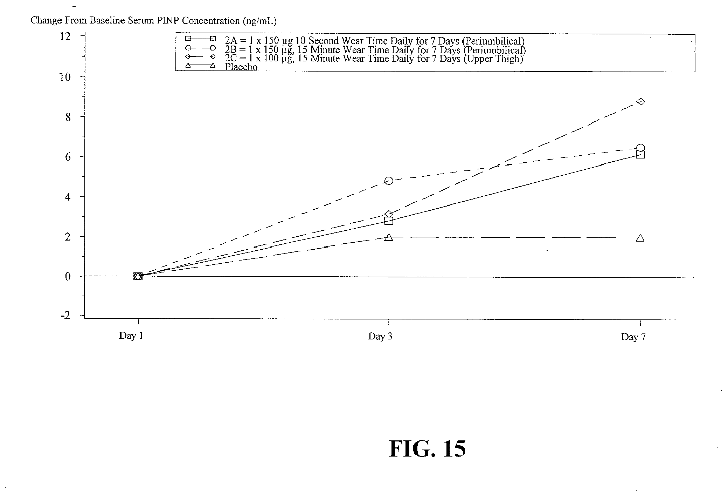

The mean change from baseline P1NP concentrations following $Glu^{22,25}$, $Leu^{23,28,31}$, $Aib^{29}$, $Lys^{26,30}$]hPTHrP(1-34)NH$_2$-sMTS study groups on Days 1, 3, and 7 in Study Period 2 are presented in Figure 15 .

Based on change from baseline values, mean P1NP concentrations in serum following $Glu^{22,25}$, $Leu^{23,28,31}$, $Aib^{29}$, $Lys^{26,30}$]hPTHrP(1-34)NH$_2$-sMTS study groups remained above the baseline and placebo levels on Days 3 and 7. The mean values were higher on Day 7 compared to Day 3.

Mean change from baseline serum P1NP concentrations were 2.8 and 6.2 ng/mL on Days 3 and 7, respectively, following $Glu^{22,25}$, $Leu^{23,28,31}$, $Aib^{29}$, $Lys^{26,30}$]hPTHrP(1-34)NH$_2$-sMTS 150 $\mu$g applied into the periumbilical region with 10 second wear time (Study Group 2A);.6 and 7.2 ng/mL on Days 3 and 7, respectively, following $Glu^{22,25}$, $Leu^{23,28,31}$, $Aib^{29}$, $Lys^{26,30}$]hPTHrP(1-34)NH$_2$-sMTS 150 $\mu$g applied into the periumbilical region with 15 minute wear time (Study Group 2B); 3.2 and 8.8 ng/mL on Days 3 and 7, respectively, following $Glu^{22,25}$, $Leu^{23,28,31}$, $Aib^{29}$, $Lys^{26,30}$]hPTHrP(1-34)NH$_2$-sMTS 100 $\mu$g applied into the upper thigh region with 15 minute wear time (Study Group 2C); and 2.0 ng/mL following placebo.

The mean maximum change from baseline in serum P1NP concentrations ($\Delta_{max}$) were 5.5 ng/mL following Study Group 2A, 7.8 ng/mL following Study Group 2B, 8.8 ng/mL following Study Group 2C, and 1.0 ng/mL following placebo.

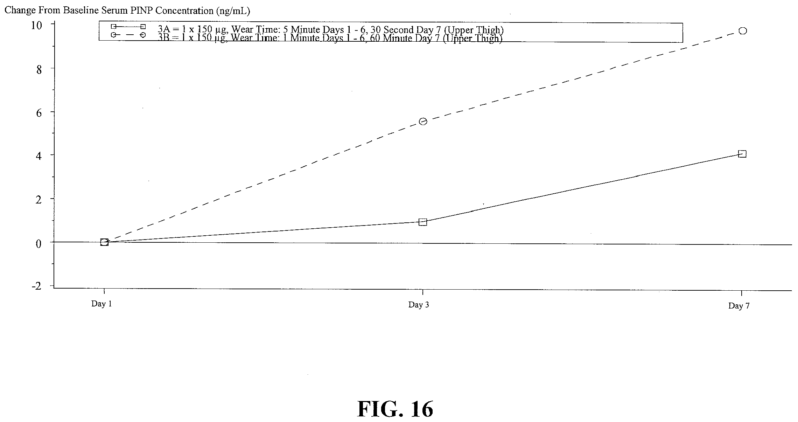

The mean change from baseline P1NP concentrations following $Glu^{22,25}$, $Leu^{23,28,31}$, $Aib^{29}$, $Lys^{26,30}$]hPTHrP(1-34)NH$_2$-sMTS study groups on Days 1, 3, and 7 in Study Period 3 are presented in Figure 16.

Based on change from baseline values, mean P1NP concentrations in serum following $Glu^{22,25}$, $Leu^{23,28,31}$, $Aib^{29}$, $Lys^{26,30}$]hPTHrP(1-34)NH$_2$-sMTS study groups increased above the baseline level (Day 1 predose) and were higher on Day 7 compared to Day 3.

Mean change from baseline serum P1NP concentrations were 1.0 and 4.2 ng/mL following $Glu^{22,25}$, $Leu^{23,28,31}$, $Aib^{29}$, $Lys^{26,30}$]hPTHrP(1-34)NH$_2$-sMTS 150 $\mu$g applied to the upper thigh region with 5 minute wear time on Day 1 and 30 second wear time on Day 7 (Study Group 3A) and were 5.6 and 9.8 ng/mL following $Glu^{22,25}$, $Leu^{23,28,31}$, $Aib^{29}$, $Lys^{26,30}$]hPTHrP(1-34)NH$_2$-sMTS 150 $\mu$g applied to the upper thigh region with 1 minute wear time on Day 1 and 60 minute wear time on Day 7 (Study Group 3B).

The mean maximum change from baseline in serum P1NP concentrations ($\Delta_{max}$) were 4.7 ng/mL following Study Group 3A and 10.4 ng/mL following Study Group 2B.

The systematic delivery of [$Glu^{22,25}$, $Leu^{23,28,31}$, $Aib^{29}$, $Lys^{26,30}$]hPTHrP(1-34)NH$_2$ using microneedle technology has been clearly demonstrated in preclinical models (rats) and postmenopausal women. The release profile appears to be extremely rapid with high $C_{max}$ values that were quickly reached. The levels obtained, bone marker response and increases in bone mineral density clearly indicate the clinical utility of the many embodiments of this invention.

While this invention has been particularly shown and described with references to example embodiments thereof, it will be understood by those skilled in the art that various changes in form and details may be made therein without departing from the scope of the invention encompassed by the appended claims.

We set out below certain aspects of the invention:

1. An aqueous formulation comprising [$Glu^{22,25}$, $Leu^{23,28,31}$, $Aib^{29}$, $Lys^{26,30}$]hPTHrP(1-34)NH$_2$, wherein said formulation is suitable for coating a microprojection array and wherein said formulation comprises at least 5% by weight of [$Glu^{22,25}$, $Leu^{23,28,31}$, $Aib^{29}$, $Lys^{26,30}$]hPTHrP(1-34)NH$_2$.

2. An aqueous formulation according to aspect 1, wherein said formulation comprises at least 40% by weight of [$Glu^{22,25}$, $Leu^{23,28,31}$, $Aib^{29}$, $Lys^{26,30}$]hPTHrP(1-34)NH$_2$.

3. An aqueous formulation according to aspect 1, wherein said formulation comprises at least 45% by weight of [$Glu^{22,25}$, $Leu^{23,28,31}$, $Aib^{29}$, $Lys^{26,30}$]hPTHrP(1-34)NH$_2$.

4. An aqueous formulation according to aspect 1, wherein said formulation comprises between 40% and 63% by weight [$Glu^{22,25}$, $Leu^{23,28,31}$, $Aib^{29}$, $Lys^{26,30}$]hPTHrP(1-34)NH$_2$.

5. An aqueous formulation according to any of the preceding aspects, wherein said formulation has a viscosity of greater than 500 centipoises at 25 °C and a shear rate of 100 s$^{-1}$.

6. An aqueous formulation according to any of the preceding aspects, wherein said formulation has a viscosity of greater than 1,000 centipoises at 25 °C and a shear rate of 100 s$^{-1}$.

7. An aqueous formulation according to any of the preceding aspects, wherein said formulation has a viscosity of greater than 1,250 centipoises at 25 °C and a shear rate of 100 s$^{-1}$.

8. An aqueous formulation according to any of the preceding aspects, wherein said formulation has a viscosity of greater than 1,500 centipoises at 25 °C and a shear rate of 100 s$^{-1}$.

9. An aqueous formulation according to any of aspects 1-4, wherein said formulation has a viscosity of between 1,000 centipoises and 3,000 centipoises at 25 °C and a shear rate of 100 s$^{-1}$.

10. An aqueous formulation according to any of the preceding aspects, wherein said aqueous solution further comprises acetate and/or acetic acid.

11. An aqueous solution according to any of the preceding aspects wherein, said aqueous solution further comprises from 4% to 10% by weight acetate.

12. An aqueous solution according to any of the preceding aspects wherein, said aqueous solution further comprises from 1% to 15% by weight histidine.

13. The solution according to aspect 12 wherein said histidine is 3% by weight.

14. The solution according to aspect 12 wherein said histidine is 5% by weight.

15. An aqueous solution according to any of the preceding aspects wherein, said aqueous solution further comprises from 2% to 10% by weight potassium chloride.

16. The solution according to aspect 15 wherein said potassium chloride is 9% by weight.

17. An aqueous formulation according to any of the preceding aspects, further comprising an amphiphilic surfactant.

18. An aqueous formulation according to any of the preceding aspects, further comprising an uncharged amphiphilic surfactant.

19. An aqueous formulation according to any of the preceding aspects further comprising a polysaccharide.

20. An aqueous formulation according to any of the preceding aspects further comprising sucrose or hydroxyethyl cellulose.

21. An aqueous formulation according to any of the preceding aspects further comprising a buffer.

22. An aqueous formulation according to any of the previous aspects further comprising a buffered saline solution.

23. An aqueous formulation according to any one of the preceding aspects further comprising a IX phosphate buffered saline solution.

24. An aqueous formulation according to any of the preceding aspects wherein said aqueous solution has a pH from 3.0 to 8.0.

25. An aqueous formulation according to any of the previous aspects wherein said aqueous formulation has a pH from 3.0 to 7.0.

26. An aqueous formulation according to any of the previous aspects wherein said aqueous formulation has a pH from 3.5 to 6.5.

27. An aqueous formulation according to any of the previous aspects wherein said aqueous formulation has a pH from 4.0 to 6.0.

28. An aqueous formulation according to any of the previous aspects wherein said aqueous formulation has a pH from 4.5 to 5.5.

29. A microprojection array suitable for transdermal drug delivery wherein said microprojection array comprises a backing material with a plurality of attached microprojections wherein at least one of said microprojections comprises a coating of a formulation comprising [Glu$^{22,25}$, Leu$^{23,28,31}$, Aib$^{29}$, Lys$^{26,30}$]hPTHrP(1-34)NH$_2$.

30. A microprojection array according to aspect 29 wherein said coating is manufactured by a process step comprising application of an aqueous formulation described in any of aspects 1-28.

31. A microprojection array according to aspect 30 wherein said process step comprising application is followed by a process step comprising drying of said coating.

32. A microprojection array according to any of aspects 29 - 31 wherein said microprojections comprise a carbon containing polymeric material.

33. A microprojection array according to any of aspects 29-32 wherein said microprojections comprise a polycarbonate polymer.

34. A microprojection array according to any of aspects 29-33 wherein said microprojections comprise a liquid crystal polymer.

35. A microprojection array according to any of aspects 29-34 wherein said microprojections comprise a base with a width greater than two times the width at the tip.

36. A microprojection array according to any of aspects 29-35 wherein said microprojections comprise a base with a width greater than four times the width at the tip.

37. A microprojection array according to any of aspects 29-36 wherein said microprojections comprise a base with a width greater than six times the width at the tip.

38. A microprojection array according to any of aspects 29-37 wherein said microprojections are microneedles.

39. A microprojection array according to any of aspects 29-38 wherein said microprojections are pyramidal in shape.

40. A microprojection array according to any of aspects 29-39 wherein said microprojections have a rectangular base and rectangular tip.

41. A microprojection array according to any of aspects 29-39 wherein said microprojections have a square base and square tip.

42. A microprojection array according to any of aspects 29-41 wherein said microprojections have a flexural modulus of greater than 1,000 MPa (ISO 178).

43. A microprojection array according to any of aspects 29-42 wherein said microprojections have a flexural modulus of greater than 2,000 MPa (ISO 178).

44. A microprojection array according to any of aspects 29-43 wherein said microprojections have a flexural modulus of greater than 3,000 MPa (ISO 178).

45. A microprojection array according to any of aspects 29-44 wherein said microprojections have a flexural modulus from about 3,000 MPa to about 15,000 MPa (ISO 178).

46. A microprojection array according to any of aspects 29-45 wherein said microprojections have a flexural modulus from about 5,000 MPa to about 12,000 MPa (ISO 178).

47. A microprojection array according to any of aspects 29-46 wherein said microprojections have a flexural modulus from about 8,000 MPa and 12,000 MPa (ISO 178).

48. A microprojection array according to any of aspects 29-47 wherein said microprojections have a flexural modulus from about 9,000 MPa to about 10,000 MPa (ISO 178).

49. A microprojection array according to any of aspects 29-48 wherein said microprojections have a flexural modulus of between 9,000 MPa and 9,500 MPa (ISO 178).

50. A microprojection array according to any of aspects 29-49 wherein said microprojections are more than 100 microns and less than 1,000 microns in length.

51. A microprojection array according to any of aspects 29-50 wherein said microprojections are more than 250 microns and less than 750 microns in length.

52. A microprojection array according to any of aspects 29-51 wherein said microprojections are more than 400 microns and less than 600 microns in length.

53. A microprojection array according to any of aspects 29-52 wherein said microprojections are about 500 microns in length.

54. A microprojection array according to any of aspects 29-53 wherein said array has a microprojection density between 20 and 1,000 microprojections per $cm^2$.

55. A microprojection array according to any of aspects 29-54 wherein said array has a microprojection density between 100 and 500 microprojections per $cm^2$.

56. A microprojection array according to any of aspects 29-55 wherein said array comprises between 50 and 600 microprojections.

57. A microprojection array according to any of aspects 29-56 wherein said array comprises between 100 and 500 microprojections.

58. A microprojection array according to any of aspects 29-57 wherein said array comprises between 250 and 400 microprojections.

59. A microprojection array according to any of aspects 29-58 wherein said array comprises between 300 and 375 microprojections.

60. A microprojection array according to any of aspects 29-59 wherein said array comprises about 366 microprojections.

61. A microprojection array according to any of aspects 29-59 wherein said array comprises about 316 microprojections.

62. A microprojection array according to any of aspects 29-61 wherein said array comprises from about 63.75 $\mu$g to about 86.25 $\mu$g of [Glu$^{22,25}$, Leu$^{23,28,31}$, Aib$^{29}$, Lys$^{26,30}$]hPTHrP(1-34)NH$_2$.

63. A microprojection array according to any of aspects 29-62 wherein said array comprises about 75 $\mu$g of [Glu$^{22,25}$, Leu$^{23,28,31}$, Aib$^{29}$, Lys$^{26,30}$]hPTHrP(1-34)NH$_2$.

64. A microprojection array according to any of aspects 29-61 wherein said array comprises from about 85 $\mu$g to about 115 $\mu$g of [Glu$^{22,25}$, Leu$^{23,28,31}$, Aib$^{29}$, Lys$^{26,30}$]hPTHrP(1-34)NH$_2$.

65. A microprojection array according to any of aspects 29-61 and 64 wherein said array comprises about 100 $\mu$g of [Glu$^{22,25}$, Leu$^{23,28,31}$, Aib$^{29}$, Lys$^{26,30}$]hPTHrP(1-34)NH$_2$.

66. A microprojection array according to any of aspects 29-61 wherein said array comprises from about 106.25 $\mu$g to about 143.75 $\mu$g of [Glu$^{22,25}$, Leu$^{23,28,31}$, Aib$^{29}$, Lys$^{26,30}$]hPTHrP(1-34)NH$_2$.

67. A microprojection array according to any of aspects 29-61 and 66 wherein said array comprises about 125 $\mu$g

of $[Glu^{22,25}, Leu^{23,28,31}, Aib^{29}, Lys^{26,30}]hPTHrP(1-34)NH_2$.

68. A microprojection array according to any of aspects 29-62 wherein said array comprises from about 127.5 $\mu$g to about 172.5 $\mu$g of $[Glu^{22,25}, Leu^{23,28,31}, Aib^{29}, Lys^{26,30}]hPTHrP(1-34)NH_2$.

69. A microprojection array according to any of aspects 29-61 and 68 wherein said array comprises about 150 $\mu$g of $[Glu^{22,25}, Leu^{23,28,31}, Aib^{29}, Lys^{26,30}]hPTHrP(1-34)NH_2$.

70. A method of administering $[Glu^{22,25}, Leu^{23,28,31}, Aib^{29}, Lys^{26,30}]hPTHrP(1-34)NH_2$ to a mammal comprising contacting the skin of said mammal with a microprojection array according to any of aspects 29-69 wherein said contacting occurs with sufficient force to cause penetration of one or more of said microprojections comprising a coating of said formulation into the skin of said mammal.

71. The method of aspect 70 wherein said mammal is a human.

72. The method of aspect 71 wherein said human has osteopenia or osteoporosis.

73. The method of any of aspects 71-72 wherein said human is a post-menopausal woman.

74. The method of any of aspects 71-73 wherein said human has glucocorticoid-induced osteopenia or osteoporosis.

75. A method of treating osteoporosis in a human in need thereof comprising administering $[Glu^{22,25}, Leu^{23,28,31}, Aib^{29}, Lys^{26,30}]hPTHrP(1-34)NH_2$ once-daily according to the method of any of aspects 71-74.

76. A method of treating osteoporosis in a human in need thereof comprising administering $[Glu^{22,25}, Leu^{23,28,31}, Aib^{29}, Lys^{26,30}]hPTHrP(1-34)NH_2$ once-weekly according to the method of any of aspects 71-74.

77. The method of aspect 75 or 76 wherein said microprojection array is contacted on the surface of the stomach area of said human.

78. The method of aspect 75 or 76 wherein said microprojection array is contacted on the surface of the deltoid area of said human.

79. The method of aspect 75 or 76 wherein said microprojection array is contacted on the surface of the thigh of said human.

80. The method according to any of aspects 70-79 wherein said microprojection array is left in place with one or more of said microprojections embedded in the skin after said contacting for a period of from about 10 seconds to about 24 hours.

81. The method of aspect 80 wherein said period is from about 10 seconds to about 1 hour.

82. The method of aspect 80 wherein said period is from about 10 seconds to about 30 minutes.

83. The method of aspect 80 wherein said period is from about 10 seconds to about 15 minutes.

84. The method of aspect 80 wherein said period is from about 10 seconds to about 5 minutes.

85. The method of aspect 80 wherein said period is about 10 seconds.

86. The method of aspect 80 wherein said period is about 30 seconds.

87. The method of aspect 80 wherein said period is about 1 minute.

88. The method of aspect 80 wherein said period is about 5 minutes.

89. The method of aspect 80 wherein said period is about 15 minutes.

90. The method of aspect 80 wherein said period is about 30 minutes.

91. A method of treating post-menopausal osteoporosis comprising administering [$Glu^{22,25}$, $Leu^{23,28,31}$, $Aib^{29}$, $Lys^{26,30}$]hPTHrP(1-34)$NH_2$ to a woman in need thereof said administering comprising contacting the skin of said woman with a microprojection array as described in any of aspects 29-69 wherein said contacting occurs with sufficient force to cause penetration of one or more of said microprojection members into the skin of said woman and wherein said administration results in $C_{max}$ plasma levels of [$Glu^{22,25}$, $Leu^{23,28,31}$, $Aib^{29}$, $Lys^{26,30}$]hP-THrP(1-34)$NH_2$ of greater than 200 pg/mL.

92. The method of aspect 91 wherein said administering results in $C_{max}$ plasma levels of [$Glu^{22,25}$, $Leu^{23,28,31}$, $Aib^{29}$, $Lys^{26,30}$]hPTHrP(1-34)$NH_2$ of greater than 300 pg/mL.

93. The method of aspect 91 wherein said administering results in $C_{max}$ plasma levels of [$Glu^{22,25}$, $Leu^{23,28,31}$, $Aib^{29}$, $Lys^{26,30}$]hPTHrP(1-34)$NH_2$ of greater than 400 pg/mL.

94. The method of aspect 91 wherein said administering results in $C_{max}$ plasma levels of [$Glu^{22,25}$, $Leu^{23,28,31}$, $Aib^{29}$, $Lys^{26,30}$]hPTHrP(1-34)$NH_2$ of greater than 500 pg/mL.

95. The method of any of aspects 91-94 wherein the plasma $T_{max}$ occurs at less than one hour post-administration time.

96. The method of any of the aspects 91-95 wherein the plasma $T_{max}$ occurs at less than ½ hour post administration time.

97. A method of treating one or more fractured bones comprising administering [$Glu^{22,25}$, $Leu^{23,28,31}$, $Aib^{29}$, $Lys^{26,30}$]hPTHrP(1-34)$NH_2$ to a human in need thereof by contacting the skin of said human with a microprojection array as described in any of aspects 29-69 wherein said contacting occurs with sufficient force to cause penetration of one or more of said microprojection members into the skin of said human.

98. The method of aspect 97 wherein said administering is a once-daily administration.

99. The method of any of aspects 97 or 98 said microprojection array is left in place with one or more of said microprojections imbedded in the skin after said contacting for a period of from about 10 seconds to about 24 hours.

100. The method of aspect 99 wherein said period is from about 10 seconds to about 1 hour.

101. The method of aspect 99 wherein said period is from about 10 seconds to about 30 minutes.

102. The method of aspect 99 wherein said period is from about 10 seconds to about 15 minutes.

103. The method of aspect 99 wherein said period is from about 10 seconds to about 5 minutes.

104. The method of aspect 99 wherein said period is about 10 seconds.

105. The method of aspect 99 wherein said period is about 30 seconds.

106. The method of aspect 99 wherein said period is about 1 minutes.

107. The method of aspect 99 wherein said period is about 5 minutes.

108. The method of aspect 99 wherein said period is about 15 minutes.

109. The method of aspect 99 wherein said period is about 30 minutes.

110. The method of any of aspects 97-109 wherein said administering results in $C_{max}$ plasma levels of [$Glu^{22,25}$, $Leu^{23,28,31}$, $Aib^{29}$, $Lys^{26,30}$]hPTHrP(1-34)$NH_2$ of greater than 200 pg/mL.

111. The method of any of aspects 97-110 wherein said administering results in $C_{max}$ plasma levels of [$Glu^{22,25}$,

Leu$^{23,28,31}$, Aib$^{29}$, Lys$^{26,30}$]hPTHrP(1-34)NH$_2$ of greater than 300 pg/mL.

112. The method of any of aspects 97-111 wherein said administering results in C$_{max}$ plasma levels of [Glu$^{22,25}$, Leu$^{23,28,31}$, Aib$^{29}$, Lys$^{26,30}$]hPTHrP(1-34)NH$_2$ of greater than 400 pg/mL.

113. The method of any of aspects 97-112 wherein said administering results in C$_{max}$ plasma levels of Glu$^{22,25}$, Leu$^{23,28,31}$, Aib$^{29}$, Lys$^{26,30}$]hPTHrP(1-34)NH$_2$ of greater than 500 pg/mL.

114. The method of any of aspects 97-113 wherein the plasma T$_{max}$ occurs at less than one hour post-administration time.

115. The method of any of the aspects 97-114 wherein the plasma T$_{max}$ occurs at less than ½ hour post administration time.

116. The method of any of aspects 97-115 wherein said microprojection array is contacted on the surface of the stomach area of said human.

117. The method of any of aspects 97-115 wherein said microprojection array is contacted on the surface of the deltoid region of said human.

118. The method of any of aspects 97-115 wherein said microprojection array is contacted on the surface of the thigh of said human.

119. The method of any of aspects 97-118 wherein said one or more fractured bones was detected within 3 months from the time that said administering begins.

120. The method of any of aspects 97-119 wherein said one or more fractured bones was detected within 1 month from the time that said administering begins.

121. The method of any of aspects 97-120 wherein said one or more fractured bones was detected within two weeks from the time that said administering begins.

122. A method of treating osteoarthritis comprising administering [Glu$^{22,25}$, Leu$^{23,28,31}$, Aib$^{29}$, Lys$^{26,30}$]hP-THrP(1-34)NH$_2$ to a human in need thereof by contacting the skin of said human with a microprojection array as described in any of aspects 29-69 wherein said contacting occurs with sufficient force to cause penetration of one or more of said microprojection members into the skin of said human.

123. The method of aspect 122 wherein said administering is a once-daily administration.

124. The method of any of aspects 122 or 123 wherein said microprojection array is left in place with one or more of said microprojections embedded in the skin after said contacting for a period of from about 10 seconds to about 24 hours.

125. The method of aspect 124 wherein said period is from about 10 seconds to about 1 hour.

126. The method of aspect 124 wherein said period is from about 10 seconds to about 30 minutes.

127. The method of aspect 124 wherein said period is from about 10 seconds to about 15 minutes.

128. The method of aspect 124 wherein said period is from about 10 seconds to about 5 minutes.

129. The method of aspect 124 wherein said period is about 10 seconds.

130. The method of aspect 124 wherein said period is about 30 seconds.

131. The method of aspect 124 wherein said period is about 1 minute.

132. The method of aspect 124 wherein said period is about 5 minutes.

133. The method of aspect 124 wherein said period is about 15 minutes.

134. The method of aspect 124 wherein said period is about 30 minutes.

135. The method of any of aspects 122-134 wherein said administration results in $C_{max}$ plasma levels of [Glu$^{22,25}$, Leu$^{23,28,31}$, Aib$^{29}$, Lys$^{26,30}$]hPTHrP(1-34)NH$_2$ of greater than 200 pg/mL.

136. The method of any of aspects 122-134 wherein said administering results in $C_{max}$ plasma levels of [Glu$^{22,25}$, Leu$^{23,28,31}$, Aib$^{29}$, Lys$^{26,30}$]hPTHrP(1-34)NH$_2$ of greater than 300 pg/mL.

137. The method of any of aspects 122-134 wherein said administering results in $C_{max}$ plasma levels of [Glu$^{22,25}$, Leu$^{23,28,31}$, Aib$^{29}$, Lys$^{26,30}$]hPTHrP(1-34)NH$_2$ of greater than 400 pg/mL.

138. The method of any of aspects 122-134 wherein said administering results in $C_{max}$ plasma levels of [Glu$^{22,25}$, Leu$^{23,28,31}$, Aib$^{29}$, Lys$^{26,30}$]hPTHrP(1-34)NH$_2$ of greater than 500 pg/mL.

139. The method of any of aspects 122-134 wherein the plasma $T_{max}$ occurs at less than one hour post-administration time.

140. The method of any of the aspects 122-134 wherein the plasma $T_{max}$ occurs at less than ½ hour post administration time.

141. The method of any of aspects 122-140 wherein said microprojection array is contacted on the surface of the stomach area of said human.

142. The method of any of aspects 122-140 wherein said microprojection array is contacted on the surface of the deltoid region of said human.

143. The method of any of aspects 122-140 wherein said microprojection array is contacted on the surface of the thigh of said human.

144. The method of any of aspects 122-140 wherein said microprojection array is contacted on the surface of the upper chest of said human.

## Claims

1. A formulation comprising [Glu$^{22,25}$, Leu$^{23,28,31}$, Aib$^{29}$, Lys$^{26,30}$]hPTHrP(1-34)NH$_2$ (SEQ ID NO.: 2), for use in a method of treating osteoporosis, one or more fractured bones, or osteoarthritis; wherein the formulation is administered using a microprojection array, wherein the microprojection array comprises a backing material with a plurality of attached microprojections, wherein
at least one of said microprojections comprises a coating of the formulation; and
the microprojection array is suitable for transdermal administration to a subject resulting in a peak plasma concentration of greater than 400 pg/mL of [Glu$^{22,25}$,Leu$^{23,28,31}$,Aib$^{29}$,Lys$^{26,30}$]hPTHrP(1-34)NH$_2$.

2. The formulation for use according to claim 1, wherein the microprojection array is suitable for transdermal administration to a subject resulting in a peak plasma concentration of greater than 500 pg/mL of [Glu$^{22,25}$,Leu$^{23,28,31}$,Aib$^{29}$,L ys$^{26,30}$]hPTHrP(1-34)NH$_2$.

3. The formulation for use according to claim 1, wherein the microprojection array is suitable for transdermal administration to a subject resulting in a peak plasma concentration of between 419 and 933 pg/mL of [Glu$^{22,25}$,Leu$^{23,28,31}$,Aib$^{29}$,Lys$^{26,30}$]hPTHrP(1-34)NH$_2$.

4. The formulation for use according to any one of the previous claims, wherein the microprojection array comprises from 80 μg to 450 μg of [Glu$^{22,25}$, Leu$^{23,28,31}$, Aib$^{29}$, Lys$^{26,30}$]hPTHrP(1-34)NH$_2$ (SEQ ID NO.: 2).

5. The formulation for use according to any one of the previous claims, wherein the microprojection array comprises 100 μg of [Glu$^{22,25}$, Leu$^{23,28,31}$, Aib$^{29}$, Lys$^{26,30}$]hPTHrP(1-34)NH$_2$ (SEQ ID NO.: 2).

6.  The formulation for use according to any one of the previous claims, wherein said microprojections comprise a liquid crystal polymer.

7.  The formulation for use according to any one of the previous claims, wherein said microprojections are microneedles.

8.  The formulation for use according to any one of the previous claims, wherein said microprojections are pyramidal in shape.

9.  The formulation for use according to any one of the previous claims, wherein said microprojections have a square base and square tip.

10. The formulation for use according to any one of the previous claims, wherein said microprojections are 500 microns in length.

11. The formulation for use according to claim 3 wherein Cl/F of the subject after the transdermal administration is between 264.6 and 540.6.

12. The formulation for use according to any one of the previous claims wherein the transdermal administration is to the thigh.

13. The formulation for use according to any one of the previous claims wherein said subject is a post-menopausal woman.

**FIG. 1**

**FIG. 2**

**FIG. 3**

**FIG. 4**

**FIG. 5**

**FIG. 6**

**FIG. 7**

FIG. 8

* p < 0.05 compared to treatment corresponding placebo control

**FIG. 9**

**FIG. 10**

**FIG. 11**

**FIG. 12**

Change From Baseline Serum CTX Concentration (ng/mL)

**FIG. 13**

Change From Baseline Serum CTX Concentration (ng/mL)

FIG. 14

Change From Baseline Serum PINP Concentration (ng/mL)

Legend:
- 2A = 1 x 150 µg 10 Second Wear Time Daily for 7 Days (Periumbilical)
- 2B = 1 x 150 µg, 15 Minute Wear Time Daily for 7 Days (Periumbilical)
- 2C = 1 x 100 µg, 15 Minute Wear Time Daily for 7 Days (Upper Thigh)
- Placebo

**FIG. 15**

Change From Baseline Serum PINP Concentration (ng/mL)

**FIG. 16**

Europäisches
Patentamt

European
Patent Office

Office européen
des brevets

# EUROPEAN SEARCH REPORT

**Application Number**

EP 17 19 0659

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2009/137093 A1 (RADIUS HEALTH INC [US]; IPSEN PHARMA SAS [FR]; DEY MICHAEL J [GB]; MON) 12 November 2009 (2009-11-12)<br>* abstract *<br>* page 2, line 4 - line 29 *<br>* page 6, line 20 - line 26 *<br>* page 9, line 4 - line 9 *<br>* page 27, line 21 - line 22 * | 1-13 | INV.<br>A61K38/29<br>A61P19/10<br>A61K9/20<br>A61M37/00 |
| Y | US 2009/117158 A1 (AMERI MAHMOUD [US] ET AL) 7 May 2009 (2009-05-07)<br>* abstract *<br>* claim 13 * | 1-13 | |
| Y | WO 2007/084247 A2 (ALZA CORP [US]; AMERI MAHMOUD [US]; CORMIER MICHEL J N [US]; SELLERS S) 26 July 2007 (2007-07-26)<br>* abstract *<br>* page 3, paragraph 15 *<br>* page 5, paragraph 32 * | 1-13 | |
| Y | WO 2005/044333 A2 (ALZA CORP [US]; TRAUTMAN JOSEPH C [US]; OLSON LORIN [US]) 19 May 2005 (2005-05-19)<br>* abstract *<br>* page 6, paragraph 27 *<br>* page 7, paragraph 31 *<br>* page 10, paragraph 36 *<br>* page 16, paragraph 66 *<br>* page 22, paragraph 97 * | 1-13 | **TECHNICAL FIELDS SEARCHED (IPC)**<br>A61P<br>A61K<br>A61M |
| Y | WO 2005/044985 A2 (ALZA CORP [US]; ZALIPSKY SAMUEL [US]; BENTZ JOHANNA H [US]) 19 May 2005 (2005-05-19)<br>* abstract *<br>* page 7, paragraph 29 *<br>* page 13, paragraph 60 * | 1-13 | |

The present search report has been drawn up for all claims

| Place of search | Date of completion of the search | Examiner |
|---|---|---|
| Munich | 21 February 2018 | Grötzinger, Thilo |

## ANNEX TO THE EUROPEAN SEARCH REPORT
## ON EUROPEAN PATENT APPLICATION NO.

EP 17 19 0659

This annex lists the patent family members relating to the patent documents cited in the above-mentioned European search report.
The members are as contained in the European Patent Office EDP file on
The European Patent Office is in no way liable for these particulars which are merely given for the purpose of information.

21-02-2018

| Patent document cited in search report | | | Publication date | Patent family member(s) | | | Publication date |
|---|---|---|---|---|---|---|---|
| WO 2009137093 | A1 | | 12-11-2009 | US | 2009227498 | A1 | 10-09-2009 |
| | | | | US | 2011092425 | A1 | 21-04-2011 |
| | | | | US | 2013157955 | A1 | 20-06-2013 |
| | | | | WO | 2009137093 | A1 | 12-11-2009 |
| US 2009117158 | A1 | | 07-05-2009 | US | 2009117158 | A1 | 07-05-2009 |
| | | | | WO | 2009054988 | A1 | 30-04-2009 |
| WO 2007084247 | A2 | | 26-07-2007 | AU | 2006336187 | A1 | 26-07-2007 |
| | | | | CA | 2635251 | A1 | 26-07-2007 |
| | | | | EP | 1981547 | A2 | 22-10-2008 |
| | | | | ES | 2551305 | T3 | 17-11-2015 |
| | | | | JP | 2009522288 | A | 11-06-2009 |
| | | | | US | 2007184096 | A1 | 09-08-2007 |
| | | | | US | 2014260096 | A1 | 18-09-2014 |
| | | | | US | 2017135951 | A1 | 18-05-2017 |
| | | | | WO | 2007084247 | A2 | 26-07-2007 |
| WO 2005044333 | A2 | | 19-05-2005 | AR | 046696 | A1 | 21-12-2005 |
| | | | | AT | 539687 | T | 15-01-2012 |
| | | | | AU | 2004287414 | A1 | 19-05-2005 |
| | | | | BR | PI0415629 | A | 12-12-2006 |
| | | | | CA | 2543641 | A1 | 19-05-2005 |
| | | | | CN | 1901840 | A | 24-01-2007 |
| | | | | EP | 1680154 | A2 | 19-07-2006 |
| | | | | ES | 2377647 | T3 | 29-03-2012 |
| | | | | JP | 4682144 | B2 | 11-05-2011 |
| | | | | JP | 2007509706 | A | 19-04-2007 |
| | | | | KR | 20060099523 | A | 19-09-2006 |
| | | | | TW | 200633675 | A | 01-10-2006 |
| | | | | US | 2005096586 | A1 | 05-05-2005 |
| | | | | US | 2007027427 | A1 | 01-02-2007 |
| | | | | WO | 2005044333 | A2 | 19-05-2005 |
| WO 2005044985 | A2 | | 19-05-2005 | AR | 047229 | A1 | 11-01-2006 |
| | | | | AU | 2004287059 | A1 | 19-05-2005 |
| | | | | BR | PI0415967 | A | 23-01-2007 |
| | | | | CA | 2543280 | A1 | 19-05-2005 |
| | | | | CN | 1897883 | A | 17-01-2007 |
| | | | | EP | 1677687 | A2 | 12-07-2006 |
| | | | | JP | 2007535337 | A | 06-12-2007 |
| | | | | KR | 20070001886 | A | 04-01-2007 |
| | | | | TW | 200539907 | A | 16-12-2005 |
| | | | | US | 2005106227 | A1 | 19-05-2005 |
| | | | | WO | 2005044985 | A2 | 19-05-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61478466 A
- US 61578120 A
- WO 2008063279 A
- US 20080051699 A
- US 20090198189 A
- US 20050096586 A
- US 20090227498 A
- US 5969095 A
- US 20050256045 A

**Non-patent literature cited in the description**

- **NEER et al.** *New England Journal of Medicine,* 10 May 2001, vol. 344, 1434-1441